# EUROPÄISCHE PATENTSCHRIFT

(11) **EP 0 741 694 B1**
(45) Veröffentlichungstag und Bekanntmachung des Hinweises auf die Patenterteilung: **02.04.2003**
(21) Anmeldenummer: 95905614.4
(22) Anmeldetag: 03.01.1995
(51) Int. Cl.: C07C 251/38, C07C 251/40, C07C 251/44, C07C 251/48, C07C 251/52, C07C 251/54, C07C 251/58, C07D 213/62, C07D 207/36, C07D 239/46, C07D 261/12

(54) **PHENYLESSIGSÄUREDERIVATE, VERFAHREN UND ZWISCHENPRODUKTE ZU IHRER HERSTELLUNG UND SIE ENTHALTENDE MITTEL**
PHENYL ACETIC ACID DERIVATIVES, PROCESS AND INTERMEDIATE PRODUCTS FOR THEIR PRODUCTION AND AGENTS CONTAINING THEM
DERIVES D'ACIDE ACETIQUE PHENYLIQUE, LEUR PROCEDE DE PREPARATION, PRODUITS INTERMEDIAIRES A CET EFFET ET AGENTS LES CONTENANT

(30) Priorität: 04.02.1994 DE 4403448; 17.06.1994 DE 4421182
(43) Veröffentlichungstag der Anmeldung: 13.11.1996
(73) Patentinhaber: BASF AKTIENGESELLSCHAFT, 67056 Ludwigshafen (DE)
(72) Erfinder: BAYER, Herbert, D-68159 Mannheim (DE); SAUTER, Hubert, D-68167 Mannheim (DE); MÜLLER, Ruth, D-56626 Andernach (DE); GRAMMENOS, Wassilios, D-67063 Ludwigshafen (DE); HARREUS, Albrecht, D-67063 Ludwigshafen (DE); KIRSTGEN, Reinhard, D-67434 Neustadt (DE); RÖHL, Franz, D-67105 Schifferstadt (DE); AMMERMANN, Eberhard, D-64646 Heppenheim (DE); LORENZ, Gisela, D-67434 Hambach (DE)
(86) Internationale Anmeldenummer: EP9500007
(87) Internationale Veröffentlichungsnummer: WO95021154

(56) Entgegenhaltungen:
- EP-A- 0 463 488
- EP-A- 0 585 751
- EP-A- 0 602 514
- WO-A-92/13830
- WO-A-94/26700
- PATENT ABSTRACTS OF JAPAN vol. 17 no. 644 (C-1134) ,30.November 1993 & JP,A,05 201946 (MITSUBISHI KASEI CORP.) 10.August 1993,
- PATENT ABSTRACTS OF JAPAN vol. 18 no. 19 (C-1152) ,13.Januar 1994 & JP,A,05 255012 (MITSUBISHI KASEI CORP.) 5.Oktober 1993,
- PATENT ABSTRACTS OF JAPAN vol. 18 no. 93 (C-1166) ,16.Februar 1994 & JP,A,05 294948 (NIPPON NOHYAKU CO. LTD.) 9.November 1993,

## Beschreibung

Die vorliegende Erfindung betrifft Phenylessigsäurederivate der Formel I 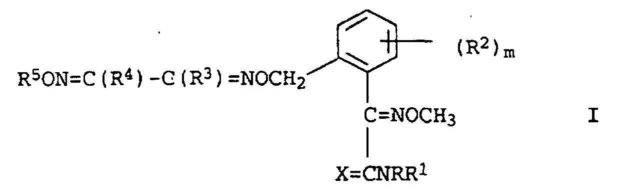 in der die Substituenten und der Index die folgende Bedeutung haben:
- X: Sauerstoff oder Schwefel;
- R: Wasserstoff und C₁-C₄-Alkyl;
- R¹: Wasserstoff und C₁-C₄-Alkyl;
- R²: Cyano, Nitro, Trifluormethyl, Halogen, C₁-C₄-Alkyl und C₁-C₄-Alkoxy;
- m: 0, 1 oder 2, wobei die Reste R² verschieden sein können, wenn m für 2 steht;
- R³: Wasserstoff, Cyano, Nitro, Hydroxy, Amino, Halogen,
C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylamino oder Di-C₁-C₄-alkylamino;
- R⁴: Wasserstoff, Nitro, Hydroxy, Amino, Halogen, C₁-C₆-Alkyl, C₁-C₆-Alkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkenylthio, C₂-C₆-Alkenylamino, N-C₂-C₆-Alkenyl-N-C₁-C₆-alkylamino, C₂-C₆-Alkinyl, C₂-C₆-Alkinyloxy, C₂-C₆-Alkinylthio, C₂-C₆-Alkinylamino, N-C₂-C₆-Alkinyl-N-C₁-C₆-alkylamino, wobei die Kohlenwasserstoffreste dieser Gruppen partiell oder vollständig halogeniert sein können oder einen bis drei der folgenden Reste tragen können: Cyano, Nitro, Hydroxy, Merkapto,Amino, Carboxyl, Aminocarbonyl, Aminothiocarbonyl, Halogen, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Hetaryl, Hetaryloxy, Hetaryl-C₁-C₄-alkoxy, Hetarylthio, Hetaryl-C₁-C₄-alkylthio, wobei die cyclischen Reste ihrerseitspartiell oder vollständig halogeniert sein können und/oder ein bis drei der folgenden Gruppen tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio und C(=NOR⁶)-Aₙ-R⁷;
C₃-C₆-Cycloalkyl, C₃-C₆-Cycloalkyloxy, C₃-C₆-Cycloalkylthio, C₃-C₆-Cycloalkylamino, N-C₃-C₆-Cycloalkyl-N-C₁-C₆-alkylamino, C₃-C₆-Cycloalkenyl, C₃-C₆-Cycloalkenyloxy, C₃-C₆-Cycloalkenylthio, C₃-C₆-Cycloalkenylamino, N-C₃-C₆-Cycloalkenyl-N-C₁-C₆-alkylamino, Heterocyclyloxy, Heterocyclylthio, Heterocyclylamino, N-Heterocyclyl-N-C₁-C₆-alkylamino, Aryl, Aryloxy, Arylthio, Arylamino, N-Aryl-N-C₁-C₆-alkylamino, Hetaryl, Hetaryloxy, Hetarylthio, Hetarylamino, N-Hetaryl-N-C₁-C₆-alkylamino, wobei die cyclischen Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl und Hetaryloxy;
- R⁵: Wasserstoff,
C₁-C₁₀-Alkyl, C₃-C₆-Cycloalkyl, C₂-C₁₀-Alkenyl, C₂-C₁₀-Alkinyl, C₁-C₁₀-Alkylcarbonyl, C₂-C₁₀-Alkenylcarbonyl, C₃-C₁₀-Alkinylcarbonyl oder C₁-C₁₀-Alkylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxyl, C₃-C₆-Cycloalkyl, C₃-C₆-Cycloalkyloxy, Heterocyclyl, Heterocyclyloxy, Benzyl, Benzyloxy, Aryl, Aryloxy, Arylthio, Hetaryl, Hetaryloxy und Hetarylthio, wobei die cyclischen Gruppen ihrerseits partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C(=NOR⁶)-Aₙ-R⁷;
Aryl, Arylcarbonyl, Arylsulfonyl, Hetaryl, Hetarylcarbonyl oder Hetarylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbony, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Hetaryl, Hetaryloxy oder C(=NOR⁶) -Aₙ-R⁷;
wobei
- A: für Sauerstoff, Schwefel oder Stickstoff steht und wobei der Stickstoff Wasserstoff oder C₁-C₆-Alkyl trägt;
- n: 0 oder 1 bedeutet;
- R⁶: Wasserstoff oder C₁-C₆-Alkyl bedeutet;
- R⁷: Wasserstoff oder C₁-C₆-Alkyl bedeutet;
Aryl für Phenyl, Naphthyl oder Phenanthrenyl steht;
Hetaryl
5-gliedriges Heteroaryl, enthaltend ein bis vier Stickstoffatome oder ein bis drei Stickstoffatome und ein Schwefel- oder Sauerstoffatom oder ein Sauerstoff oder ein Schwefelatom,
benzokondensiertes 5-gliedriges Heteroaryl, enthaltend ein bis drei Stickstoffatome oder ein Stickstoffatom und/oder ein Sauerstoff- oder Schwefelatom,
über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome,
6-gliedriges Heteroaryl, enthaltend ein bis vier Stickstoffatome oder
benzokondensiertes 6-gliedriges Heteroaryl, enthaltend ein bis vier Stickstoffatome bedeutet;
Heterocyclyl drei- bis sechsgliedrige, gesättigte oder partiell ungesättigte mono- oder polycyclische Heterocyclen, die ein bis drei Heteroatome ausgewählt aus einer. Gruppe bestehend aus Sauerstoff, Stickstoff und Schwefel enthalten, bedeutet;
ausgenommen Verbindungen der Formel I, in denen
- X: Sauerstoff;
NRR¹NHCH₃;
- m: 0;
- R³: Wasserstoff, Cyano, C₁-C₄-Alkyl, C₁-C₄-Halogenalkyl oder Methylthio;
- R⁴: Wasserstoff, unsubstituiertes C₁-C₆-Alkyl, unsubstituiertes C₃-C₆-Cycloalkyl, Thienyl oder
Phenyl, das ggf. substituiert ist durch:
Halogen, Cyano, Nitro, C₁-C₄-Alkyl, C₁-C₄-Alkoxy, C₁-C₂-Halogenalkyl, C₁-C₂-Halogenalkoxy, C₃-C₆-Alkenyloxy, C₃-C₆-Alkinyloxy, C₃-C₄-Alkylendioxy; oder
folgende über Sauerstoff, C₁-C₄-Alkylenoxy, C₁-C₄-Oxyalkylen, S(O)ₙ, C₁-C₄-Alkylen-S(O)ₙ, S(O)ₙ-C₁-C₄-Alkylen, wobei n für 0, 1 oder 2 steht,
   gebundene Reste:
   C₁-C₆-Alkyl, C₁-C₆-Halogenalkyl, C₃-C₆-Cycloalkyl oder C₂-C₆-Alkenyl oder C₂-C₄-Alkinyl-C₁-C₂-alkyl, wobei diese Reste 1 bis 3-fach halogeniert sein können; oder
   ggf. durch Halogen, Trimethylsilyl, C₁-C₆-Alkyl, C₁-C₆-Halogenalkyl, C₁-C₆-Alkoxy, C₁-C₆-Halogenalkoxy, ggf. substituiertes Cyclopropyl oder Cyclopropyl-C₁-C₃-alkyl substituierte Aryl- oder Heterocyclylreste;
und
- R⁵: Wasserstoff, C₁-C₆-Alkyl, C₁-C₆-Halogenalkyl; C₁-C₄-Alkoxy-C₁-C₂-alkyl, ggf. 1- bis 3-fach halogeniertes C₂-C₄-Alkenyl-C₁-C₂-alkyl, C₂-C₄-Alkinyl-C₁-C₂-alkyl, ggf. 1- bis 4-fach halogeniertes C₃-C₆-Cycloalkyl, ggf. 1- bis 4-fach halogeniertes C₃-C₆-Cycloalkyl-C₁-C₂-alkyl, Cyano-C₁-C₄-alkyl, C₁-C₄-Alkoxycarbonyl-C₁-C₂-alkyl;
Phenyl-C₁-C₃-alkyl, das substituiert sein kann durch: Halogen, Cyano, Nitro, C₁-C₃-Alkyl, C₁-C₄-Alkoxy, C₁-C₄-Halogenalkyl,
wobei die Phenylgruppe ggf. 1- bis 3-fach durch gleiche oder verschiedene Reste substituiert sein kann;
Phenyl, das ggf. 1- oder 2-fach substituiert sein kann durch: Halogen, Cyano, Nitro, C₁-C₄-Alkyl, C₁-C₄-Alkoxy, C₁-C₂-Halogenalkyl, wobei dieser Rest 1 bis 3 Halogenatome trägt;
oder
Pyridyl, das 1- bis 2-fach substituiert sein kann durch: Halogen, Cyano, Nitro, C₁-C₄-Alkyl, C₁-C₄-Alkoxy, C₁-C₂-Halogenalkyl, wobei dieser Rest 1 bis 3 Halogenatome trägt, bedeuten,
wobei von den aus genommenen Verbindungen die folgenden Einzelverbindungen der Formel I.3a

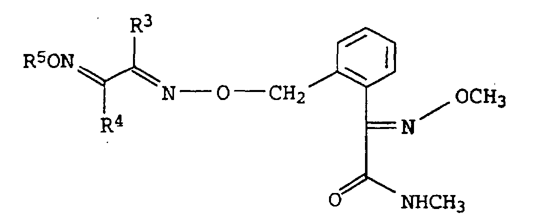

**Tabelle A**

| Nr. | R³ | R⁴ | R⁵ |
|---|---|---|---|
| 631 | | C₆H₅ | C₂H₅ |
| 1157 | | 2-F-C₆H₄ | C₂H₅ |
| 1168 | | 3-F-C₆H₄ | CH₃ |
| 1169 | | 3-F-C₆H₄ | C₂H₅ |
| 1181 | | 4-F-C₆H₄ | C₂H₅ |
| 1192 | | 2-Cl-C₆H₄ | CH₃ |
| 1193 | | 2-Cl-C₆H₄ | C₂H₅ |
| 1205 | | 3-Cl-C₆H₄ | C₂H₅ |
| 1217 | | 4-Cl-C₆H₄ | C₂H₅ |
| 1228 | | 2,3-Cl₂-C₆H₃ | CH₃ |
| 1229 | | 2,3-Cl₂-C₆H₃ | C₂H₅ |
| 1240 | | 2,4-Cl₂-C₆H₃ | CH₃ |
| 1241 | | 2,4-Cl₂-C₆H₃ | C₂H₅ |
| 1252 | | 2,5-Cl₂-C₆H₃ | CH₃ |
| 1253 | | 2,5-Cl₂-C₆H₃ | C₂H₅ |
| 1264 | | 2,6-Cl₂-C₆H₃ | CH₃ |
| 1265 | | 2,6-Cl₂-C₆H₃ | C₂H₅ |
| 1276 | | 3,4-Cl₂-C₆H₃ | CH₃ |
| 1277 | | 3,4-Cl₂-C₆H₃ | C₂H₅ |
| 1288 | | 3,5-Cl₂-C₆H₃ | CH₃ |
| 1289 | | 3,5-Cl₂-C₆H₃ | C₂H₅ |
| 1300 | | 2-Br-C₆H₄ | CH₃ |
| 1301 | | 2-Br-C₆H₄ | C₂H₅ |
| 1313 | | 3-Br-C₆H₄ | C₂H₅ |
| 1325 | | 4-Br-C₆H₄ | C₂H₅ |
| 1336 | | 2-I-C₆H₄ | CH₃ |
| 1337 | | 2-I-C₆H₄ | C₂H₅ |
| 1348 | | 3-I-C₆H₄ | CH₃ |
| 1349 | | 3-I-C₆H₄ | C₂H₅ |
| 1360 | | 4-I-C₆H₄ | CH₃ |
| 1361 | | 4-I-C₆H₄ | C₂H₅ |
| 1372 | | 2-CN-C₆H₄ | CH₃ |
| 1373 | | 2-CN-C₆H₄ | C₂H₅ |
| 1384 | | 3-CN-C₆H₄ | CH₃ |
| 1385 | | 3-CN-C₆H₄ | C₂H₅ |
| 1396 | | 4-CN-C₆H₄ | CH₃ |
| 1397 | | 4-CN-C₆H₄ | C₂H₅ |
| 1408 | | 2-NO₂-C₆H₄ | CH₃ |
| 1409 | | 2-NO₂-C₆H₄ | C₂H₅ |
| 1420 | | 3-NO₂-C₆H₄ | CH₃ |
| 1421 | | 3-NO₂-C₆H₄ | C₂H₅ |
| 1432 | | 4-NO₂-C₆H₄ | CH₃ |
| 1433 | | 4-NO₂-C₆H₄ | C₂H₅ |
| 1445 | | 2-CH₃-C₆H₄ | C₂H₅ |
| 1456 | | 3-CH₃-C₆H₄ | CH₃ |
| 1457 | | 3-CH₃-C₆H₄ | C₂H₅ |
| 1469 | | 4-CH₃-C₆H₄ | C₂H₅ |
| 1480 | | 2,3-(CH₃)₂-C₆H₃ | CH₃ |
| 1481 | | 2,3-(CH₃)₂-C₆H₃ | C₂H₅ |
| 1492 | | 2,4-(CH₃)₂-C₆H₃ | CH₃ |
| 1493 | | 2,4-(CH₃)₂-C₆H₃ | C₂H₅ |
| 1504 | | 2,5-(CH₃)₂-C₆H₃ | CH₃ |
| 1505 | | 2,5-(CH₃)₂-C₆H₃ | C₂H₅ |
| 1516 | | 2,6-(CH₃)₂-C₆H₃ | CH₃ |
| 1517 | | 2,6-(CH₃)₂-C₆H₃ | C₂H₅ |
| 1528 | | 3,4-(CH₃)₂-C₆H₃ | CH₃ |
| 1529 | | 3,4-(CH₃)₂-C₆H₃ | C₂H₅ |
| 1540 | | 3,5-(CH₃)₂-C₆H₃ | CH₃ |
| 1541 | | 3,5-(CH₃)₂-C₆H₃ | C₂H₅ |
| 1552 | | 2-C₂H₅-C₆-H₄ | CH₃ |
| 1553 | | 2-C₂H₅-C₆-H₄ | C₂H₅ |
| 1562 | | 3-C₂H₅-C₆-H₄ | CH₃ |
| 1563 | | 3-C₂H₅-C₆-H₄ | C₂H₅ |
| 1574 | | 4-C₂H₅-C₆-H₄ | CH₃ |
| 1575 | | 4-C₂H₅-C₆-H₄ | C₂H₅ |
| 1586 | | 2-i-C₃H₇-C₆H₄ | CH₃ |
| 1587 | | 2-i-C₃H₇-C₆H₄ | C₂H₅ |
| 1598 | | 3-i-C₃H₇-C₆H₄ | CH₃ |
| 1599 | | 3-i-C₃H₇-C₆H₄ | C₂H₅ |
| 1610 | | 4-i-C₃H₇-C₆H₄ | CH₃ |
| 1611 | | 4-i-C₃H₇-C₆H₄ | C₂H₅ |
| 1658 | | 2-OCH₃-C₆H₄ | CH₃ |
| 1659 | | 2-OCH₃-C₆H₄ | C₂H₅ |
| 1670 | | 3-OCH₃-C₆H₄ | CH₃ |
| 1671 | | 3-OCH₃-C₆H₄ | C₂H₅ |
| 1683 | | 4-OCH₃-C₆H₄ | C₂H₅ |
| 1694 | | 2-OC₂H₅-C₆H₄ | CH₃ |
| 1695 | | 2-OC₂H₅-C₆H₄ | C₂H₅ |
| 1706 | | 3-OC₂H₅-C₆H₄ | CH₃ |
| 1707 | | 3-OC₂H₅-C₆H₄ | C₂H₅ |
| 1719 | | 4-OC₂H₅-C₆H₄ | C₂H₅ |
| 1730 | | 2-O-(i-C₃H₇)-C₆H₄ | CH₃ |
| 1731 | | 2-O-(i-C₃H₇)-C₆H₄ | C₂H₅ |
| 1742 | | 3-O-(i-C₃H₇)-C₆H₄ | CH₃ |
| 1743 | | 3-O-(i-C₃H₇)-C₆H₄ | C₂H₅ |
| 1754 | | 4-O-(i-C₃H₇)-C₆H₄ | CH₃ |
| 1755 | | 4-O-(i-C₃H₇)-C₆H₄ | C₂H₅ |
| 1766 | | 2-O-(t-C₄H₉)-C₆H₄ | CH₃ |
| 1767 | | 2-O-(t-C₄H₉)-C₆H₄ | C₂H₅ |
| 1778 | | 3-O-(t-C₄H₉)-C₆H₄ | CH₃ |
| 1779 | | 3-O-(t-C₄H₉)-C₆H₄ | C₂H₅ |
| 1790 | | 4-O-(t-C₄H₉)-C₆H₄ | CH₃ |
| 1791 | | 4-O-(t-C₄H₉)-C₆H₄ | C₂H₅ |
| 1803 | | 2-CF₃-C₆H₄ | C₂H₅ |
| 1815 | | 3-CF₃-C₆H₄ | C₂H₅ |
| 1826 | | 4-CF₃-C₆H₄ | CH₃ |
| 1827 | | 4-CF₃-C₆H₄ | C₂H₅ |
| 1946 | | 2-OCF₃-C₆H₄ | CH₃ |
| 1947 | | 2-OCF₃-C₆H₄ | C₂H₅ |
| 1960 | | 3-OCF₃-C₆H₄ | CH₃ |
| 1961 | | 3-OCF₃-C₆H₄ | C₂H₅ |
| 1972 | | 4-OCF₃-C₆H₄ | CH₃ |
| 1973 | | 4-OCF₃-C₆H₄ | C₂H₅ |
| 1984 | | 2-SCH₃-C₆H₄ | CH₃ |
| 1985 | | 2-SCH₃-C₆H₄ | C₂H₅ |
| 1996 | | 3-SCH₃-C₆H₄ | CH₃ |
| 1997 | | 3-SCH₃-C₆H₄ | C₂H₅ |
| 2008 | | 4-SCH₃-C₆H₄ | CH₃ |
| 2009 | | 4-SCH₃-C₆H₄ | C₂H₅ |
| 2020 | | 2-Methyl-sulfonyl-C₆H₄ | CH₃ |
| 2021 | | 2-Methyl-sulfonyl-C₆H₄ | C₂H₅ |
| 2032 | | 3-Methyl-sulfonyl-C₆H₄ | CH₃ |
| 2033 | | 3-Methyl-sulfonyl-C₆H₄ | C₂H₅ |
| 2044 | | 4-Methyl-sulfonyl-C₆H₄ | CH₃ |
| 2045 | | 4-Methyl-sulfonyl-C₆H₄ | C₂H₅ |

ausgeschlossen sind.

Außerdem betrifft die Erfindung Verfahren zur Herstellung dieser Verbindungen sowie sie enthaltende Mittel zur Bekämpfung tierischer Schädlinge und Schadpilze.

Aus der Literatur sind Phenylessigsäurederivate zur Schädlingsbekämpfung bekannt (EP-A 398 692, EP-A 477 631, EP-A 513 580, EP-A 567 828, EP-A 528 682, EP-A 463 488, WO-A 92/13,830).

In den nachveröffentlichten Schriften WO-A 95/18789 und WO-A 96/11183 sind weitere Phenylessigsäurederivate zur Schädlingsbekämpfung beschrieben.

Der vorliegenden Erfindung lagen neue Verbindungen mit verbesserter Wirkung als Aufgabe zugrunde.

Demgemäß wurden die eingangs definierten Phenyiessigsäurederivate I gefunden. Außerdem wurden Verfahren und Zwischenprodukte zu ihrer Herstellung sowie sie enthaltende Mittel zur Bekämpfung von tierischen Schädlingen und Schadpilzen und ihre Verwendung in diesem Sinne gefunden.

Die Verbindungen I sind auf verschiedenen Wegen nach an sich in der Literatur bekannten Verfahren erhältlich.

Grundsätzlich ist es bei der Synthese der Verbindungen I unerheblich, ob zunächst die Gruppierung -C(NOCH₃)-CONRR¹ oder die Gruppierung -CH₂ON=C(R³)-C(R⁴)=NOR⁵ aufgebaut wird.

Der Aufbau der Gruppierung -C(NOCH₃)-CONRR¹ ist beispielsweise aus der eingangs zitierten Literatur bekannt.

Die Art der Synthese der -CH₂ON=C(R³)-C(R⁴)=NOR⁵ Seitenkette richtet sich im wesentlichen nach der Art der Substituenten R³ und R⁴.
1. Für den Fall, daß R³ und R⁴ nicht Halogen bedeuten, geht man beim Aufbau der Gruppierung -CH₂ON=C(R³)-C(R⁴)=NOR⁵ im allgemeinen so vor, daß man ein Benzylderivat der Formel II mit einem Hydroxyimin der Formel III umsetzt. 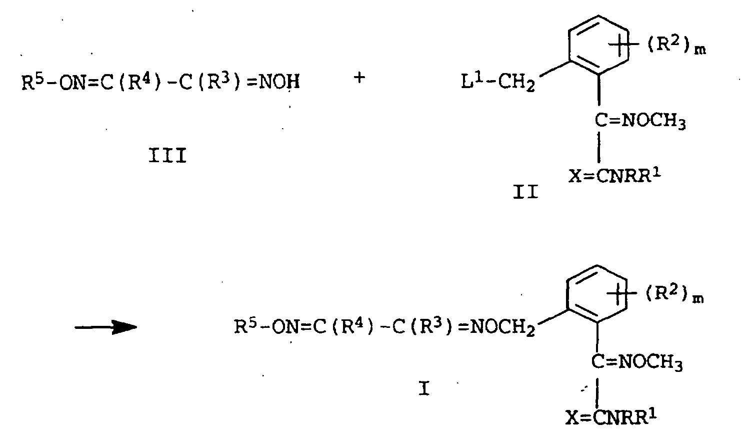
   L¹ in der Formel II steht für eine nukleophil austauschbare Abgangsgruppe, z.B. Halogen oder Sulfonatgruppen, vorzugsweise Chlor, Brom, Iod, Mesylat, Tosylat oder Triflat.
   Die Umsetzung erfolgt in an sich bekannter Weise in einem inerten organischen Lösungsmittel in Gegenwart einer Base, z.B. Natriumhydrid, Kaliumhydroxid, Kaliumcarbonat und Triethylamin gemäß den in Houben-Weyl, Bd. E 14b, S. 370 f und Houben-Weyl, Bd. 10/1, S. 1189 f beschriebenen Methoden.
   Das benötigte Hydroxyimin III erhält man z.B. durch Umsetzung eines entsprechenden Dihydroxyimins IV mit einem nukleophil substituierten Reagens VI 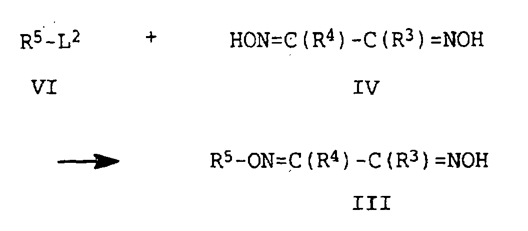
   L² in der Formel VI steht für eine nukleophil austauschbare Abgangsgruppe, z.B. Halogen oder Sulfonatgruppen, vorzugsweise Chlor, Brom, Iod, Mesylat, Tosylat oder Triflat.
   Die Umsetzung erfolgt in an sich bekannter Weise in einem inerten organischen Lösungsmittel in Gegenwart einer Base, z.B. Kaliumcarbonat, Kaliumhydroxid, Natriumhydrid, Pyridin und Triethylamin gemäß den in Houben-Weyl, Bd. E 14b, S. 307 f, S. 370 f und S. 385 f; Houben-Weyl, Bd. 10/4, S. 55 f, S. 180 f und S. 217 f; Houben-Weyl, Bd. E5, S. 780 f, beschriebenen Methoden.
1.1 Alternativ können die Verbindungen I auch dadurch erhalten werden, daß das Benzylderivat II zunächst mit dem Dihydroxyimin IV in ein entsprechendes Benzyloxim der Formel V umgesetzt wird, wobei V anschließend mit dem nukleophil substituierten Reagens VI zu I umgesetzt wird. 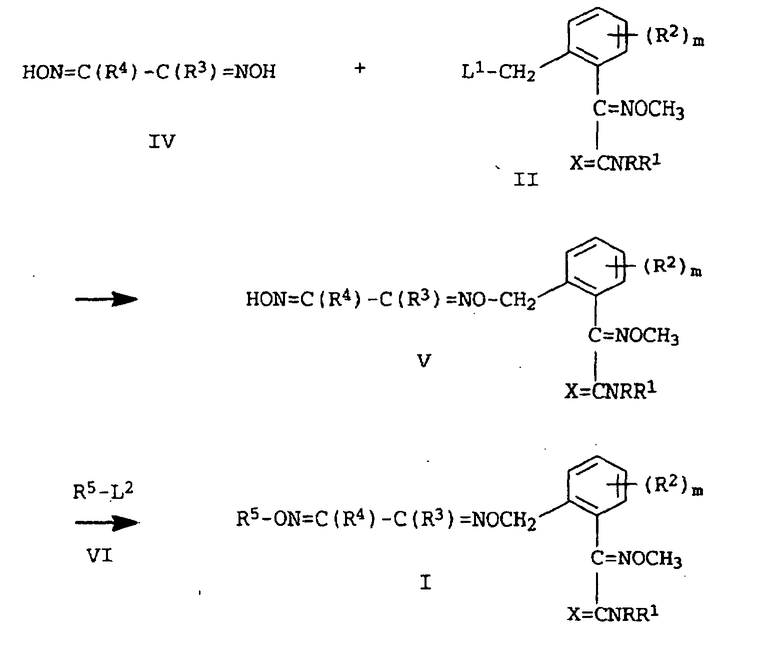
   Die Umsetzung erfolgt in an sich bekannter Weise in einem inerten organischen Lösungsmittel in Gegenwart einer Base, z.B. Kaliumcarbonat, Kaliumhydroxid, Natriumhydrid, Pyridin und Triethylamin gemäß den in Houben-Weyl, Bd. 10/1, S. 1189 f; Houben-Weyl, Bd. E 14b, S. 307 f, S. 370 f und S. 385 f; Houben-Weyl, Bd. 10/4, S. 55 f, S. 180 f und S. 217 f; Houben-Weyl, Bd. E5, S. 780 f, beschriebenen Methoden.
1.2 Analog ist es ebenfalls möglich, das benötigte Hydroxyimin der Formel III aus einem Carbonylhydroxyimin VII durch Umsetzung mit einem Hydroxylamin IXa oder seinem Salz IXb herzustellen. 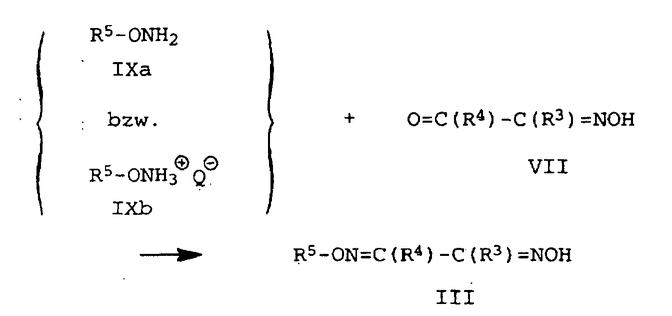
   Q^{⊖} in der Formel IXb steht für das Anion einer Säure, insbesondere einer anorganischen Säure, z.B. Halogenid wie Chlorid.
   Die Umsetzung erfolgt in an sich bekannter Weise in einem inerten organischen Lösungsmittel gemäß den in EP-A 513 .580; Houben-Weyl, Band 10/4, S. 73 f; Houben-Weyl, Bd. E14b, S. 369 f und S. 385 f beschriebenen Methoden.
1.3 Alternativ können die Verbindungen I auch dadurch erhalten werden, daß das Benzylderivat II zunächst mit dem Carbonylhydroxyimin VII in ein entsprechendes Benzylketoxyimin der Formel VIII umgesetzt wird, wobei VIII anschließend mit dem Hydroxylamin IXa bzw. dessen Salz IXb zu I umgesetzt wird. 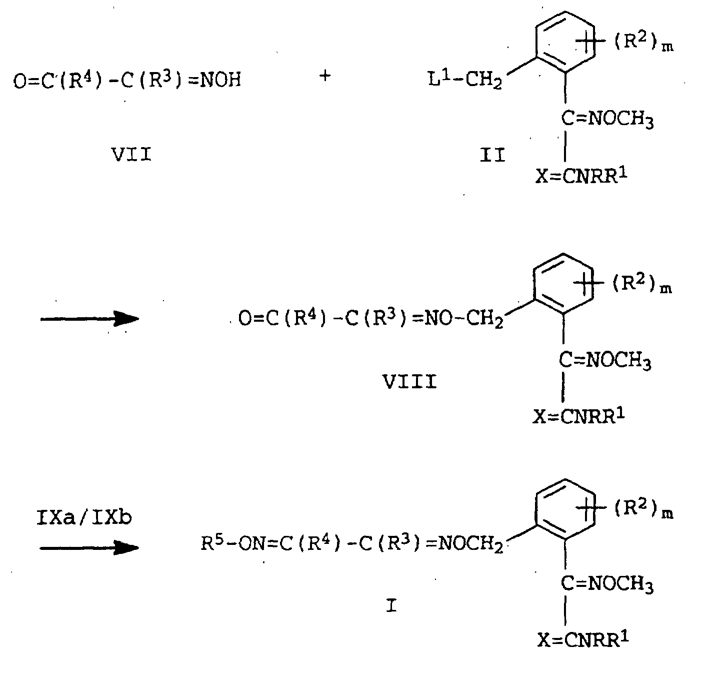
   Die Umsetzung erfolgt in an sich bekannter Weise in einem inerten organischen Lösungsmittel gemäß den in Houben-Weyl, Bd. E14b, S. 369 f; Houben-Weyl, Bd. 10/1, S. 1189 f und Houben-Weyl, Bd. 10/4, S. 73 f oder EP-A 513 580 beschriebenen Methoden.
1.4 Eine weitere Möglichkeit zur Herstellung der Verbindungen I ist die Umsetzung des Benzylderivats II mit N-Hydroxyphthalimid und nachfolgender Hydrazinolyse zum Benzylhydroxylamin IIa und die weitere Umsetzung von IIa mit einer Carbonylverbindung X. 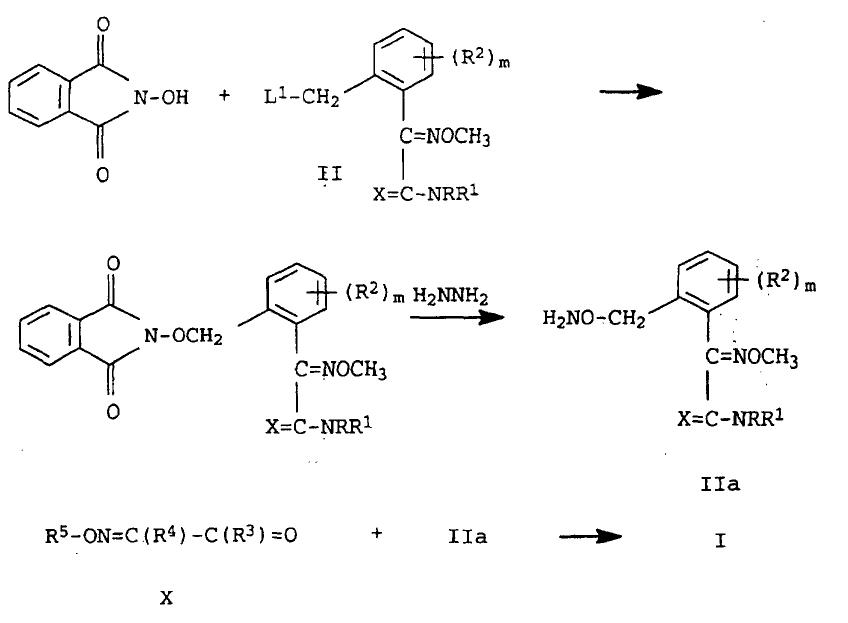
   Die Umsetzung erfolgt in an sich bekannter Weise in einem inerten organischen Lösungsmittel gemäß den in EP-A 463 488 und DE-Anm. Nr. 42 28 867.3 beschriebenen Methoden.
   Die benötigte Carbonylverbindung X erhält man z.B. durch Umsetzung eines entsprechenden Hydroxyiminocarbonyls VIIa mit einem nukleophil substituierten Reagens VI 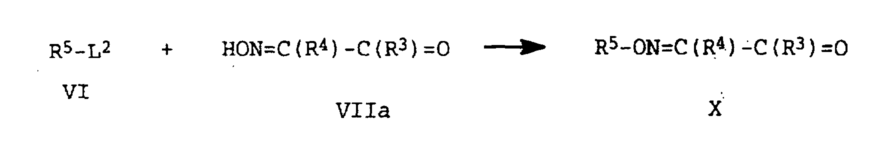 oder durch Umsetzung eines entsprechenden Dicarbonyls XI mit einem Hydroxylamin IXa oder dessen Salz IXb 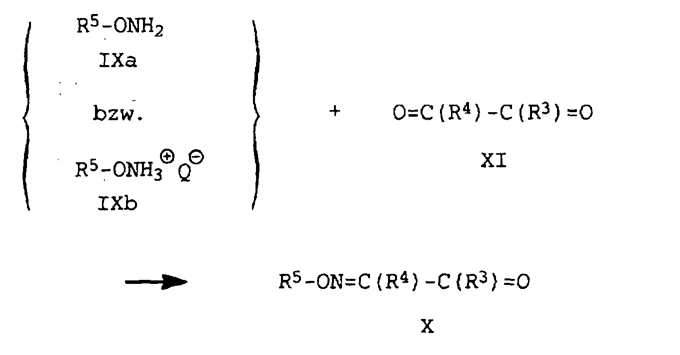
   Die Umsetzungen erfolgen in an sich bekannter Weise in einem inerten organischen Lösungsmittel gemäß den in EP-A 513 580, Houben-Weyl, Bd. 10/4, S. 55 f, S. 73 f, S. 180 f und S. 217 f, Houben-Weyl, Bd. E14b, S. 307 f und 369 f, Houben-Weyl, Bd. E5, S. 780 f, beschriebenen Methoden.
1.5 Entsprechend können die Verbindungen I auch dadurch erhalten werden, daß das Benzylhydroxylamin IIa zunächst mit dem Hydroxyiminocarbonyl VIIa in das entsprechende Benzyloxyimin der Formel V umgesetzt wird, wobei V anschließend mit dem nukleophil substituierten Reagens VI wie vorstehend:beschrieben zu I umgesetzt wird. 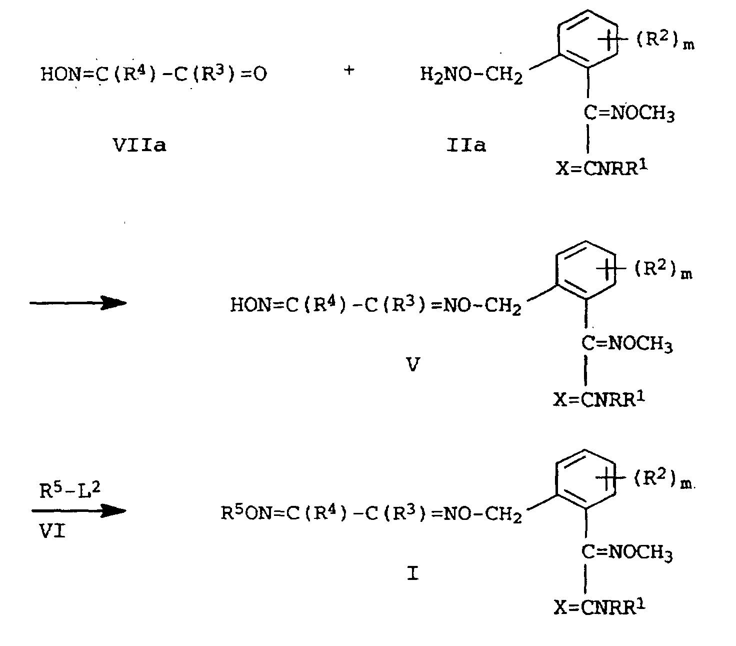
1.6 Analog können die Verbindungen I ebenfalls dadurch hergestellt werden, daß das Benzylhydroxylamin IIa zunächst mit dem Dicarbonyl der Formel XI in das Benzyloxyimin der Formel VIII überführt wird und VIII anschließend mit dem Hydroxylamin IXa oder dessen Salz IXb wie vorstehdend beschrieben zu I umgesetzt wird. 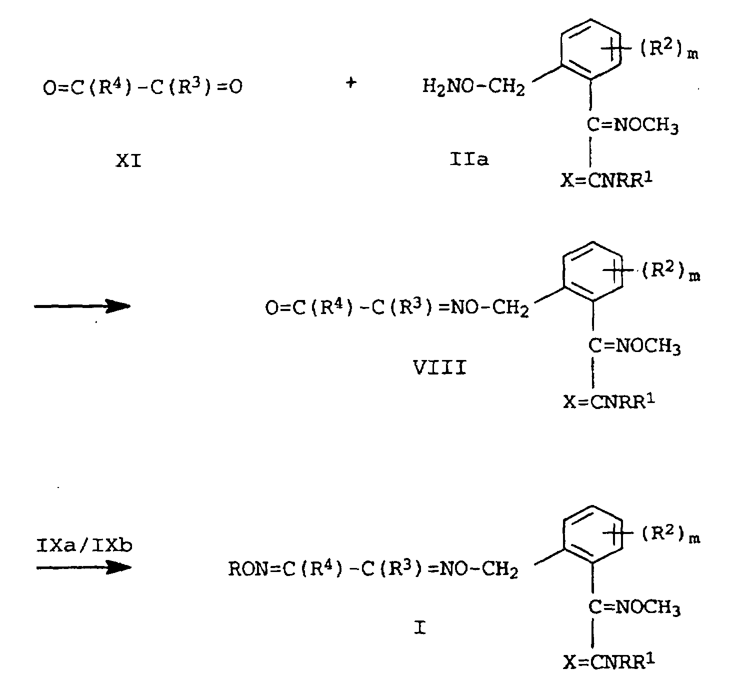
2. Verbindungen, in denen R³ und/oder R⁴ für ein Halogenatom stehen, erhält man aus den entsprechenden Vorstufen, in denen der betreffende Rest für eine Hydroxygruppe steht nach an sich bekannten Methoden (vgl. Houben-Weyl, Vol. E5, S. 631; J. Org. Chem. 36, 233 (1971); J. Org. Chem. 57, 3245 (1992)). Vorzugsweise werden die entsprechenden Umsetzungen zum Halogenderivat auf den Stufen I und VIII vorgenommen.
3. Verbindungen, in denen R³ und/oder R⁴ über ein O-, S- oder N-Atom an das Gerüst gebunden sind, erhält man aus den entsprechenden Vorstufen, in denen der betreffende Rest für ein Halogenatom steht nach an sich bekannten Methoden (vgl. Houben-Weyl, Bd. E5, S. 826 f und 1280 f, J. Org. Chem. 36, 233 (1971), J. Org. Chem. 46, 3623 (1981)). Vorzugsweise werden die entsprechenden Umsetzungen der Halogenderivat auf den Stufen I und VIII vorgenommen.
4. Verbindungen, in denen R³ und/oder R⁴ über ein Sauerstoffatom an das Gerüst gebunden sind, erhält man z.T. auch aus den entsprechenden Vorstufen, in denen der betreffende Rest für eine Hydroxygruppe steht nach an sich bekannten Methoden (vgl. Houben-Weyl, Bd. E5, S. 826-829, Aust. J. Chem. 27, 1341-9 (1974)). Vorzugsweise werden die entsprechenden Umsetzungen zu den Alkoxyderivaten auf den Stufen I und VIII vorgenommen.
5. Verbindungen, in denen R³ nicht Halogen bedeutet, erhält man bevorzugt dadurch, daß man eine Verbindung X gemäß den in EP-A 493 711 beschriebenen Methoden mit einem Lacton XII zunächst in die entsprechende Benzoesäure XIII überführt und XIII über die entsprechenden Halogenide in die Cyanocarbonsäuren XIV überführt, welche dann im Wege der Pinner-Reaktion (Angew. Chem. 94, 1 (1982)) in die α-Ketoester XV überführt werden. Aus den Derivaten XV erhält man durch Amidierung die entsprechenden Carbonsäureamide, die anschließend in die Verbindungen I überführt werden. 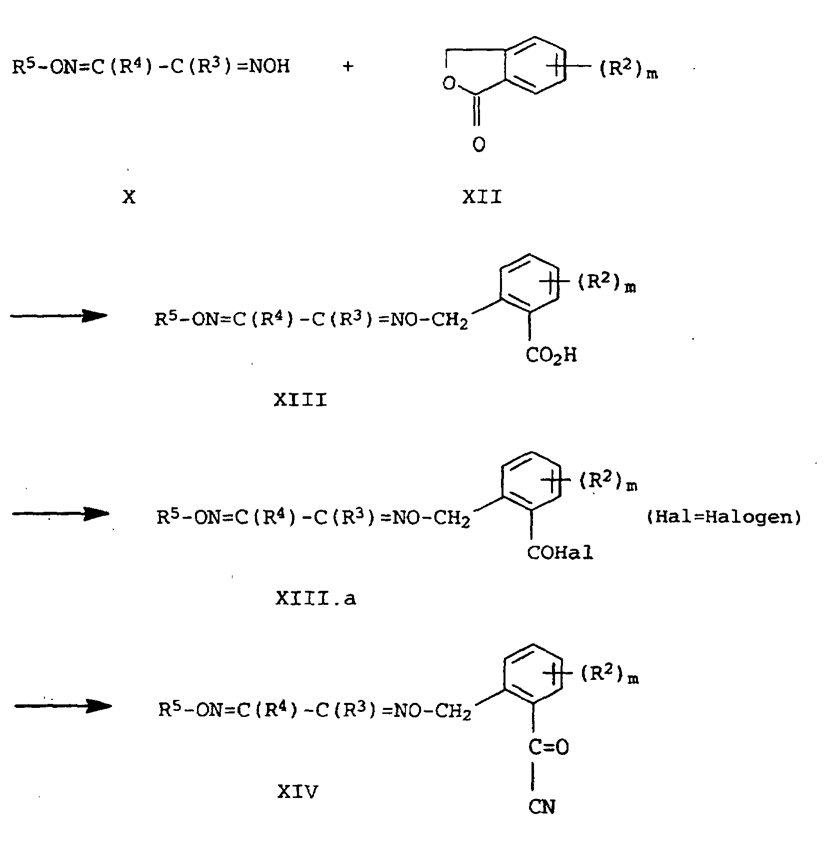 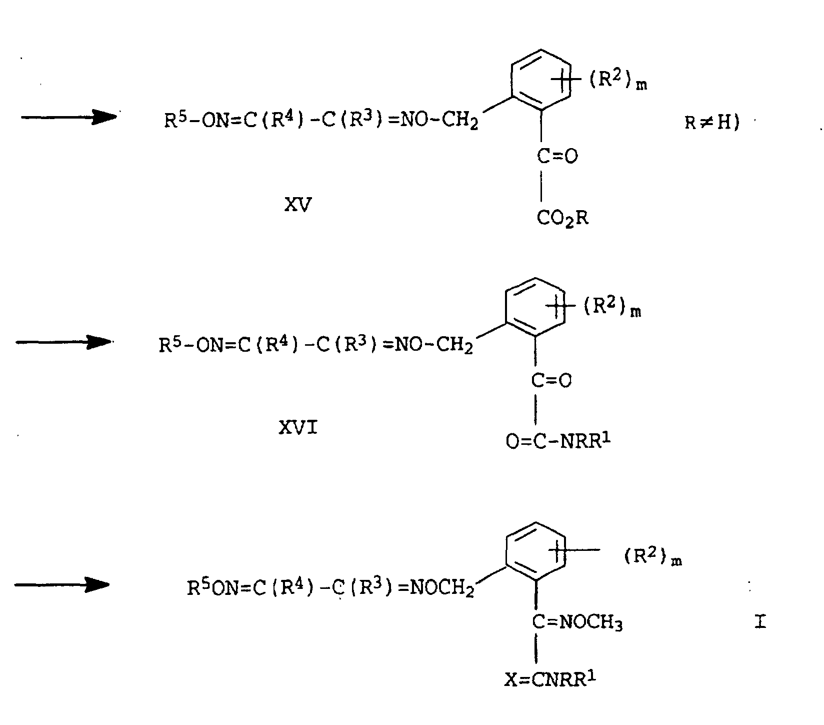
6. In Abwandlung des unter 5. beschriebenen Verfahrens kann man auch direkt aus den Carbonsäurehalogeniden durch Umsetzung mit Isocyanaten und anschließender Hydrolyse direkt die Verbindungen XVI erhalten, in denen R Wasserstoff bedeutet (EP 547 825). 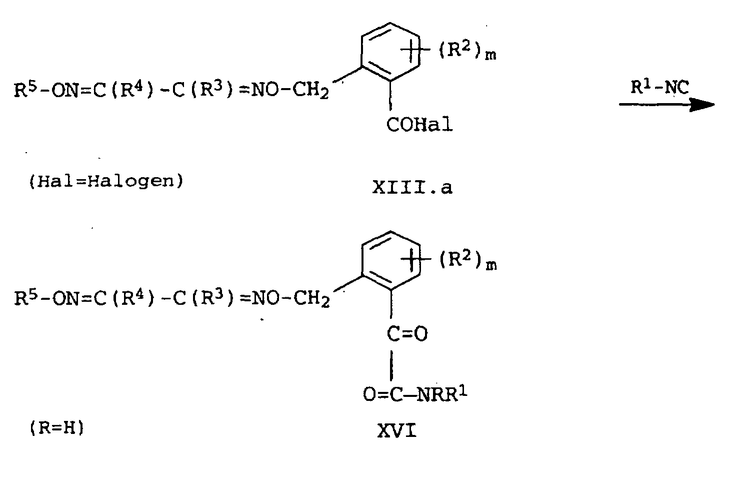
7. In einer weiteren Variante erhält man Verbindungen XVI dadurch, daß man eine ortho-Halogenverbindung nach Metallierung mit Oxalylchlorid in das entsprechende Ketosäurechlorid überführt, welches anschließend mit einem Amin in das entsprechende Amid XVI überführt wird (vgl. J. Org. Chem. 46, 212 f (1981); DE-A 40 42 280; Houben Weyl, Bd. E5, S. 972 f). 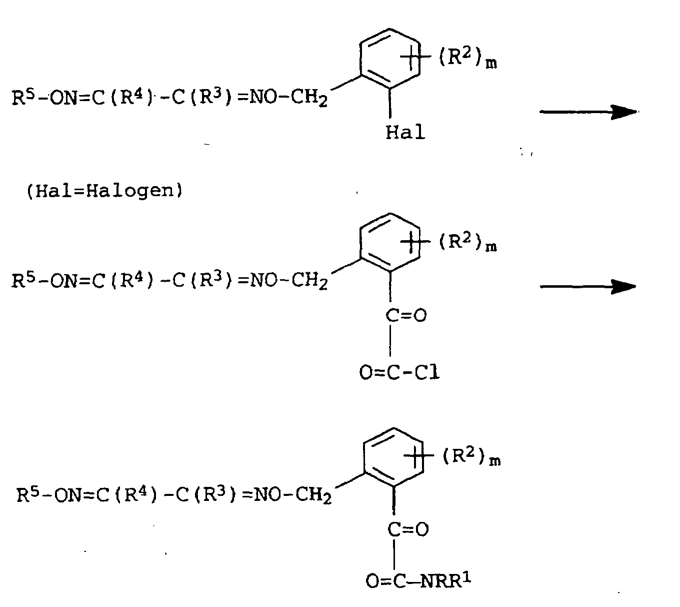
8. In einer weiteren Variante erhält man die Verbindungen.I in denen X Sauerstoff bedeutet, ausgehend von den Ketoestern XV, indem man zunächst die Ketofunktion in den Oximether überführt und den so erhaltenen Oximester mit einem entsprechenden Amin in I überführt (Houben-Weyl, Bd. E5, S. 941 f.) 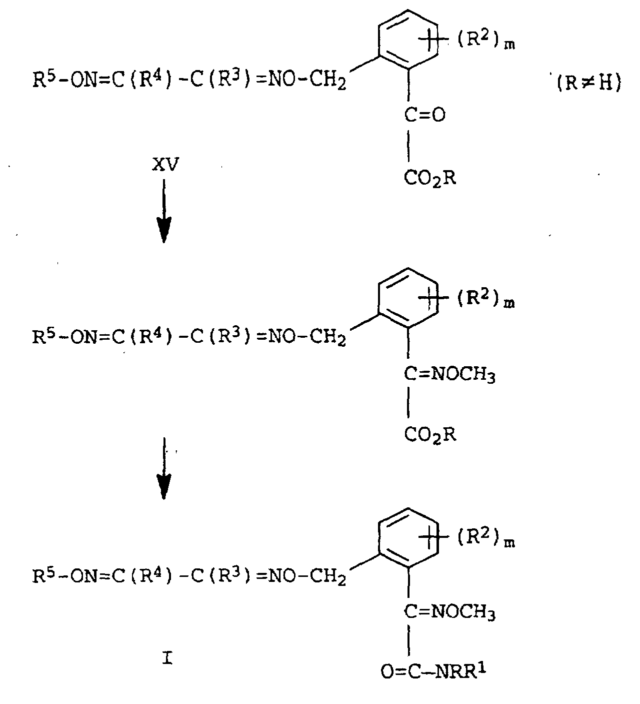
9. Die Verbindungen I, in denen X Schwefel bedeutet, erhält man aus den entsprechenden Amiden I durch Umsetzung mit einem Schwefelungsmittel (z.B. Phosphorsulfid oder Zawessons Reagens; vgl. Houben-Weyl, Bd. IX, 764 f).

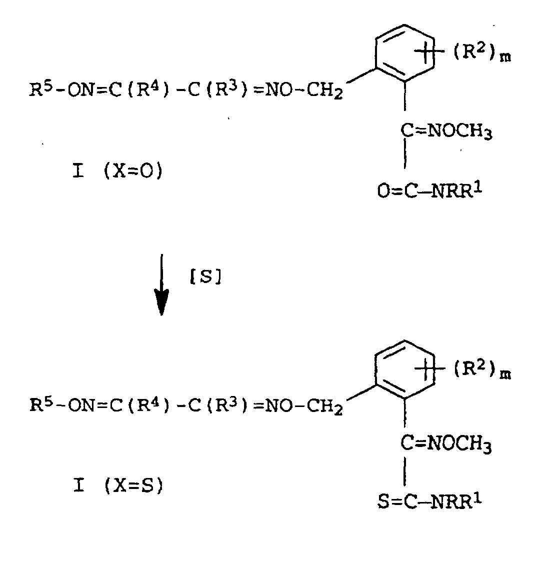

Die Verbindungen II sind bekannt (EP-A 477 631, EP-A 463 488) oder können nach den dort beschriebenen Methoden hergestellt werden.

Die Verbindungen I können bei der Herstellung aufgrund ihrer C=C und C=N Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

In Bezug auf die C=NOCH₃ Doppelbindung werden hinsichtlich ihrer Wirksamkeit die cis-Isomere der Verbindungen I bevorzugt (Konfiguration bezogen auf die -OCH₃ Gruppe im Verhältnis zur -CXNRR¹-Gruppe).

In Bezug auf die -C(R³)=NOCH₂- Doppelbindung werden hinsichtlich ihrer Wirksamkeit die Z-Isomere der Verbindungen I bevorzugt (Konfiguration bezogen auf den Rest R³ im Verhältnis zur -OCH₂-Gruppe).

Bei der eingangs angegebenen Definitionen der Verbindungen I wurden Sammelbegriffe verwendet, die allgemein repräsentativ für die folgenden Gruppen stehen:
**Halogen:** Fluor, Chlor, Brom und Jod;
**Alkyl:** geradkettige oder verzweigte Alkylgruppen mit 1 bis 4, 6 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1- Ethyl-2-methylpropyl;
**Alkylamino:** eine Aminogruppe, welche eine geradkettige oder verzweigte Alkylgruppe mit 1 bis 6 Kohlenstoffatomen wie vorstehend genannt trägt;
**Dialkylamino:** eine Aminogruppe, welche zwei voneinander unabhängige, geradkettige oder verzweigte Alkylgruppen mit jeweils 1 bis 6 Kohlenstoffatomen wie vorstehend genannt, trägt;
**Alkylcarbonyl:** geradkettige oder verzweigte Alkylgruppen mit 1 bis 10 Kohlenstoffatomen, welche über eine Carbonylgruppe (-CO-) an das Gerüst gebunden sind;
**Alkylsulfonyl:** geradkettige oder verzweigte Alkylgruppen mit 1 bis 6 oder 10 Kohlenstoffatomen, welche über eine Sulfonylgruppe (-S(=O)₂-) an das Gerüst gebunden sind;
**Alkylsulfoxyl:** geradkettige oder verzweigte Alkylgruppen mit 1 bis 6 Kohlenstoffatomen, welche über eine Sulfoxylgruppe (-S(=O)-) an das Gerüst gebunden sind;
**Alkylaminocarbonyl:** Alkylaminogruppen mit 1 bis 6 Kohlenstoffatomen wie vorstehend genannt, welche über eine Carbonylgruppe (-CO-) an das Gerüst gebunden sind;
**Dialkylaminocarbonyl:** Dialkylaminogruppen mit jeweils 1 bis 6 Kohlenstoffatomen pro Alkylrest wie vorstehend genannt, welche über eine Carbonylgruppe (-CO-) an das Gerüst gebunden sind;
**Alkylaminothiocarbonyl:** Alkylaminogruppen mit 1 bis 6 Kohlenstoffatomen wie vorstehend genannt, welche über eine Thiocarbonylgruppe (-CS-) an das Gerüst gebunden sind;
**Dialkylaminothiocarbonyl:** Dialkylaminogruppen mit jeweils 1 bis 6 Kohlenstoffatomen pro Alkylrest wie vorstehend genannt, welche über eine Thiocarbonylgruppe (-CS-) an das Gerüst gebunden sind;
**Halogenalkyl:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 4 oder 6 Kohlenstoffatomen wie vorstehend genannt, welche über ein Sauerstoffatom (-O-) an das Gerüst gebunden sind, z.B. C₁-C₆-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy;
**Alkoxycarbonyl:** geradkettige oder verzweigte Alkylgruppen mit 1 bis 6 Kohlenstoffatomen, welche über eine Oxycarbonylgruppe (-OC(=O)-) an das Gerüst gebunden sind;
**Halogenalkoxy:** geradkettige oder verzweigte Alkylgruppen mit 1 bis 6 Kohlenstoffatomen, wobei in diesen Gruppen teilweise oder vollständig die Wasserstoffatome durch Halogenatome.wie vorstehend genannt ersetzt sein können, und wobei diese Gruppen über ein Sauerstoffatom an das Gerüst gebunden sind;
**Alkylthio:** geradkettige oder verzweigte Alkylgruppen mit 1 bis 4 oder 6 Kohlenstoffatomen wie vorstehend genannt, welche über ein Schwefelatom (-S-) an das Gerüst gebunden sind, z.B. C₁-C₆-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
**Cycloalkyl:** monocyclische Alkylgruppen mit 3 bis 6 Kohenstoffringgliedern, z.B. Cyclopropyl, Cyclobutyl, Cyclopentyl und Cyclohexyl;
**Alkenyl:** geradkettige oder verzweigte Alkenylgruppen mit 2 bis 6 oder 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl,. 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1- Ethyl-1-methyl-2-propenyl, 1-Ethyl-2-methyl-1-propenyl und 1-Ethyl-2-methyl-2-propenyl;
**Alkenyloxy:** geradkettige oder verzweigte Alkenylgruppen mit 2 bis 6 Kohlenstoffatomen und einer Doppelbindung in einer beliebigen Position, welche über ein Sauerstoffatom (-O-) an das Gerüst gebunden sind;
**Alkenylcarbonyl:** geradkettige oder verzweigte Alkenylgruppen mit 2 bis 10 Kohlenstoffatomen und einer Doppelbindung in einer beliebigen Position, welche über eine Carbonylgruppe (-CO-) an das Gerüst gebunden sind;
**Alkenylthio bzw. Alkenylamino:** geradkettige oder verzweigte Alkenylgruppen mit 2 bis 6 Kohlenstoffatomen und einer Doppelbindung in einer beliebigen Position, welche (Alkenylthio) über ein Schwefelatom bzw. (Alkenylamino) ein Stickstoffatom an das Gerüst gebunden sind;
**Alkinyl:** geradkettige oder verzweigte Alkinylgruppen mit 2 bis 10 Kohlenstoffatomen und einer Dreifachbindung in einer beliebigen Position, z.B. C₂-C₆-Alkinyl wie Ethinyl, 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
**Alkinylcarbonyl:** geradkettige oder verzweigte Alkinylgruppen mit 3 bis 10 Kohlenstoffatomen und einer Dreifachbindung in einer beliebigen Position, welche über eine Carbonylgruppe (-CO-) an das Gerüst gebunden sind;
**Alkinyloxy bzw. Alkinylthio und Alkinylamino:** geradkettige oder verzweigte Alkinylgruppen mit 2 bis 6 Kohlenstoffatomen und einer Dreifachbindung in einer beliebigen Position, welche (Alkinyloxy) über ein Sauerstoffatom bzw. (Alkinylthio) über ein Schwefelatom oder (Alkinylamino) über ein Stickstoffatom an das Gerüst gebunden sind;
**Cycloalkenyl bzw. Cycloalkenyloxy, Cycloalkenylthio und Cycloalkylamino:** monocyclische Alkenylgruppen mit 3 bis 6 Kohlenstoffringgliedern, welche direkt bzw. (Cycloalkenyloxy) über ein Sauerstoffatom oder (Cycloalkenylthio) ein Schwefelatom oder (Cycloalkenylamino) über ein Stickstoffatom an das Gerüst gebunden sind, z.B. Cyclopropenyl, Cyclobutenyl, Cyclopentenyl oder Cyclohexenyl;
**Cycloalkoxy bzw. Cycloalkylthio und Cycloalkylamino:** monocyclische Alkenylgruppen mit 3 bis 6 Kohlenstoffringgliedern, welche (Cycloalkyloxy) über ein Sauerstoffatom oder (Cycloalkylthio) ein Schwefelatom oder (Cycloalkylamino) über ein Stickstoffatom an das Gerüst gebunden sind, z.B. Cyclopropyl, Cyclobutyl, Cyclopentyl oder Cyclohexyl;
**Heterocyclyl bzw. Heterocyclyloxy, Heterocyclylthio und Heterocyclylamino:** drei- bis sechsgliedrige, gesättigte oder partiell ungesättigte mono- oder polycyclische Heterocyclen, die ein bis drei Hereroatome ausgewählt aus einer Gruppe bestehend aus Sauerstoff, Stickstoff und Schwefel enthalten, und welche direkt bzw. (Heterocyclyloxy) über ein Sauerstoffatom oder (Heterocyclylthio) über ein Schwefelatom oder (Heterocyclylamino) über ein Stickstoffatom an das Gerüst gebunden sind, wie z.B. 2-Tetrahydrofuranyl, Oxiranyl, 3-Tetrahydrofuranyl, 2-Tetrahydrothienyl, 3-Tetrahydrothienyl, 2-Pyrrolidinyl, 3-Pyrrolidinyl, 3-Isoxazoldinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl; 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,3-Dihydro-fur-4-yl, 2,3-Dihydro-fur-5-yl, 2,5-Dihydro-fur-2-yl, 2,5-Dihydro-fur-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydrpisoxazol-4-yl, 4,5-Dihydroisoxazol-5-yl, 2,5-Dihydroisothiazol-3-yl, 2,5-Dihydroisothiazol-4-yl, 2,5-Dihydroisothiazol-5-yl, 2,3-Dihydroisopyrazol-3-yl, 2,3-Dihydroisopyrazol-4-yl, 2,3-Dihydroisopyrazol-5-yl, 4,5-Dihydroisopyrazol-3-yl, 4,5-Dihydroisopyrazol-4-yl, 4,5-Dihydroisopyrazol-5-yl, 2,5-Dihydroisopyrazol-3-yl, 2,5-Dihydroisopyrazol-4-yl, 2,5-Dihydroisopyrazol-5-yl, 2,3-Dihydrooxazol-3-yl, 2,3-Dihydrooxazol-4-yl, 2,3-Dihydrooxazol-5-yl, 4,5-Dihydrooxazol-3-yl, 4,5-Dihydrooxazol-4-yl, 4,5-Dihydrooxazol-5-yl, 2,5-Dihydrooxazol-3-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 1,3-Dioxolan-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl, 1,3-Dithian-2-yl,
**Aryl bzw. Aryloxy, Arylthio, Arylcarbonyl und Arylsulfonyl:** aromatische mono- oder polycyclische Kohenwasserstoffreste welche direkt bzw. (Aryloxy) über ein Sauerstoffatom (-O-) oder (Arylthio) ein Schwefelatom (-S-), (Arylcarbonyl) über eine Carbonylgruppe (-CO-) oder (Arylsulfonyl) über eine Sulfonylgruppe (-SO₂-) an das Gerüst gebunden sind, z.B. Phenyl, Naphthyl und Phenanthrenyl bzw. Phenyloxy, Naphthyloxy und Phenanthrenyloxy und die entsprechenden Carbonyl- und Sulfonylreste;
**Arylamino:** aromatische mono- oder polycyclische Kohlenwasserstoffreste, welche über ein Stickstoffatom an das Gerüst gebunden sind;
**Hetaryl bzw. Hetaryloxy, Hetarylthio, Hetarylcarbonyl und Hetarylsulfonyl:** aromatische mono- oder polycyclische Reste welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können und welche direkt bzw. (Hetaryloxy) über ein Sauerstoffatom (-O-) oder (Hetarylthio) ein Schwefelatom (-s-), (Hetarylcarbonyl) über eine Carbonylgruppe (-CO-) oder (Hetarylsulfonyl) über eine Sulfonylgruppe (-SO-) an das Gerüst gebunden sind, z.B.
   - 5-gliedriges Heteroaryl, enthaltend ein bis drei Stickstoffatome: 5-Ring Heteroarylgruppen, welche neben Kohlenstoffatomen ein bis drei Stickstoffatome als Ringglieder enthalten können, z.B. 2-Pyrrolyl, 3-Pyrrolyl, 3-Pyrazolyl, 4-Pyrazolyl, 5-Pyrazolyl, 2-Imidazolyl, 4-Imidazolyl, 1,2,4-Triazol-3-yl und 1,3,4-Triazol-2-yl;
   - 5-gliedriges Heteroaryl, enthaltend ein bis vier Stickstoffatome oder ein bis drei Stickstoffatome und ein Schwefel - oder Sauerstoffatom oder ein Sauerstoff oder ein Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol -3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl;
   - benzokondensiertes 5-gliedriges Heteroaryl, enthaltend ein bis drei Stickstoffatome oder ein Stickstoffatom und/oder ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder ein Schwefelatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
   - über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien- 1,4-diylgruppe verbrückt sein können, wobei diese Ringe über eines der Stickstoffringglieder an das Gerüst gebunden sind;
   - 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazinyl;
   - benzokondensiertes 6-gliedriges Heteroaryl, enthaltend ein bis vier Stickstoffatome: 6-Ring Heteroarylgruppen in welchen zwei benachbarte Kohlenstoffringglieder durch eine Buta-1,3-dien-1,4-diylgruppe verbrückt sein können, z.B. Chinolin, Isochinolin, Chinazolin und Chinoxalin,
bzw. die entsprechenden Oxy-, Thio-, Carbonyl- oder Sulfonylgruppen.

**Hetarylamino:** aromatische mono- oder polycyclische Reste, welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können und welche über ein Stickstoffatom an das Gerüst gebunden sind.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome wie vorstehend genannt ersetzt sein können.

Im Hinblick auf ihre biologische Wirkung sind Verbindungen der Formel I bevorzugt, in denen m für 0 steht.

Gleichermaßen bevorzugt sind Verbindungen der Formel I, in denen R für Wasserstoff oder Methyl steht.

Gleichermaßen bevorzugt sind Verbindungen der Formel I, in denen R¹ für Wasserstoff oder Methyl steht.

Besonders bevorzugt sind Verbindungen der Formel I, in denen R Wasserstoff und R¹ Wasserstoff oder Methyl bedeutet.

Besonders bevorzugt sind auch Verbindungen der Formel I, in denen R und R¹ gleichzeitig Wasserstoff oder Methyl bedeuten.

Daneben werden Verbindungen I bevorzugt, in denen R³ für Wasserstoff, Hydroxy, Cyclopropyl, Chlor, Methyl, Methoxy, Methylthio oder Phenyl steht.

Außerdem werden Verbindungen I bevorzugt, in denen R³ für Methyl steht.

Des weiteren werden Verbindungen I bevorzugt, in denen R³ für Methoxy steht.

Außerdem werden Verbindungen I bevorzugt, in denen R³ für Hydroxy steht.

Des weiteren werden Verbindungen I bevorzugt, in.denen R³ für Chlor steht.

Des weiteren werden Verbindungen I bevorzugt, in denen R⁴ für Wasserstoff, Hydroxy, Cyclopropyl, Chlor, Methyl, Ethyl, iso-Propyl, Methoxy oder Methyl thio steht.

Außerdem werden Verbindungen I bevorzugt, in denen R⁴ für Methyl steht.

Des weiteren werden Verbindungen I bevorzugt, in denen R⁴ für Methoxy steht.

Außerdem werden Verbindungen I bevorzugt, in denen R⁴ für Hydroxy steht.

Des weiteren werden Verbindungen I bevorzugt, in denen R⁴ für Ethyl steht.

Außerdem werden Verbindungen I bevorzugt, in denen R⁴ für iso-Propyl steht.

Außerdem werden Verbindungen I bevorzugt, in denen R⁴ für Cyclopropyl steht.

Des weiteren werden Verbindungen I bevorzugt, in denen R⁴ für ggf. subst. Aryl oder Hetaryl steht.

Des weiteren werden Verbindungen I bevorzugt, in denen R⁴ für ggf. subst. Pyridyl, Pyrimidyl, Pyrazinyl, Pyridazinyl oder Triazinyl steht.

Des weiteren werden Verbindungen I bevorzugt, in denen R⁴ für ggf. subst. Furyl, Thienyl oder Pyrrolyl steht.

Des weiteren werden Verbindungen I bevorzugt, in denen R⁴ für ggf. subst. Oxazolyl, Thiazolyl, Isoxazolyl, Isothiazolyl, Pyrazolyl oder Imidazolyl steht.

Des weiteren werden Verbindungen I bevorzugt, in denen R⁴ für ggf. subst. Oxdiazolyl, Thiadiazolyl oder Triazolyl steht.

Außerdem werden Verbindungen I bevorzugt, in denen R⁴ für Phenyl steht, welches unsubstituiert ist oder ein oder zwei der folgenden Gruppen trägt: Nitro, Cyano, Hydroxy, Amino, Aminocarbonyl, Aminothiocarbonyl, Halogen, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-Alkylamino, C₁-C₄-Alkylsulfonyl, C₁-C₄-Alkoxycarbonyl, C₁-C₄-Alkylaminocarbonyl or Di-C₁-C₄-Alkylaminocarbonyl.

Außerdem werden Verbindungen I bevorzugt, in denen R⁵ für Wasserstoff, C₁-C₆-Alkyl, Arylalkyl, Hetarylalkyl, Aryloxyalkyl, Hetaryloxyalkyl, Aryl oder Hetaryl steht.

Des weiteren werden Verbindungen I bevorzugt, in denen R⁵ für C₁-C₆-Alkyl steht.

Außerdem werden Verbindungen I bevorzugt, in denen R⁵ für Methyl oder Ethyl steht.

Des weiteren werden Verbindungen I bevorzugt, in denen R⁵ für Arylalkyl oder Hetarylalkyl steht.

Außerdem werden Verbindungen I bevorzugt, in denen R⁵ für Aryloxyalkyl oder Hetaryloxyalkyl steht.

Außerdem werden Verbindungen I bevorzugt, in denen R⁵ für Aryl oder Hetaryl steht.

Daneben werden Verbindungen der Formel I bevorzugt, in denen X für O steht.

Daneben werden Verbindungen der Formel I bevorzugt, in denen X für S steht.

Insbesondere sind im Hinblick auf ihre Verwendung die in den folgenden Tabellen zusammengestellten Verbindungen I bevorzugt.

### Tabelle 1:

Verbindungen der allgemeinen Formel I.1, in denen (R²)ₘ Wasserstoff bedeutet und die Kombination der Substituenten R³, R⁴ und R⁵ für eine Verbindung jeweils einer Zeile der Tabelle A entspricht

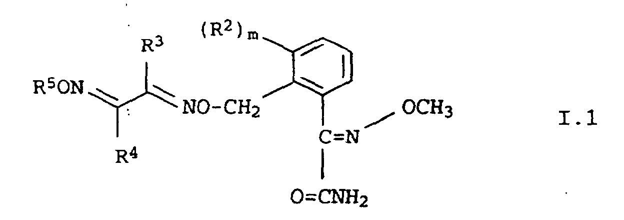

### Tabelle 2

Verbindungen der allgemeinen Formel I.1, in denen (R²)ₘ Chlor bedeutet und die Kombination der Substituenten R³, R⁴ und R⁵ für eine Verbindung jeweils einer Zeile der Tabelle A entspricht

### Tabelle 3:

Verbindungen der allgemeinen Formel I.2, in denen (R²)ₘ Wasserstoff bedeutet und die Kombination der Substituenten R³, R⁴ und R⁵ für eine Verbindung jeweils einer Zeile der Tabelle A entspricht

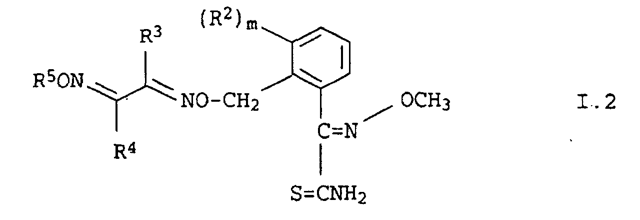

### Tabelle 4:

Verbindungen der allgemeinen Formel I.2, in denen (R²)ₘ Chlor bedeutet und die Kombination der Substituenten R³, R⁴ und R⁵ für eine Verbindung jeweils einer Zeile der Tabelle A entspricht

### Tabelle 5:

Verbindungen der allgemeinen Formel I.3, in denen (R²)ₘ Wasserstoff bedeutet und die Kombination der Substituenten R³, R⁴ und R⁵ für eine Verbindung jeweils einer Zeile der Tabelle A entspricht

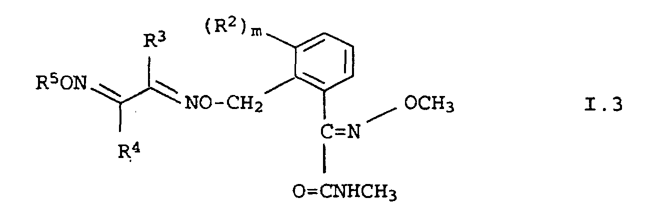

### Tabelle 6:

Verbindungen der allgemeinen Formel I.3, in denen (R²)ₘ Chlor bedeutet und die Kombination der Substituenten R³, R⁴ und R⁵ für eine Verbindung jeweils einer Zeile der Tabelle A entspricht

### Tabelle 7:

Verbindungen der allgemeinen Formel I.4, in denen (R²)ₘ Wasserstoff bedeutet und die Kombination der Substituenten R³, R⁴ und R⁵ für eine Verbindung jeweils einer Zeile der Tabelle A entspricht

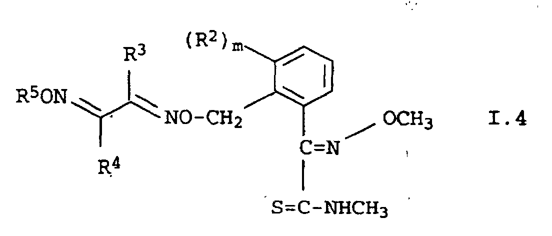

**Tabelle A**

| Nr. | R³ | R⁴ | R⁵ |
|---|---|---|---|
| 17 | CH₃ | CH₃ | n-C₈H₁₇ |
| 32 | CH₃ | CH₃ | 3-Methoxyprop-1-yl |
| 33 | CH₃ | CH₃ | 3-Ethoxyprop-1-yl |
| 34 | CH₃ | CH₃ | 3-Isopropoxyprop-1-yl |
| 35 | CH₃ | CH₃ | 4-Methoxybut-1-yl |
| 36 | CH₃ | CH₃ | 4-Isopropoxybut-1-yl |
| 37 | CH₃ | CH₃ | Propen-3-yl |
| 38 | CH₃ | CH₃ | But-2-en-1-yl |
| 39 | CH₃ | CH₃ | 3-Methylbut-2-en-1-yl |
| 40 | CH₃ | CH₃ | 2-Vinyloxyeth-1-yl |
| 41 | CH₃ | CH₃ | Allyloxyeth-1-yl |
| 42 | CH₃ | CH₃ | 2-Trifluormethoxyeth-1-yl |
| 43 | CH₃ | CH₃ | 3-Trifluormethoxyprop-1-yl |
| 44 | CH₃ | CH₃ | 4-Difluormethoxybut-1-yl |
| 45 | CH₃ | CH₃ | Hydroxycarbonylmethyl |
| 47 | CH₃ | CH₃ | Aminocarbonylmethyl |
| 48 | CH₃ | CH₃ | N-Methylaminocarbonylmethyl |
| 49 | CH₃ | CH₃ | N,N-Dimethylaminocarbonylmethyl |
| 50 | CH₃ | CH₃ | 2-Hydroxycarbonyleth-1-yl |
| 52 | CH₃ | CH₃ | 2-Aminocarbonyleth-1-yl |
| 53 | CH₃ | CH₃ | 2-N-Methylaminocarbonyleth-1-yl |
| 54 | CH₃ | CH₃ | 2-Dimethylaminocarbonyleth-1-yl |
| 55 | CH₃ | CH₃ | 2-Aminoeth-1-yl |
| 56 | CH₃ | CH₃ | 2-Aminoprop-1-yl |
| 57 | CH₃ | CH₃ | 4-Aminobut-1-yl |
| 58 | CH₃ | CH₃ | 3-Dimethylaminoprop-1-yl |
| 59 | CH₃ | CH₃ | 4-Aminothiocarbonylbut-1-yl |
| 60 | CH₃ | CH₃ | 2-Oxopropyl |
| 64 | CH₃ | CH₃ | 2-Methoxyiminoprop-1-yl |
| 65 | CH₃ | CH₃ | 2-Methoxyiminoeth-1-yl |
| 66 | CH₃ | CH₃ | 6-Aminocarbonylhex-1-yl |
| 67 | CH₃ | CH₃ | 3-Aminothiocarbonylprop-1-yl |
| 68 | CH₃ | CH₃ | 2-Aminothiocarbonyleth-1-yl |
| 69 | CH₃ | CH₃ | Aminothiocarbonylmethyl |
| 70 | CH₃ | CH₃ | 4-(N,N-Dimethylamino)but-1-yl |
| 71 | CH₃ | CH₃ | 2-(Methylthio)eth-1-yl |
| 72 | CH₃ | CH₃ | 2-(Methylsulfonyl)eth-1-yl |
| 73 | CH₃ | CH₃ | 4-(Methylthio)prop-1-yl |
| 74 | CH₃ | CH₃ | 4-(Methylsulfonyl)prop-1-yl |
| 157 | CH₃ | CH₃ | 2-Cyclohexyl-C₆H₄-CH₂ |
| 158 | CH₃ | CH₃ | 3-Cyclohexyl-C₆H₄-CH₂ |
| 159 | CH₃ | CH₃ | 4-Cyclohexyl-C₆H₄-CH₂ |
| 160 | CH₃ | CH₃ | 2-Vinyl-C₆H₄-CH₂ |
| 161 | CH₃ | CH₃ | 3-Vinyl-C₆H₄-CH₂ |
| 162 | CH₃ | CH₃ | 4-Vinyl-C₆H₄-CH₂ |
| 163 | CH₃ | CH₃ | 2-Allyl-C₆H₄-CH₂ |
| 164 | CH₃ | CH₃ | 3-Allyl-C₆H₄-CH₂ |
| 165 | CH₃ | CH₃ | 4-Allyl-C₆H₄-CH₂ |
| 166 | CH₃ | CH₃ | 2-C₆H₅-C₆H₄-CH₂ |
| 167 | CH₃ | CH₃ | 3-C₆H₅-C₆H₄-CH₂ |
| 168 | CH₃ | CH₃ | 4-C₆H₅-C₆H₄-CH₂ |
| 170 | CH₃ | CH₃ | 2-OH-C₆H₄-CH₂ |
| 171 | CH₃ | CH₃ | 3-OH-C₆H₄-CH₂ |
| 172 | CH₃ | CH₃ | 4-OH-C₆H₄-CH₂ |
| 194 | CH₃ | CH₃ | 2-O-Allyl-C₆H₄-CH₂ |
| 195 | CH₃ | CH₃ | 3-O-Allyl-C₆H₄-CH₂ |
| 196 | CH₃ | CH₃ | 4-O-Allyl-C₆H₄-CH₂ |
| 200 | CH₃ | CH₃ | 2-Acetyl-C₆H₄-CH₂ |
| 201 | CH₃ | CH₃ | 3-Acetyl-C₆H₄-CH₂ |
| 202 | CH₃ | CH₃ | 4-Acetyl-C₆H₄-CH₂ |
| 203 | CH₃ | CH₃ | 2-Methoxycarbonyl-C₆H₄-CH₂ |
| 204 | CH₃ | CH₃ | 3-Methoxycarbonyl-C₆H₄-CH₂ |
| 205 | CH₃ | CH₃ | 4-Methoxycarbonyl-C₆H₄-CH₂ |
| 206 | CH₃ | CH₃ | 2-Aminocarbonyl-C₆H₄-CH₂ |
| 207 | CH₃ | CH₃ | 3-Aminocarbonyl-C₆H₄-CH₂ |
| 208 | CH₃ | CH₃ | 4-Aminocarbonyl-C₆H₄-CH₂ |
| 209 | CH₃ | CH₃ | 2-Dimethylaminocarbonyl-C₆H₄-CH₂ |
| 210 | CH₃ | CH₃ | 3-Dimethylaminocarbonyl-C₆H₄-CH₂ |
| 211 | CH₃ | CH₃ | 4-Dimethylaminocarbonyl-C₆H₄-CH₂ |
| 212 | CH₃ | CH₃ | 2-(N-Methylaminocarbonyl)-C₆H₄-CH₂ |
| 213 | CH₃ | CH₃ | 3-(N-Methylaminocarbonyl)-C₆H₄-CH₂ |
| 214 | CH₃ | CH₃ | 4-(N-Methylaminocarbonyl)-C₆H₄-CH₂ |
| 215 | CH₃ | CH₃ | 2-H₂N-C₆H₄-CH₂ |
| 216 | CH₃ | CH₃ | 3-H₂N-C₆H₄-CH₂ |
| 217 | CH₃ | CH₃ | 4-H₂N-C₆H₄-CH₂ |
| 218 | CH₃ | CH₃ | 2-Aminothiocarbonyl-C₆H₄-CH₂ |
| 219 | CH₃ | CH₃ | 3-Aminothiocarbonyl-C₆H₄-CH₂ |
| 220 | CH₃ | CH₃ | 4-Aminothiocarbonyl-C₆H₄-CH₂ |
| 221 | CH₃ | CH₃ | 2-Methoxyiminomethyl-C₆H₄-CH₂ |
| 222 | CH₃ | CH₃ | 3-Methoxyiminomethyl-C₆H₄-CH₂ |
| 223 | CH₃ | CH₃ | 4-Methoxyiminomethyl-C₆H₄-CH₂ |
| 224 | CH₃ | CH₃ | 2-Formyl-C₆H₄-CH₂ |
| 225 | CH₃ | CH₃ | 3-Formyl-C₆H₄-CH₂ |
| 226 | CH₃ | CH₃ | 4-Formyl-C₆H₄-CH₂ |
| 227 | CH₃ | CH₃ | 2-(1'-Methoxyiminoeth-1'-yl)-C₆H₄-CH₂ |
| 228 | CH₃ | CH₃ | 3-(1'-Methoxyiminoeth-1'-yl)-C₆H₄-CH₂ |
| 229 | CH₃ | CH₃ | 4-(1'-Methoxyiminoeth-1'-yl)-C₆H₄-CH₂ |
| 230 | CH₃ | CH₃ | 2-SCH₃-C₆H₄-CH₂ |
| 231 | CH₃ | CH₃ | 3-SCH₃-C₆H₄-CH₂ |
| 232 | CH₃ | CH₃ | 4-SCH₃-C₆H₄-CH₂ |
| 233 | CH₃ | CH₃ | 2-SO₂CH₃-C₆H₄-CH₂ |
| 234 | CH₃ | CH₃ | 3-SO₂CH₃-C₆H₄-CH₂ |
| 235 | CH₃ | CH₃ | 4-SO₂CH₃-C₆H₄-CH₂ |
| 236 | CH₃ | CH₃ | 2-OCF₃-C₆H₄-CH₂ |
| 237 | CH₃ | CH₃ | 3-OCF₃-C₆H₄-CH₂ |
| 238 | CH₃ | CH₃ | 4-OCF₃-C₆H₄-CH₂ |
| 239 | CH₃ | CH₃ | 2-OCHF₂-C₆H₄-CH₂ |
| 240 | CH₃ | CH₃ | 3-OCHF₂-C₆H₄-CH₂ |
| 241 | CH₃ | CH₃ | 4-OCHF₂-C₆H₄-CH₂ |
| 242 | CH₃ | CH₃ | 3-CF₃, 4-OCF₃-C₆H₃-CH₂ |
| 243 | CH₃ | CH₃ | 1-Naphthyl-CH₂ |
| 244 | CH₃ | CH₃ | 2-Naphthyl-CH₂ |
| 245 | CH₃ | CH₃ | 2-Phenoxyeth-1-yl |
| 246 | CH₃ | CH₃ | 2-(2'-Chlorphenoxy)eth-1-yl |
| 247 | CH₃ | CH₃ | 2-(3'-Chlorphenoxy)eth-1-yl |
| 248 | CH₃ | CH₃ | 2-(4'-Chlorphenoxy)eth-1-yl |
| 249 | CH₃ | CH₃ | 2-(3',5'-Dichlorphenoxy)eth-1-yl |
| 250 | CH₃ | CH₃ | 2-(2'-Cyanophenoxy)eth-1-yl |
| 251 | CH₃ | CH₃ | 2-(3'-Cyanophenoxy)eth-1-yl |
| 252 | CH₃ | CH₃ | 2-(4'-Cyanophenoxy)eth-1-yl |
| 253 | CH₃ | CH₃ | 2-(2'-Methylphenoxy)eth-1-yl |
| 254 | CH₃ | CH₃ | 2-(3'-Methylphenoxy)eth-1-yl |
| 255 | CH₃ | CH₃ | 2-(4'-Methylphenoxy)eth-1-yl |
| 256 | CH₃ | CH₃ | 2-(3'-t-Butylphenoxy)eth-1-yl |
| 257 | CH₃ | CH₃ | 2-(4'-t-Butylphenoxy)eth-1-yl |
| 258 | CH₃ | CH₃ | 2-(2'-Nitrophenoxy)eth-1-yl |
| 259 | CH₃ | CH₃ | 2-(3'-Nitrophenoxy)eth-1-yl |
| 260 | CH₃ | CH₃ | 2-(4'-Nitrophenoxy)eth-1-yl |
| 261 | CH₃ | CH₃ | 2-(2'-Methoxyphenoxy)eth-1-yl |
| 262 | CH₃ | CH₃ | 2-(3'-Methoxyphenoxy)eth-1-yl |
| 263 | CH₃ | CH₃ | 2-(4'-Methoxyphenoxy)eth-1-yl |
| 264 | CH₃ | CH₃ | 2-(2'-Trifluormethylphenoxy)eth-1-yl |
| 265 | CH₃ | CH₃ | 2-(3'-Trifluormethylphenoxy)eth-1-yl |
| 266 | CH₃ | CH₃ | 2-(4'-Trifluormethylphenoxy)eth-1-yl |
| 267 | CH₃ | CH₃ | 2-(2'-Acetylphenoxy)eth-1-yl |
| 268 | CH₃ | CH₃ | 2-(3'-Acetylphenoxy)eth-1-yl |
| 269 | CH₃ | CH₃ | 2-(4'-Acetylphenoxy)eth-1-yl |
| 273 | CH₃ | CH₃ | 2-(2'-Dimethylaminocarbonyl)eth-1-yl |
| 274 | CH₃ | CH₃ | 2-(3'-Dimethylaminocarbonyl)eth-1-yl |
| 275 | CH₃ | CH₃ | 2-(4'-Dimethylaminocarbonyl)eth-1-yl |
| 276 | CH₃ | CH₃ | 2-(2'-Aminothiocarbonyl)eth-1-yl |
| 277 | CH₃ | CH₃ | 2-(3'-Aminothiocarbonyl)eth-1-yl |
| 278 | CH₃ | CH₃ | 2-(4'-Aminothiocarbonyl)eth-1-yl |
| 279 | CH₃ | CH₃ | 2-(2'-Methylsulfonyl)eth-1-yl |
| 280 | CH₃ | CH₃ | 2-(3'-Methylsulfonyl)eth-1-yl |
| 281 | CH₃ | CH₃ | 2-(4'-Methylsulfonyl)eth-1-yl |
| 282 | CH₃ | CH₃ | 3-Phenoxyprop-1-yl |
| 283 | CH₃ | CH₃ | 3-(2'-Chlorphenoxy)prop-1-yl |
| 284 | CH₃ | CH₃ | 3- (3'-Chlorphenoxy)prop-1-yl |
| 285 | CH₃ | CH₃ | 3-(4'-Chlorphenoxy)prop-1-yl |
| 286 | CH₃ | CH₃ | 3-(3',5',Dichlorphenoxy)prop-1-yl |
| 287 | CH₃ | CH₃ | 3-(2'-Cyanophenoxy)prop-1-yl |
| 288 | CH₃ | CH₃ | 3-(3'-Cyanophenoxy)prop-1-yl |
| 289 | CH₃ | CH₃ | 3-(4'-Cyanophenoxy)prop-1-yl |
| 290 | CH₃ | CH₃ | 3-(2'-Methylphenoxy)prop-1-yl |
| 291 | CH₃ | CH₃ | 3-(3'-Methylphenoxy)prop-1-yl |
| 292 | CH₃ | CH₃ | 3-(4'-Methylphenoxy)prop-1-yl |
| 293 | CH₃ | CH₃ | 3-(2'-Methoxyphenoxy)prop-1-yl |
| 294 | CH₃ | CH₃ | 3-(3'-Methoxyphenoxy)prop-1-yl |
| 295 | CH₃ | CH₃ | 3-(4'-Methoxyphenoxy)prop-1-yl |
| 296 | CH₃ | CH₃ | 3-(2'-Trifluormethylphenoxy)prop-1-yl |
| 297 | CH₃ | CH₃ | 3-(3'-Trifluormethylphenoxy)prop-1-yl |
| 298 | CH₃ | CH₃ | 3-(4'-Trifluormethylphenoxy)prop-1-yl |
| 299 | CH₃ | CH₃ | 4-Phenoxybut-1-yl |
| 325 | CH₃ | CH₃ | 4-Phenylbut-1-yl |
| 326 | CH₃ | CH₃ | 4-(4'-Chlorphenyl)but-1-yl |
| 327 | CH₃ | CH₃ | 6-(4'-Chlorphenyl)hex-1-yl |
| 328 | CH₃ | CH₃ | 2-Pyridylmethyl |
| 329 | CH₃ | CH₃ | 3-Pyridylmethyl |
| 330 | CH₃ | CH₃ | 4-Pyridylmethyl |
| 331 | CH₃ | CH₃ | 4-Chlorpyridin-2-ylmethyl |
| 332 | CH₃ | CH₃ | 5-Chlorpyridin-2-ylmethyl |
| 333 | CH₃ | CH₃ | 6-Chlorpyridin-2-ylmethyl |
| 334 | CH₃ | CH₃ | 5-Chlorpyridin-3-ylmethyl |
| 335 | CH₃ | CH₃ | 6-Chlorpyridin-3-ylmethyl |
| 336 | CH₃ | CH₃ | 2-Chlorpyridin-4-ylmethyl |
| 337 | CH₃ | CH₃ | 2-Pyrimidinylmethyl |
| 338 | CH₃ | CH₃ | 4-Chlorpyrimidin-2-ylmethyl |
| 339 | CH₃ | CH₃ | 5-Chlorpyrimidin-2-ylmethyl |
| 340 | CH₃ | CH₃ | 2-Chlorpyrimidin-4-ylmethyl |
| 341 | CH₃ | CH₃ | 6-Chlorpyrimidin-4-ylmethyl |
| 342 | CH₃ | CH₃ | 2-Chlorpyrimidin-5-ylmethyl |
| 343 | CH₃ | CH₃ | 4-Pyridazinylmethyl |
| 344 | CH₃ | CH₃ | 2-Pyrazinylmethyl |
| 345 | CH₃ | CH₃ | 5-Chlorpyrazin-2-ylmethyl |
| 346 | CH₃ | CH₃ | 6-Chlorpyrazin-2-ylmethyl |
| 347 | CH₃ | CH₃ | 3-Pyridazinylmethyl |
| 348 | CH₃ | CH₃ | 6-Chlorpyridazin-3-ylmethyl |
| 349 | CH₃ | CH₃ | 1,3,5-Triazinylmethyl |
| 350 | CH₃ | CH₃ | 2-Furylmethyl |
| 351 | CH₃ | CH₃ | 3-Furylmethyl |
| 352 | CH₃ | CH₃ | 4-Bromfur-2-ylmethyl |
| 353 | CH₃ | CH₃ | 5-Chlorfur-2-ylmethyl |
| 354 | CH₃ | CH₃ | 2-Thienylmethyl |
| 355 | CH₃ | CH₃ | 3-Thienylmethyl |
| 356 | CH₃ | CH₃ | 5-Methylthien-3-ylmethyl |
| 357 | CH₃ | CH₃ | 5-Chlorthien-2-ylmethyl |
| 358 | CH₃ | CH₃ | 2-Chlorthien-4-ylmethyl |
| 359 | CH₃ | CH₃ | 2-Pyrrolylmethyl |
| 360 | CH₃ | CH₃ | 3-Pyrrolylmethyl |
| 361 | CH₃ | CH₃ | 2-Oxazolylmethyl |
| 362 | CH₃ | CH₃ | 4-Methyloxazol-2-ylmethyl |
| 363 | CH₃ | CH₃ | 5-Methyloxazol-2-ylmethyl |
| 364 | CH₃ | CH₃ | 4-Chloroxazol-2-ylmethyl |
| 365 | CH₃ | CH₃ | 5-Chloroxazol-2-ylmethyl |
| 366 | CH₃ | CH₃ | 4-Oxazolylmethyl |
| 367 | CH₃ | CH₃ | 2-Methyloxazol-4-ylmethyl |
| 368 | CH₃ | CH₃ | 5-Methyloxazol-4-ylmethyl |
| 369 | CH₃ | CH₃ | 2-Chloroxazol-4-ylmethyl |
| 370 | CH₃ | CH₃ | 5-Chloroxazol-4-ylmethyl |
| 371 | CH₃ | CH₃ | 5-Oxazolylmethyl |
| 372 | CH₃ | CH₃ | 2-Methyloxazol-5-ylmethyl |
| 373 | CH₃ | CH₃ | 4-Methyloxazol-5-ylmethyl |
| 374 | CH₃ | CH₃ | 2-Chloroxazol-5-ylmethyl |
| 375 | CH₃ | CH₃ | 4-Chloroxazol-5-ylmethyl |
| 376 | CH₃ | CH₃ | 2-Thiazolylmethyl |
| 377 | CH₃ | CH₃ | 4-Methylthiazol-2-ylmethyl |
| 378 | CH₃ | CH₃ | 5-Methylthiazol-2-ylmethyl |
| 379 | CH₃ | CH₃ | 4-Chlorthiazol-2-ylmethyl |
| 380 | CH₃ | CH₃ | 5-Chlorthiazol-2-ylmethyl |
| 381 | CH₃ | CH₃ | 4-Thiazolylmethyl |
| 382 | CH₃ | CH₃ | 2-Methylthiazol-4-ylmethyl |
| 383 | CH₃ | CH₃ | 5-Methylthiazol-4-ylmethyl |
| 384 | CH₃ | CH₃ | 2-Chlorthiazol-4-ylmethyl |
| 385 | CH₃ | CH₃ | 5-Chlorthiazol-4-ylmethyl |
| 386 | CH₃ | CH₃ | 5-Thiazolylmethyl |
| 387 | CH₃ | CH₃ | 2-Methylthiazol-5-ylmethyl |
| 388 | CH₃ | CH₃ | 4-Methylthiazol-5-ylmethyl |
| 389 | CH₃ | CH₃ | 2-Chlorthiazol-5-ylmethyl |
| 390 | CH₃ | CH₃ | 4-Chlorthiazol-5-ylmethyl |
| 391 | CH₃ | CH₃ | 3-Isoxazolylmethyl |
| 392 | CH₃ | CH₃ | 4-Methylisoxazol-3-ylmethyl |
| 393 | CH₃ | CH₃ | 5-Methylisoxazol-3-ylmethyl |
| 394 | CH₃ | CH₃ | 4-Chlorisoxazol-3-ylmethyl |
| 395 | CH₃ | CH₃ | 5-Chlorisoxazol-3-ylmethyl |
| 396 | CH₃ | CH₃ | 4-Isoxazolylmethyl |
| 397 | CH₃ | CH₃ | 3-Methylisoxazol-4-ylmethyl |
| 398 | CH₃ | CH₃ | 5-Methylisoxazol-4-ylmethyl |
| 399 | CH₃ | CH₃ | 3-Chlorisoxazol-4-ylmethyl |
| 400 | CH₃ | CH₃ | 5-Chlorisoxazol-4-ylmethyl |
| 401 | CH₃ | CH₃ | 5-Isoxazolylmethyl |
| 402 | CH₃ | CH₃ | 3-Methylisoxazol-5-ylmethyl |
| 403 | CH₃ | CH₃ | 4-Methylisoxazol-5-ylmethyl |
| 404 | CH₃ | CH₃ | 3-Chlorisoxazol-5-ylmethyl, |
| 405 | CH₃ | CH₃ | 4-Chlorisoxazol-5-ylmethyl |
| 406 | CH₃ | CH₃ | 3-Isothiazolylmethyl |
| 407 | CH₃ | CH₃ | 4-Methylisothiazol-3-ylmethyl |
| 408 | CH₃ | CH₃ | 5-Methylisothiazol-3-ylmethyl |
| 409 | CH₃ | CH₃ | 4-Chlorisothiazol-3-ylmethyl |
| 410 | CH₃ | CH₃ | 5-Chlorisothiazol-3-ylmethyl |
| 411 | CH₃ | CH₃ | 4-Isothiazolylmethyl |
| 412 | CH₃ | CH₃ | 3-Methylisothiazol-4-ylmethyl |
| 413 | CH₃ | CH₃ | 5-Methylisothiazol-4-ylmethyl |
| 414 | CH₃ | CH₃ | 3-Chlorisothiazol-4-ylmethyl |
| 415 | CH₃ | CH₃ | 5-Chlorisothiazol-4-ylmethyl |
| 416 | CH₃ | CH₃ | 5-Isothiazolylmethyl |
| 417 | CH₃ | CH₃ | 3-Methylisothiazol-5-ylmethyl |
| 418 | CH₃ | CH₃ | 4-Methylisothiazol-5-ylmethyl |
| 419 | CH₃ | CH₃ | 3-Chlorisothiazol-5-ylmethyl |
| 420 | CH₃ | CH₃ | 4-Chlorisothiazol-5-ylmethyl |
| 421 | CH₃ | CH₃ | 4-Imidazolylmethyl |
| 422 | CH₃ | CH₃ | 1-Phenylpyrazol-3-ylmethyl |
| 423 | CH₃ | CH₃ | 1-Methylimidazol-4-ylmethyl |
| 424 | CH₃ | CH₃ | 1-Phenyl-1,2,4-triazol-3-ylmethyl |
| 425 | CH₃ | CH₃ | 1,2,4-Oxadiazol-3-ylmethyl |
| 426 | CH₃ | CH₃ | 5-Chlor-1,2,4-oxadiazol-3-ylmethyl |
| 427 | CH₃ | CH₃ | 5-Methyl-1,2,4-oxadiazol-3-ylmethyl |
| 428 | CH₃ | CH₃ | 5-Trifluormethyl-1,2,4-oxadiazol-3-ylmethyl |
| 429 | CH₃ | CH₃ | 1,3,4-Oxadiazol-2-ylmethyl |
| 430 | CH₃ | CH₃ | 5-Chlor-1,3,4-oxadiazol-2-ylmethyl |
| 431 | CH₃ | CH₃ | 5-Methyl-1,3,4-oxadiazol-2-ylmethyl |
| 432 | CH₃ | CH₃ | 5-Methoxy-1,3,4-oxadiazol-2-ylmethyl |
| 433 | CH₃ | CH₃ | 1,2,4-Thiadiazol-3-ylmethyl |
| 434 | CH₃ | CH₃ | 5-Chlor-1,2,4-thiadiazol-3-ylmethyl |
| 435 | CH₃ | CH₃ | 5-Methyl-1,2,4-thiadiazol-3-ylmethyl |
| 436 | CH₃ | CH₃ | 1,3,4-Thiadiazol-2-ylmethyl |
| 437 | CH₃ | CH₃ | 5-Chlor-1,3,4-thiadiazol-2-ylmethyl |
| 438 | CH₃ | CH₃ | 5-Methyl-1,3,4-thiadiazol-2-ylmethyl |
| 439 | CH₃ | CH₃ | 5-Cyano-1,3,4-thiadiazol-2-ylmethyl |
| 440 | CH₃ | CH₃ | 2-(2'-Pyridinyloxy)eth-1-yl |
| 441 | CH₃ | CH₃ | 2-(3'-Pyridinyloxy)eth-1-yl |
| 442 | CH₃ | CH₃ | 2-(4'-Pyridinyloxy)eth-1-yl |
| 443 | CH₃ | CH₃ | 2-(2'-Pyrimidinyloxy)eth-1-yl |
| 444 | CH₃ | CH₃ | 2-(4'-Pyrimidinyloxy)eth-1-yl |
| 445 | CH₃ | CH₃ | 2-(5'-Pyrimidinyloxy)eth-1-yl |
| 446 | CH₃ | CH₃ | 2-(2'-Pyrazinyloxy)eth-1-yl |
| 447 | CH₃ | CH₃ | 2-(2'-Pyridazinyloxy)eth-1-yl |
| 448 | CH₃ | CH₃ | 2-(3'-Pyridazinyloxy)eth-1-yl |
| 449 | CH₃ | CH₃ | 2-(1',3',5'-Triazinyloxy)eth-1-yl |
| 450 | CH₃ | CH₃ | 2-(5'-Methylisoxazol-3'-yloxy)eth-1-yl |
| 451 | CH₃ | CH₃ | 2-(5'-Chlorisoxazol-3'-yloxy)eth-1-yl |
| 452 | CH₃ | CH₃ | 2-(2'-Methoxythiazol-4'-yloxy)eth-1-yl |
| 453 | CH₃ | CH₃ | 2-(4'-Chloroxazol-2'-yloxy)eth-1-yl |
| 454 | CH₃ | CH₃ | 2-(1'-Phenyl-1'H-1',2',4'-triazol-3'-yloxy)eth-1-yl |
| 455 | CH₃ | CH₃ | 2-(1'-Phenylpyrazol-3'-yloxy)eth-1-yl |
| 482 | CH₃ | CH₃ | 3-Acetyl-C₆H₄ |
| 483 | CH₃ | CH₃ | 4-Acetyl-C₆H₄ |
| 484 | CH₃ | CH₃ | 3-Methoxycarbonyl-C₆H₄ |
| 485 | CH₃ | CH₃ | 4-Methoxycarbonyl-C₆H₄ |
| 488 | CH₃ | CH₃ | 2-Naphthyl |
| 489 | CH₃ | CH₃ | 6-Chlorpyridazin-3-yl |
| 490 | CH₃ | CH₃ | 5-Chlorpyrazin-2-yl |
| 491 | CH₃ | CH₃ | Chinolin-2-yl |
| 492 | CH₃ | CH₃ | 2,5-Dimethylpyrazin-3-yl |
| 493 | CH₃ | CH₃ | Pyrazin-2-yl |
| 502 | CH₃ | CH₃ | Benzothiazol-2-yl |
| 503 | CH₃ | CH₃ | 7-Chlorchinolin-4-yl |
| 505 | CH₃ | CH₃ | Pyrrol-3-yl |
| 506 | CH₃ | CH₃ | Pyrrol-2-yl |
| 507 | CH₃ | CH₃ | 2,6-Dioctylpyrid-4-yl |
| 511 | CH₃ | CH₃ | Pyrimidin-2-yl |
| 512 | CH₃ | CH₃ | Pyrimidin-4-yl |
| 513 | CH₃ | CH₃ | Chinazolin-4-yl |
| 514 | CH₃ | CH₃ | 6-Chlorpyrimidin-4-yl |
| 515 | CH₃ | CH₃ | 6-Methoxypyrimidin-4-yl |
| 516 | CH₃ | CH₃ | 2,5,6-Trichlorpyrimidin-4-yl |
| 517 | CH₃ | CH₃ | 2,6-Dimethylpyrimidin-4-yl |
| 518 | CH₃ | CH₃ | 2-Methyl, 6-Chlorpyrimidin-4-yl |
| 519 | CH₃ | CH₃ | 2-Methyl, 6-Ethoxypyrimidin-4-yl |
| 520 | CH₃ | CH₃ | 4,5,6-Trichlorpyrimidin-2-yl |
| 521 | CH₃ | CH₃ | 4,6-Dimethoxypyrimidin-2-yl |
| 522 | CH₃ | CH₃ | 4,6-Dimethylpyrimidin-2-yl |
| 523 | CH₃ | CH₃ | 4,6-Dichlorpyrimidin-2-yl |
| 524 | CH₃ | CH₃ | 4-Methyl, 6-methoxypyrimidin-2-yl |
| 525 | CH₃ | CH₃ | 4-Chlor, 6-methoxypyrimidin-2-yl |
| 526 | CH₃ | CH₃ | 6-Chlorchinoxalin-2-yl |
| 527 | CH₃ | CH₃ | 3,6-Dichlor-1,2,4-triazin-5-yl |
| 528 | CH₃ | CH₃ | 4-Methoxy-1,3,5-triazin-2-yl |
| 529 | CH₃ | CH₃ | 4-Ethoxy-1,3,5-triazin-2-yl |
| 530 | CH₃ | CH₃ | 4,6-Dichlor-1,3,5-triazin-2-yl |
| 531 | CH₃ | CH₃ | 4-Ethoxy, 6-Chlor-1,3,5-triazin-2-yl |
| 532 | CH₃ | CH₃ | Isoxazol-3-yl |
| 533 | CH₃ | CH₃ | Thien-2-yl |
| 534 | CH₃ | CH₃ | Fur-2-yl |
| 535 | CH₃ | CH₃ | Thiatriazol-5-yl |
| 536 | CH₃ | CH₃ | (E)-1-Chlorpropen-3-yl |
| 537 | CH₃ | CH₃ | (E)-4-(4'-Chlorphenyl)but-2-en-1-yl |
| 539 | CH₃ | CH₃ | Methylcarbonyl |
| 540 | CH₃ | CH₃ | Ethylcarbonyl |
| 541 | CH₃ | CH₃ | n-Propylcarbonyl |
| 542 | CH₃ | CH₃ | i-Propylcarbonyl |
| 543 | CH₃ | CH₃ | n-Butylcarbonyl |
| 544 | CH₃ | CH₃ | s-Butylcarbonyl |
| 545 | CH₃ | CH₃ | i-Butylcarbonyl |
| 546 | CH₃ | CH₃ | t-Butylcarbonyl |
| 547 | CH₃ | CH₃ | n-Pentylcarbonyl |
| 548 | CH₃ | CH₃ | i-Pentylcarbonyl |
| 549 | CH₃ | CH₃ | neo-Pentylcarbonyl |
| 550 | CH₃ | CH₃ | n-Hexylcarbonyl |
| 551 | CH₃ | CH₃ | n-Octylcarbonyl |
| 552 | CH₃ | CH₃ | 1-Propenylcarbonyl |
| 553 | CH₃ | CH₃ | 2-Penten-1-yl-carbonyl |
| 554 | CH₃ | CH₃ | 2,5-Heptadien-1-yl-carbonyl |
| 555 | CH₃ | CH₃ | Benzoyl |
| 556 | CH₃ | CH₃ | 2-Chlorbenzoyl |
| 557 | CH₃ | CH₃ | 3-Chlorbenzoyl |
| 558 | CH₃ | CH₃ | 4-Chlorbenzoyl |
| 559 | CH₃ | CH₃ | 2-Cyanobenzoyl |
| 560 | CH₃ | CH₃ | 3-Cyanobenzoyl |
| 561 | CH₃ | CH₃ | 4-Cyanobenzoyl |
| 562 | CH₃ | CH₃ | 4-Methoxybenzoyl |
| 563 | CH₃ | CH₃ | 2-Pyridylcarbonyl |
| 564 | CH₃ | CH₃ | 3-Pyridylcarbonyl |
| 565 | CH₃ | CH₃ | 4-Pyridylcarbonyl |
| 566 | CH₃ | CH₃ | 2-Pyrimidinylcarbonyl |
| 567 | CH₃ | CH₃ | 2-Oxazolylcarbonyl |
| 568 | CH₃ | CH₃ | 4-Methylisoxazol-5-yl-carbonyl |
| 569 | CH₃ | CH₃ | Methylsulfonyl |
| 570 | CH₃ | CH₃ | Ethylsulfonyl |
| 571 | CH₃ | CH₃ | n-Propylsulfonyl |
| 572 | CH₃ | CH₃ | i-Propylsulfonyl |
| 573 | CH₃ | CH₃ | n-Butylsulfonyl |
| 574 | CH₃ | CH₃ | t-Butylsulfonyl |
| 575 | CH₃ | CH₃ | n-Pentylsulfonyl |
| 576 | CH₃ | CH₃ | neo-Pentylsulfonyl |
| 577 | CH₃ | CH₃ | n-Hexylsulfonyl |
| 578 | CH₃ | CH₃ | n-Octylsulfonyl |
| 579 | CH3 | CH₃ | Phenylsulfonyl |
| 580 | CH₃ | CH₃ | 2-Chlorphenylsulfonyl |
| 581 | CH₃ | CH₃ | 3-Chlorphenylsulfonyl |
| 582 | CH₃ | CH₃ | 4-Chlorphenylsulfonyl |
| 583 | CH₃ | CH₃ | 2-Cyanophenylsulfonyl |
| 584 | CH₃ | CH₃ | 3-Cyanophenylsulfonyl |
| 585 | CH₃ | CH₃ | 4-Cyanophenylsulfonyl |
| 586 | CH₃ | CH₃ | 2-Pyridylsulfonyl |
| 587 | CH₃ | CH₃ | 3-Pyridylsulfonyl |
| 588 | CH₃ | CH₃ | 4-Pyridylsulfonyl |
| 589 | CH₃ | CH₃ | 2-Pyrimidinylsulfonyl |
| 590 | CH₃ | CH₃ | 4-Oxazolylsulfonyl |
| 591 | CH₃ | CH₃ | 5-Chlorthiazol-2ylsulfonyl |
| 595 | CH₃ | CH₃ | 2-(4'-Chlorthiazol-2'-yloxy) eth-1-yl |
| 596 | CH₃ | CH₃ | 2-(1'-Methylpyrazol-4'-yloxy)eth-1-yl |
| 600 | CH₃ | CH₃ | 3-Dimethylaminocarbonyl-C₆H₄ |
| 601 | CH₃ | CH₃ | 4-Dimethylaminocarbonyl-C₆H₄ |
| 602 | CH₃ | CH₃ | 2-Hydroxyprop-1-yl |
| 603 | CH₃ | CH₃ | 6-Hydroxy-2-methylpyrimidin-4-ylmethyl |
| 604 | CH₃ | CH₃ | [6-OH,2-CH(CH₃)₂-pyrimidin-4-yl]-CH₂ |
| 605 | CH₃ | CH₃ | [6-OH,2-CH(CH₂)₂-pyrimidin-4-yl]-CH₂ |
| 606 | CH₃ | CH₃ | 5-(2'-Furan)-pent-1-yl |
| 607 | CH₃ | CH₃ | 5-(2'-N-Methylpyrrol)-pent-1-yl |
| 608 | CH₃ | CH₃ | [2-(4-Cl-C₆H₄)-oxazol-4-yl]-CH₂ |
| 611 | CH₃ | CH₃ | 6-(2'-Thienyl)hex-1-yl |
| 624 | CH₃ | t-C₄H₉ | 2-Naphthyl-CH₂ |
| 626 | CH₃ | t-C₄H₉ | (E)-4-(4'-Chlorphenyl)but-2-en-1-yl |
| 627 | CH₃ | t-C₄H₉ | 6-(4'-Chlorphenyl)hex-1-yl |
| 631 | CH₃ | C₆H₅ | C₂H₅ |
| 640 | CH₃ | C₆H₅ | 6-(4'-Chlorphenyl)hex-1-yl |
| 641 | CH₃ | C₆H₅ | (E)-4-(4'-Chlorphenyl)but-2-en-1-yl |
| 647 | CH₃ | OH | H |
| 648 | CH₃ | OH | CH₃ |
| 649 | CH₃ | OH | C₂H₅ |
| 650 | CH₃ | OH | n-C₃H₇ |
| 651 | CH₃ | OH | i-C₃H₇ |
| 652 | CH₃ | Cl | CH₃ |
| 653 | CH₃ | Cl | C₂H₅ |
| 654 | CH₃ | Cl | n-C₃H₇ |
| 655 | CH₃ | Cl | i-C₃H₇ |
| 656 | CH₃ | OCH₃ | H |
| 657 | CH₃ | OCH₃ | CH₃ |
| 658 | CH₃ | OCH₃ | C₂H₅ |
| 659 | CH₃ | OCH₃ | n-C₃H₇ |
| 660 | CH₃ | OCH₃ | i-C₃H₇ |
| 661 | CH₃ | SCH₃ | H |
| 662 | CH₃ | SCH₃ | CH₃ |
| 663 | CH₃ | SCH₃ | C₂H₅ |
| 664 | CH₃ | SCH₃ | n-C₃H₇ |
| 665 | CH₃ | SCH₃ | i-C₃H₇ |
| 671 | CH₃ | 2-Pyridyl | H |
| 672 | CH₃ | 2-Pyridyl | CH₃ |
| 673 | CH₃ | 2-Pyridyl | C₂H₅ |
| 674 | CH₃ | 2-Pyridyl | n-C₃H₇ |
| 675 | CH₃ | 2-Pyridyl | i-C₃H₇ |
| 676 | CH₃ | 3-Pyridyl | H |
| 677 | CH₃ | 3-Pyridyl | CH₃ |
| 678 | CH₃ | 3-Pyridyl | C₂H₅ |
| 679 | CH₃ | 3-Pyridyl | n-C₃H₇ |
| 680 | CH₃ | 3-Pyridyl | i-C₃H₇ |
| 681 | CH₃ | 4-Pyridyl | H |
| 682 | CH₃ | 4-Pyridyl | CH₃ |
| 683 | CH₃ | 4-Pyridyl | C₂H₅ |
| 684 | CH₃ | 4-Pyridyl | n-C₃H₇ |
| 685 | CH₃ | 4-Pyridyl | i-C₃H₇ |
| 686 | CH₃ | 2-Pyridimidyl | H |
| 687 | CH₃ | 2-Pyridimidyl | CH₃ |
| 688 | CH₃ | 2-Pyridimidyl | C₂H₅ |
| 689 | CH₃ | 2-Pyridimidyl | n-C₃H₇ |
| 690 | CH₃ | 2-Pyridimidyl | i-C₃H₇ |
| 691 | CH₃ | 4-Pyridimidyl | H |
| 692 | CH₃ | 4-Pyridimidyl | CH₃ |
| 693 | CH₃ | 4-Pyridimidyl | C₂H₅ |
| 694 | CH₃ | 4-Pyridimidyl | n-C₃H₇ |
| 695 | CH₃ | 4-Pyridimidyl | i-C₃H₇ |
| 696 | CH₃ | 5-Pyridimidyl | H |
| 697 | CH₃ | 5-Pyridimidyl | CH₃ |
| 698 | CH₃ | 5-Pyridimidyl | C₂H₅ |
| 699 | CH₃ | 5-Pyridimidyl | n-C₃H₇ |
| 700 | CH₃ | 5-Pyridimidyl | i-C₃H₇ |
| 701 | CH₃ | 1,3,5-Triazinyl | H |
| 702 | CH₃ | 1,3,5-Triazinyl | CH₃ |
| 703 | CH₃ | 1,3,5-Triazinyl | C₂H₅ |
| 704 | CH₃ | 1,3,5-Triazinyl | n-C₃H₇ |
| 705 | CH₃ | 1,3,5-Triazinyl | i-C₃H₇ |
| 706 | CH₃ | 2-Furyl | H |
| 707 | CH₃ | 2-Furyl | CH₃ |
| 708 | CH₃ | 2-Furyl | C₂H₅ |
| 709 | CH₃ | 2-Furyl | n-C₃H₇ |
| 710 | CH₃ | 2-Furyl | i-C₃H₇ |
| 711 | CH₃ | 3-Furyl | H |
| 712 | CH₃ | 3-Furyl | CH₃ |
| 713 | CH₃ | 3-Furyl | C₂H₅ |
| 714 | CH₃ | 3-Furyl | n-C₃H₇ |
| 715 | CH₃ | 3-Furyl | i-C₃H₇ |
| 726 | CH₃ | 2-Oxazolyl | H |
| 727 | CH₃ | 2-Oxazolyl | CH₃ |
| 728 | CH₃ | 2-Oxazolyl | C₂H₅ |
| 729 | CH₃ | 2-Oxazolyl | n-C₃H₇ |
| 730 | CH₃ | 2-Oxazolyl | i-C₃H₇ |
| 731 | CH₃ | 4-Oxazolyl | H |
| 732 | CH₃ | 4-Oxazolyl | CH₃ |
| 733 | CH₃ | 4-Oxazolyl | C₂H₅ |
| 734 | CH₃ | 4-Oxazolyl | n-C₃H₇ |
| 735 | CH₃ | 4-Oxazolyl | i-C₃H₇ |
| 736 | CH₃ | 2-Thiazolyl | H |
| 737 | CH₃ | 2-Thiazolyl | CH₃ |
| 738 | CH₃ | 2-Thiazolyl | C₂H₅ |
| 739 | CH₃ | 2-Thiazolyl | n-C₃H₇ |
| 740 | CH₃ | 2-Thiazolyl | i-C₃H₇ |
| 741 | CH₃ | 4-Thiazolyl | H |
| 742 | CH₃ | 4-Thiazolyl | CH₃ |
| 743 | CH₃ | 4-Thiazolyl | C₂H₅ |
| 744 | CH₃ | 4-Thiazolyl | n-C₃H₇ |
| 745 | CH₃ | 4-Thiazolyl | i-C₃H₇ |
| 746 | CH₃ | 3-Isoxazolyl | H |
| 747 | CH₃ | 3-Isoxazolyl | CH₃ |
| 748 | CH₃ | 3-Isoxazolyl | C₂H₅ |
| 749 | CH₃ | 3-Isoxazolyl | n-C₃H₇ |
| 750 | CH₃ | 3-Isoxazolyl | i-C₃H₇ |
| 751 | CH₃ | 5-Isoxazolyl | H |
| 752 | CH₃ | 5-Isoxazolyl | CH₃ |
| 753 | CH₃ | 5-Isoxazolyl | C₂H₅ |
| 754 | CH₃ | 5-Isoxazolyl | n-C₃H₇ |
| 755 | CH₃ | 5-Isoxazolyl | i-C₃H₇ |
| 756 | CH₃ | 2-Imidazolyl | H |
| 757 | CH₃ | 2-Imidazolyl | CH₃ |
| 758 | CH₃ | 2-Imidazolyl | C₂H₅ |
| 759 | CH₃ | 2-Imidazolyl | n-C₃H₇ |
| 760 | CH₃ | 2-Imidazolyl | i-C₃H₇ |
| 761 | CH₃ | 3-Pyrazolyl | H |
| 762 | CH₃ | 3-Pyrazolyl | CH₃ |
| 763 | CH₃ | 3-Pyrazolyl | C₂H₅ |
| 764 | CH₃ | 3-Pyrazolyl | n-C₃H₇ |
| 765 | CH₃ | 3-Pyrazolyl | i-C₃H₇ |
| 766 | CH₃ | 4-Pyrazolyl | H |
| 767 | CH₃ | 4-Pyrazolyl | CH₃ |
| 768 | CH₃ | 4-Pyrazolyl | C₂H₅ |
| 769 | CH₃ | 4-Pyrazolyl | n-C₃H₇ |
| 770 | CH₃ | 4-Pyrazolyl | i-C₃H₇ |
| 771 | OCH₃ | H | H |
| 772 | OCH₃ | H | CH₃ |
| 773 | OCH₃ | H | C₂H₅ |
| 774 | OCH₃ | H | n-C₃H₇ |
| 775 | OCH₃ | H | i-C₃H₇ |
| 776 | OCH₃ | OH | H |
| 777 | OCH₃ | OH | CH₃ |
| 778 | OCH₃ | OH | C₂H₅ |
| 779 | OCH₃ | OH | n-C₃H₇ |
| 780 | OCH₃ | OH | i-C₃H₇ |
| 781 | OCH₃ | Cl | n-C₄H₉ |
| 782 | OCH₃ | Cl | CH₃ |
| 783 | OCH₃ | Cl | C₂H₅ |
| 784 | OCH₃ | Cl | n-C₃H₇ |
| 785 | OCH₃ | Cl | i-C₃H₇ |
| 786 | OCH₃ | OCH₃ | H |
| 787 | OCH₃ | OCH₃ | CH₃ |
| 788 | OCH₃ | OCH₃ | C₂H₅ |
| 789 | OCH₃ | OCH₃ | n-C₃H₇ |
| 790 | OCH₃ | OCH₃ | i-C₃H₇ |
| 791 | OCH₃ | SCH₃ | H |
| 792 | OCH₃ | SCH₃ | CH₃ |
| 793 | OCH₃ | SCH₃ | C₂H₅ |
| 794 | OCH₃ | SCH₃ | n-C₃H₇ |
| 795 | OCH₃ | SCH₃ | i-C₃H₇ |
| 796 | OCH₃ | CH₃ | H |
| 797 | OCH₃ | CH₃ | CH₃ |
| 798 | OCH₃ | CH₃ | C₂H₅ |
| 799 | OCH₃ | CH₃ | n-C₃H₇ |
| 800 | OCH₃ | CH₃ | i-C₃H₇ |
| 801 | OCH₃ | Cyclopropyl | H |
| 802 | OCH₃ | Cyclopropyl | CH₃ |
| 803 | OCH₃ | Cyclopropyl | C₂H₅ |
| 804 | OCH₃ | Cyclopropyl | n-C₃H₇ |
| 805 | OCH₃ | Cyclopropyl | i-C₃H₇ |
| 806 | OCH₃ | 2-Pyridyl | H |
| 807 | OCH₃ | 2-Pyridyl | CH₃ |
| 808 | OCH₃ | 2-Pyridyl | C₂H₅ |
| 809 | OCH₃ | 2-Pyridyl | n-C₃H₇ |
| 810 | OCH₃ | 2-Pyridyl | i-C₃H₇ |
| 811 | OCH₃ | 3-Pyridyl | H |
| 812 | OCH₃ | 3-Pyridyl | CH₃ |
| 813 | OCH₃ | 3-Pyridyl | C₂H₅ |
| 814 | OCH₃ | 3-Pyridyl | n-C₃H₇ |
| 815 | OCH₃ | 3-Pyridyl | i-C₃H₇ |
| 816 | OCH₃ | 4-Pyridyl | H |
| 817 | OCH₃ | 4-Pyridyl | CH₃ |
| 818 | OCH₃ | 4-Pyridyl | C₂H₅ |
| 819 | OCH₃ | 4-Pyridyl | n-C₃H₇ |
| 820 | OCH₃ | 4-Pyrimidyl | i-C₃H₇ |
| 821 | OCH₃ | 2-Pyrimidyl | H |
| 822 | OCH₃ | 2-Pyrimidyl | CH₃ |
| 823 | OCH₃ | 2-Pyrimidyl | C₂H₅ |
| 824 | OCH₃ | 2-Pyrimidyl | n-C₃H₇ |
| 825 | OCH₃ | 2-Pyrimidyl | i-C₃H₇ |
| 826 | OCH₃ | 4-Pyrimidyl | H |
| 827 | OCH₃ | 4-Pyrimidyl | CH₃ |
| 828 | OCH₃ | 4-Pyrimidyl | C₂H₅ |
| 829 | OCH₃ | 4-Pyrimidyl | n-C₃H₇ |
| 830 | OCH₃ | 4-Pyrimidyl | i-C₃H₇ |
| 831 | OCH₃ | 5-Pyrimidyl | H |
| 832 | OCH₃ | 5-Pyrimidyl | CH₃ |
| 833 | OCH₃ | 5-Pyrimidyl | C₂H₅ |
| 834 | OCH₃ | 5-Pyrimidyl | n-C₃H₇ |
| 835 | OCH₃ | 5-Pyrimidyl | i-C₃H₇ |
| 836 | OCH₃ | 1,3,5-Triazinyl | H |
| 837 | OCH₃ | 1,3,5-Triazinyl | CH₃ |
| 838 | OCH₃ | 1,3,5-Triazinyl | C₂H₅ |
| 839 | OCH₃ | 1,3,5-Triazinyl | n-C₃H₇ |
| 840 | OCH₃ | 1,3,5-Triazinyl | i-C₃H₇ |
| 841 | OCH₃ | 2-Furyl | H |
| 842 | OCH₃ | 2-Furyl | CH₃ |
| 843 | OCH₃ | 2-Furyl | C₂H₅ |
| 844 | OCH₃ | 2-Furyl | n-C₃H₇ |
| 845 | OCH₃ | 2-Furyl | i-C₃H₇ |
| 846 | OCH₃ | 3-Furyl | H |
| 847 | OCH₃ | 3-Furyl | CH₃ |
| 848 | OCH₃ | 3-Furyl | C₂H₅ |
| 849 | OCH₃ | 3-Furyl | n-C₃H₇ |
| 850 | OCH₃ | 3-Furyl | i-C₃H₇ |
| 851 | OCH₃ | 2-Thienyl | H |
| 852 | OCH₃ | 2-Thienyl | CH₃ |
| 853 | OCH₃ | 2-Thienyl | C₂H₅ |
| 854 | OCH₃ | 2-Thienyl | n-C₃H₇ |
| 855 | OCH₃ | 2-Thienyl | i-C₃H₇ |
| 856 | OCH₃ | 3-Thienyl | H |
| 857 | OCH₃ | 3-Thienyl | CH₃ |
| 858 | OCH₃ | 3-Thienyl | C₂H₅ |
| 859 | OCH₃ | 3-Thienyl | n-C₃H₇ |
| 860 | OCH₃ | 3-Thienyl | i-C₃H₇ |
| 861 | OCH₃ | 2-Oxazolyl | H |
| 862 | OCH₃ | 2-Oxazolyl | CH₃ |
| 863 | OCH₃ | 2-Oxazolyl | C₂H₅ |
| 864 | OCH₃ | 2-Oxazolyl | n-C₃H₇ |
| 865 | OCH₃ | 2-Oxazolyl | i-C₃H₇ |
| 866 | OCH₃ | 4-Oxazolyl | H |
| 867 | OCH₃ | 4-Oxazolyl | CH₃ |
| 868 | OCH₃ | 4-Oxazolyl | C₂H₅ |
| 869 | OCH₃ | 4-Oxazolyl | n-C₃H₇ |
| 870 | OCH₃ | 4-Oxazolyl | i-C₃H₇ |
| 871 | OCH₃ | 2-Thiazolyl | H |
| 872 | OCH₃ | 2-Thiazolyl | CH₃ |
| 873 | OCH₃ | 2-Thiazolyl | C₂H₅ |
| 874 | OCH₃ | 2-Thiazolyl | n-C₃H₇ |
| 875 | OCH₃ | 2-Thiazolyl | i-C₃H₇ |
| 876 | OCH₃ | 4-Thiazolyl | H |
| 877 | OCH₃ | 4-Thiazolyl | CH₃ |
| 878 | OCH₃ | 4-Thiazolyl | C₂H₅ |
| 879 | OCH₃ | 4-Thiazolyl | n-C₃H₇ |
| 880 | OCH₃ | 4-Thiazolyl | i-C₃H₇ |
| 881 | OCH₃ | 3-Isoxazolyl | H |
| 882 | OCH₃ | 3-Isoxazolyl | CH₃ |
| 883 | OCH₃ | 3-Isoxazolyl | C₂H₅ |
| 884 | OCH₃ | 3-Isoxazolyl | n-C₃H₇ |
| 885 | OCH₃ | 3-Isoxazolyl | i-C₃H₇ |
| 886 | OCH₃ | 5-Isoxazolyl | H |
| 887 | OCH₃ | 5-Isoxazolyl | CH₃ |
| 888 | OCH₃ | 5-Isoxazolyl | C₂H₅ |
| 889 | OCH₃ | 5-Isoxazolyl | n-C₃H₇ |
| 890 | OCH₃ | 5-Isoxazolyl | i-C₃H₇ |
| 891 | OCH₃ | 2-Imidazolyl | H |
| 892 | OCH₃ | 2-Imidazolyl | CH₃ |
| 893 | OCH₃ | 2-Imidazolyl | C₂H₅ |
| 894 | OCH₃ | 2-Imidazolyl | n-C₃H₇ |
| 895 | OCH₃ | 2-Imidazolyl | i-C₃H₇ |
| 896 | OCH₃ | 3-Pyrazolyl | H |
| 897 | OCH₃ | 3-Pyrazolyl | CH₃ |
| 898 | OCH₃ | 3-Pyrazolyl | C₂H₅ |
| 899 | OCH₃ | 3-Pyrazolyl | n-C₃H₇ |
| 900 | OCH₃ | 3-Pyrazolyl | i-C₃H₇ |
| 901 | OCH₃ | 4-Pyrazolyl | H |
| 902 | OCH₃ | 4-Pyrazolyl | CH₃ |
| 903 | OCH₃ | 4-Pyrazolyl | C₂H₅ |
| 904 | OCH₃ | 4-Pyrazolyl | n-C₃H₇ |
| 905 | OCH₃ | 4-Pyrazolyl | i-C₃H₇ |
| 906 | OH | H | H |
| 907 | OH | H | CH₃ |
| 908 | OH | H | C₂H₅ |
| 909 | OH | H | n-C₃H₇ |
| 910 | OH | H | i-C₃H₇ |
| 911 | OH | OH | H |
| 912 | OH | OH | CH₃ |
| 913 | OH | OH | C₂H₅ |
| 914 | OH | OH | n-C₃H₇ |
| 915 | OH | OH | i-C₃H₇ |
| 916 | OH | Cl | n-C₄H₉ |
| 917 | OH | Cl | CH₃ |
| 918 | OH | Cl | C₂H₅ |
| 919 | OH | Cl | n-C₃H₇ |
| 920 | OH | Cl | i-C₃H₇ |
| 921 | OH | OCH₃ | H |
| 922 | OH | OCH₃ | CH₃ |
| 923 | OH | OCH₃ | C₂H₅ |
| 924 | OH | OCH₃ | n-C₃H₇ |
| 925 | OH | OCH₃ | i-C₃H₇ |
| 926 | OH | SCH₃ | H |
| 927 | OH | SCH₃ | CH₃ |
| 928 | OH | SCH₃ | C₂H₅ |
| 929 | OH | SCH₃ | n-C₃H₇ |
| 930 | OH | SCH₃ | i-C₃H₇ |
| 931 | OH | CH₃ | H |
| 932 | OH | CH₃ | CH₃ |
| 933 | OH | CH₃ | C₂H₅ |
| 934 | OH | CH₃ | n-C₃H₇ |
| 935 | OH | CH₃ | i-C₃H₇ |
| 936 | OH | Cyclopropyl | H |
| 937 | OH | Cyclopropyl | CH₃ |
| 938 | OH | Cyclopropyl | C₂H₅ |
| 939 | OH | Cyclopropyl | n-C₃H₇ |
| 940 | OH | Cyclopropyl | i-C₃H₇ |
| 941 | OH | 2-Pyridyl | H |
| 942 | OH | 2-Pyridyl | CH₃ |
| 943 | OH | 2-Pyridyl | C₂H₅ |
| 944 | OH | 2-Pyridyl | n-C₃H₇ |
| 945 | OH | 2-Pyridyl | i-C₃H₇ |
| 946 | OH | 3-Pyridyl | H |
| 947 | OH | 3-Pyridyl | CH₃ |
| 948 | OH | 3-Pyridyl | C₂H₅ |
| 949 | OH | 3-Pyridyl | n-C₃H₇ |
| 950 | OH | 3-Pyridyl | i-C₃H₇ |
| 951 | OH | 4-Pyridyl | H |
| 952 | OH | 4-Pyridyl | CH₃ |
| 953 | OH | 4-Pyridyl | C₂H₅ |
| 954 | OH | 4-Pyridyl | n-C₃H₇ |
| 955 | OH | 4-Pyridyl | i-C₃H₇ |
| 956 | OH | 2-Pyrimidyl | H |
| 957 | OH | 2-Pyrimidyl | CH₃ |
| 958 | OH | 2-Pyrimidyl | C₂H₅ |
| 959 | OH | 2-Pyrimidyl | n-C₃H₇ |
| 960 | OH | 2-Pyrimidyl | i-C₃H₇ |
| 961 | OH | 4-Pyrimidyl | H |
| 962 | OH | 4-Pyrimidyl | CH₃ |
| 963 | OH | 4-Pyrimidyl | C₂H₅ |
| 964 | OH | 4-Pyrimidyl | n-C₃H₇ |
| 965 | OH | 4-Pyrimidyl | i-C₃H₇ |
| 966 | OH | 5-Pyrimidyl | H |
| 967 | OH | 5-Pyrimidyl | CH₃ |
| 968 | OH | 5-Pyrimidyl | C₂H₅ |
| 969 | OH | 5-Pyrimidyl | n-C₃H₇ |
| 970 | OH | 5-Pyrimidyl | i-C₃H₇ |
| 971 | OH | 1,3,5-Triazinyl | H |
| 972 | OH | 1,3,5-Triazinyl | CH₃ |
| 973 | OH | 1,3,5-Triazinyl | C₂H₅ |
| 974 | OH | 1,3,5-Triazinyl | n-C₃H₇ |
| 975 | OH | 1,3,5-Triazinyl | i-C₃H₇ |
| 976 | OH | 2-Furyl | H |
| 977 | OH | 2-Furyl | CH₃ |
| 978 | OH | 2-Furyl | C₂H₅ |
| 979 | OH | 2-Furyl | n-C₃H₇ |
| 980 | OH | 2-Furyl | i-C₃H₇ |
| 981 | OH | 3-Furyl | H |
| 982 | OH | 3-Furyl | CH₃ |
| 983 | OH | 3-Furyl | C₂H₅ |
| 984 | OH | 3-Furyl | n-C₃H₇ |
| 985 | OH | 3-Furyl | i-C₃H₇ |
| 986 | OH | 2-Thienyl | H |
| 987 | OH | 2-Thienyl | CH₃ |
| 988 | OH | 2-Thienyl | C₂H₅ |
| 989 | OH | 2-Thienyl | n-C₃H₇ |
| 990 | OH | 2-Thienyl | i-C₃H₇ |
| 991 | OH | 3-Thienyl | H |
| 992 | OH | 3-Thienyl | CH₃ |
| 993 | OH | 3-Thienyl | C₂H₅ |
| 994 | OH | 3-Thienyl | n-C₃H₇ |
| 995 | OH | 3-Thienyl | i-C₃H₇ |
| 996 | OH | 2-Oxazolyl | H |
| 997 | OH | 2-Oxazolyl | CH₃ |
| 998 | OH | 2-Oxazolyl | C₂H₅ |
| 999 | OH | 2-Oxazolyl | n-C₃H₇ |
| 1000 | OH | 2-Oxazolyl | i-C₃H₇ |
| 1001 | OH | 4-Oxazolyl | H |
| 1002 | OH | 4-Oxazolyl | CH₃ |
| 1003 | OH | 4-Oxazolyl | C₂H₅ |
| 1004 | OH | 4-Oxazolyl | n-C₃H₇ |
| 1005 | OH | 2-Thiazolyl | i-C₃H₇ |
| 1006 | OH | 2-Thiazolyl | H |
| 1007 | OH | 2-Thiazolyl | CH₃ |
| 1008 | OH | 2-Thiazolyl | C₂H₅ |
| 1009 | OH | 2-Thiazolyl | n-C₃H₇ |
| 1010 | OH | 2-Thiazolyl | i-C₃H₇ |
| 1011 | OH | 4-Thiazolyl | H |
| 1012 | OH | 4-Thiazolyl | CH₃ |
| 1013 | OH | 4-Thiazolyl | C₂H₅ |
| 1014 | OH | 4-Isoxazolyl | n-C₃H₇ |
| 1015 | OH | 4-Isoxazolyl | i-C₃H₇ |
| 1016 | OH | 3-Isoxazolyl | H |
| 1017 | OH | 3-Isoxazolyl | CH₃ |
| 1018 | OH | 3-Isoxazolyl | C₂H₅ |
| 1019 | OH | 3-Isoxazolyl | n-C₃H₇ |
| 1020 | OH | 3-Isoxazolyl | i-C₃H₇ |
| 1021 | OH | 5-Isoxazolyl | H |
| 1022 | OH | 5-Isoxazolyl | CH₃ |
| 1023 | OH | 5-Isoxazolyl | C₂H₅ |
| 1024 | OH | 5-Isoxazolyl | n-C₃H₇ |
| 1025 | OH | 5-Isoxazolyl | i-C₃H₇ |
| 1026 | OH | 2-Imidazolyl | H |
| 1027 | OH | 2-Imidazolyl | CH₃ |
| 1028 | OH | 2-Imidazolyl | C₂H₅ |
| 1029 | OH | 2-Imidazolyl | n-C₃H₇ |
| 1030 | OH | 2-Imidazolyl | i-C₃H₇ |
| 1031 | OH | 3-Pyrazolyl | H |
| 1032 | OH | 3-Pyrazolyl | CH₃ |
| 1033 | OH | 3-Pyrazolyl | C₂H₅ |
| 1034 | OH | 3-Pyrazolyl | n-C₃H₇ |
| 1035 | OH | 3-Pyrazolyl | i-C₃H₇ |
| 1036 | OH | 4-Pyrazolyl | H |
| 1037 | OH | 4-Pyrazolyl | CH₃ |
| 1038 | OH | 4-Pyrazolyl | C₂H₅ |
| 1039 | OH | 4-Pyrazolyl | n-C₃H₇ |
| 1040 | OH | 4-Pyrazolyl | i-C₃H₇ |
| 1046 | H | OH | H |
| 1047 | H | OH | CH₃ |
| 1048 | H | OH | C₂H₅ |
| 1049 | H | OH | n-C₃H₇ |
| 1050 | H | OH | i-C₃H₇ |
| 1051 | H | Cl | n-C₄H₉ |
| 1052 | H | Cl | CH₃ |
| 1053 | H | Cl | C₂H₅ |
| 1054 | H | Cl | n-C₃H₇ |
| 1055 | H | Cl | i-C₃H₇ |
| 1056 | H | OCH₃ | H |
| 1057 | H | OCH₃ | CH₃ |
| 1058 | H | OCH₃ | C₂H₅ |
| 1059 | H | OCH₃ | n-C₃H₇ |
| 1060 | H | OCH₃ | i-C₃H₇ |
| 1071 | Cl | H | H |
| 1072 | Cl | H | CH₃ |
| 1073 | Cl | H | C₂H₅ |
| 1074 | Cl | H | n-C₃H₇ |
| 1075 | Cl | H | i-C₃H₇ |
| 1076 | Cl | OH | H |
| 1077 | Cl | OH | CH₃ |
| 1078 | Cl | OH | C₂H₅ |
| 1079 | Cl | OH | n-C₃H₇ |
| 1080 | Cl | OH | i-C₃H₇ |
| 1081 | Cl | Cl | n-C₄H₉ |
| 1082 | Cl | Cl | CH₃ |
| 1083 | Cl | Cl | C₂H₅ |
| 1084 | Cl | Cl | n-C₃H₇ |
| 1085 | Cl | Cl | i-C₃H₇ |
| 1086 | Cl | OCH₃ | H |
| 1087 | Cl | OCH₃ | CH₃ |
| 1088 | Cl | OCH₃ | C₂H₅ |
| 1089 | Cl | OCH₃ | n-C₃H₇ |
| 1090 | Cl | OCH₃ | i-C₃H₇ |
| 1091 | Cl | CH₃ | H |
| 1092 | Cl | CH₃ | CH₃ |
| 1093 | Cl | CH₃ | C₂H₅ |
| 1094 | Cl | CH₃ | n-C₃H₇ |
| 1095 | Cl | CH₃ | i-C₃H₇ |
| 1096 | Cl | Cyclopropyl | H |
| 1097 | Cl | Cyclopropyl | CH₃ |
| 1098 | Cl | Cyclopropyl | C₂H₅ |
| 1099 | Cl | Cyclopropyl | n-C₃H₇ |
| 1100 | Cl | Cyclopropyl | i-C₃H₇ |
| 1106 | SCH₃ | OH | H |
| 1107 | SCH₃ | OH | CH₃ |
| 1108 | SCH₃ | OH | C₂H₅ |
| 1109 | SCH₃ | OH | n-C₃H₇ |
| 1110 | SCH₃ | OH | i-C₃H₇ |
| 1116 | SCH₃ | SCH₃ | H |
| 1117 | SCH₃ | SCH₃ | CH₃ |
| 1118 | SCH₃ | SCH₃ | C₂H₅ |
| 1119 | SCH₃ | SCH₃ | n-C₃H₇ |
| 1120 | SCH₃ | SCH₃ | i-C₃H₇ |
| 1157 | CH₃ | 2-F-C₆H₄ | C₂H₅ |
| 1168 | CH₃ | 3-F-C₆H₄ | CH₃ |
| 1169 | CH₃ | 3-F-C₆H₄ | C₂H₅ |
| 1181 | CH₃ | 4-F-C₆H₄ | C₂H₅ |
| 1192 | CH₃ | 2-Cl-C₆H₄ | CH₃ |
| 1193 | CH₃ | 2-Cl-C₆H₄ | C₂H₅ |
| 1205 | CH₃ | 3-Cl-C₆H₄ | C₂H₅ |
| 1217 | CH₃ | 4-Cl-C₆H₄ | C₂H₅ |
| 1228 | CH₃ | 2,3-Cl₂-C₆H₃ | CH₃ |
| 1229 | CH₃ | 2,3-Cl₂-C₆H₃ | C₂H₅ |
| 1240 | CH₃ | 2,4-Cl₂-C₆H₃ | CH₃ |
| 1241 | CH₃ | 2,4-Cl₂-C₆H₃ | C₂H₅ |
| 1252 | CH₃ | 2,5-Cl₂-C₆H₃ | CH₃ |
| 1253 | CH₃ | 2,5-Cl₂-C₆H₃ | C₂H₅ |
| 1264 | CH₃ | 2,6-Cl₂-C₆H₃ | CH₃ |
| 1265 | CH₃ | 2,6-Cl₂-C₆H₃ | C₂H₅ |
| 1276 | CH₃ | 3,4-Cl₂-C₆H₃ | CH₃ |
| 1277 | CH₃ | 3,4-Cl₂-C₆H₃ | C₂H₅ |
| 1288 | CH₃ | 3,5-Cl₂-C₆H₃ | CH₃ |
| 1289 | CH₃ | 3,5-Cl₂-C₆H₃ | C₂H₅ |
| 1300 | CH₃ | 2-Br-C₆H₄ | CH₃ |
| 1301 | CH₃ | 2-Br-C₆H₄ | C₂H₅ |
| 1313 | CH₃ | 3-Br-C₆H₄ | C₂H₅ |
| 1325 | CH₃ | 4-Br-C₆H₄ | C₂H₅ |
| 1336 | CH₃ | 2-I-C₆H₄ | CH₃ |
| 1337 | CH₃ | 2-I-C₆H₄ | C₂H₅ |
| 1348 | CH₃ | 3-I-C₆H₄ | CH₃ |
| 1349 | CH₃ | 3-I-C₆H₄ | C₂H₅ |
| 1360 | CH₃ | 4-I-C₆H₄ | CH₃ |
| 1361 | CH₃ | 4-I-C₆H₄ | C₂H₅ |
| 1372 | CH₃ | 2-CN-C₆H₄ | CH₃ |
| 1373 | CH₃ | 2-CN-C₆H₄ | C₂H₅ |
| 1384 | CH₃ | 3-CN-C₆H₄ | CH₃ |
| 1385 | CH₃ | 3-CN-C₆H₄ | C₂H₅ |
| 1396 | CH₃ | 4-CN-C₆H₄ | CH₃ |
| 1397 | CH₃ | 4-CN-C₆H₄ | C₂H₅ |
| 1408 | CH₃ | 2-NO₂-C₆H₄ | CH₃ |
| 1409 | CH₃ | 2-NO₂-C₆H₄ | C₂H₅ |
| 1420 | CH₃ | 3-NO₂-C₆H₄ | CH₃ |
| 1421 | CH₃ | 3-NO₂-C₆H₄ | C₂H₅ |
| 1432 | CH₃ | 4-NO₂-C₆H₄ | CH₃ |
| 1433 | CH₃ | 4-NO₂-C₆H₄ | C₂H₅ |
| 1445 | CH₃ | 2-CH₃-C₆H₄ | C₂H₅ |
| 1456 | CH₃ | 3-CH₃-C₆H₄ | CH₃ |
| 1457 | CH₃ | 3-CH₃-C₆H₄ | C₂H₅ |
| 1469 | CH₃ | 4-CH₃-C₆H₄ | C₂H₅ |
| 1480 | CH₃ | 2,3-(CH₃)₂-C₆H₃ | CH₃ |
| 1481 | CH₃ | 2,3-(CH₃)₂-C₆H₃ | C₂H₅ |
| 1492 | CH₃ | 2,4-(CH₃)₂-C₆H₃ | CH₃ |
| 1493 | CH₃ | 2,4-(CH₃)₂-C₆H₃ | C₂H₅ |
| 1504 | CH₃ | 2,5-(CH₃)₂-C₆H₃ | CH₃ |
| 1505 | CH₃ | 2,5-(CH₃)₂-C₆H₃ | C₂H₅ |
| 1516 | CH₃ | 2,6-(CH₃)₂-C₆H₃ | CH₃ |
| 1517 | CH₃ | 2,6-(CH₃)₂-C₆H₃ | C₂H₅ |
| 1528 | CH₃ | 3,4-(CH₃)₂-C₆H₃ | CH₃ |
| 1529 | CH₃ | 3,4-(CH₃)₂-C₆H₃ | C₂H₅ |
| 1540 | CH₃ | 3,5-(CH₃)₂-C₆H₃ | CH₃ |
| 1541 | CH₃ | 3,5-(CH₃)₂-C₆H₃ | C₂H₅ |
| 1552 | CH₃ | 2-C₂H₅-C₆H₄ | CH₃ |
| 1553 | CH₃ | 2-C₂H₅-C₆H₄ | C₂H₅ |
| 1562 | CH₃ | 3-C₂H₅-C₆H₄ | CH₃ |
| 1563 | CH₃ | 3-C₂H₅-C₆H₄ | C₂H₅ |
| 1574 | CH₃ | 4-C₂H₅-C₆H₄ | CH₃ |
| 1575 | CH₃ | 4-C₂H₅-C₆H₄ | C₂H₅ |
| 1586 | CH₃ | 2-i-C₃H₇-C₆H₄ | CH₃ |
| 1587 | CH₃ | 2-i-C₃H₇-C₆H₄ | C₂H₅ |
| 1598 | CH₃ | 3-i-C₃H₇-C₆H₄ | CH₃ |
| 1599 | CH₃ | 3-i-C₃H₇-C₆H₄ | C₂H₅ |
| 1610 | CH₃ | 4-i-C₃H₇-C₆H₄ | CH₃ |
| 1611 | CH₃ | 4-i-C₃H₇-C₆H₄ | C₂H₅ |
| 1621 | CH₃ | 2-OH-C₆H₄ | H |
| 1622 | CH₃ | 2-OH-C₆H₄ | CH₃ |
| 1623 | CH₃ | 2-OH-C₆H₄ | C₂H₅ |
| 1624 | CH₃ | 2-OH-C₆H₄ | n-C₃H₇ |
| 1625 | CH₃ | 2-OH-C₆H₄ | i-C₃H₇ |
| 1626 | CH₃ | 2-OH-C₆H₄ | n-C₄H₉ |
| 1627 | CH₃ | 2-OH-C₆H₄ | t-C₄H₉ |
| 1628 | CH₃ | 2-OH-C₆H₄ | n-C₆H₁₃ |
| 1629 | CH₃ | 2-OH-C₆H₄ | Prop-1-en-3-yl |
| 1630 | CH₃ | 2-OH-C₆H₄ | (E)-1-Chlorprop-1-en-3-yl |
| 1631 | CH₃ | 2-OH-C₆H₄ | Propin-3-yl |
| 1632 | CH₃ | 2-OH-C₆H₄ | 3-Methyl-but-2-en-1-yl |
| 1633 | CH₃ | 3-OH-C₆H₄ | H |
| 1634 | CH₃ | 3-OH-C₆H₄ | CH₃ |
| 1635 | CH₃ | 3-OH-C₆H₄ | C₂H₅ |
| 1636 | CH₃ | 3-OH-C₆H₄ | n-C₃H₇ |
| 1637 | CH₃ | 3-OH-C₆H₄ | i-C₃H₇ |
| 1638 | CH₃ | 3-OH-C₆H₄ | n-C₄H₉ |
| 1639 | CH₃ | 3-OH-C₆H₄ | t-C₄H₉ |
| 1640 | CH₃ | 3-OH-C₆H₄ | n-C₆H₁₃ |
| 1641 | CH₃ | 3-OH-C₆H₄ | Prop-1-en-3-yl |
| 1642 | CH₃ | 3-OH-C₆H₄ | (E)-1-Chlorprop-1-en-3-yl |
| 1643 | CH₃ | 3-OH-C₆H₄ | Propin-3-yl |
| 1644 | CH₃ | 3-OH-C₆H₄ | 3-Methyl-but-2-en-1-yl |
| 1645 | CH₃ | 4-OH-C₆H₄ | H |
| 1646 | CH₃ | 4-OH-C₆H₄ | CH₃ |
| 1647 | CH₃ | 4-OH-C₆H₄ | C₂H₅ |
| 1648 | CH₃ | 4-OH-C₆H₄ | n-C₃H₇ |
| 1649 | CH₃ | 4-OH-C₆H₄ | i-C₃H₇ |
| 1650 | CH₃ | 4-OH-C₆H₄ | n-C₄H₉ |
| 1651 | CH₃ | 4-OH-C₆H₄ | t-C₄H₉ |
| 1652 | CH₃ | 4-OH-C₆H₄ | n-C₆H₁₃ |
| 1653 | CH₃ | 4-OH-C₆H₄ | Prop-1-en-3-yl |
| 1654 | CH₃ | 4-OH-C₆H₄ | (E)-1-Chlorprop-1-en-3-yl |
| 1655 | CH₃ | 4-OH-C₆H₄ | Propin-3-yl |
| 1656 | CH₃ | 4-OH-C₆H₄ | 3-Methyl-but-2-en-1-yl |
| 1657 | CH₃ | 2-OCH₃-C₆H₄ | H |
| 1658 | CH₃ | 2-OCH₃-C₆H₄ | CH₃ |
| 1659 | CH₃ | 2-OCH₃-C₆H₄ | C₂H₅ |
| 1670 | CH₃ | 3-OCH₃-C₆H₄ | CH₃ |
| 1671 | CH₃ | 3-OCH₃-C₆H₄ | C₂H₅ |
| 1683 | CH₃ | 4-OCH₃-C₆H₄ | C₂H₅ |
| 1694 | CH₃ | 2-OC₂H₅-C₆H₄ | CH₃ |
| 1695 | CH₃ | 2-OC₂H₅-C₆H₄ | C₂H₅ |
| 1706 | CH₃ | 3-OC₂H₅-C₆H₄ | CH₃ |
| 1707 | CH₃ | 3-OC₂H₅-C₆H₄ | C₂H₅ |
| 1719 | CH₃ | 4-OC₂H₅-C₆H₄ | C₂H₅ |
| 1730 | CH₃ | 2-O-(i-C₃H₇)-C₆H₄ | CH₃ |
| 1731 | CH₃ | 2-O-(i-C₃H₇)-C₆H₄ | C₂H₅ |
| 1742 | CH₃ | 3-O-(i-C₃H₇)-C₆H₄ | CH₃ |
| 1743 | CH₃ | 3-O-(i-C₃H₇)-C₆H₄ | C₂H₅ |
| 1754 | CH₃ | 4-O-(i-C₃H₇)-C₆H₄ | CH₃ |
| 1755 | CH₃ | 4-O-(i-C₃H₇)-C₆H₄ | C₂H₅ |
| 1766 | CH₃ | 2-O-(t-C₄H₉)-C₆H₄ | CH₃ |
| 1767 | CH₃ | 2-O-(t-C₄H₉)-C₆H₄ | C₂H₅ |
| 1778 | CH₃ | 3-O-(t-C₄H₉)-C₆H₄ | CH₃ |
| 1779 | CH₃ | 3-O-(t-C₄H₉)-C₆H₄ | C₂H₅ |
| 1790 | CH₃ | 4-O-(t-C₄H₉)-C₆H₄ | CH₃ |
| 1791 | CH₃ | 4-O-(t-C₄H₉)-C₆H₄ | C₂H₅ |
| 1803 | CH₃ | 2-CF₃-C₆H₄ | C₂H₅ |
| 1815 | CH₃ | 3-CF₃-C₆H₄ | C₂H₅ |
| 1826 | CH₃ | 4-CF₃-C₆H₄ | CH₃ |
| 1827 | CH₃ | 4-CF₃-C₆H₄ | C₂H₅ |
| 1837 | CH₃ | 2-NH₂-C₆H₄ | H |
| 1838 | CH₃ | 2-NH₂-C₆H₄ | CH₃ |
| 1839 | CH₃ | 2-NH₂-C₆H₄ | C₂H₅ |
| 1840 | CH₃ | 2-NH₂-C₆H₄ | n-C₃H₇ |
| 1841 | CH₃ | 2-NH₂-C₆H₄ | i-C₃H₇ |
| 1842 | CH₃ | 2-NH₂-C₆H₄ | n-C₄H₉ |
| 1843 | CH₃ | 2-NH₂-C₆H₄ | t-C₄H₉ |
| 1844 | CH₃ | 2-NH₂-C₆H₄ | n-C₆H₁₃ |
| 1845 | CH₃ | 2-NH₂-C₆H₄ | Prop-1-en-3-yl |
| 1846 | CH₃ | 2-NH₂-C₆H₄ | (E)-1-Chlorprop-1-en-3-yl |
| 1847 | CH₃ | 2-NH₂-C₆H₄ | Propin-3-yl |
| 1848 | CH₃ | 2-NH₂-C₆H₄ | 3-Methyl-but-2-en-1-yl |
| 1849 | CH₃ | 3-NH₂-C₆H₄ | H |
| 1850 | CH₃ | 3-NH₂-C₆H₄ | CH₃ |
| 1851 | CH₃ | 3-NH₂-C₆H₄ | C₂H₅ |
| 1852 | CH₃ | 3-NH₂-C₆H₄ | n-C₃H₇ |
| 1853 | CH₃ | 3-NH₂-C₆H₄ | i-C₃H₇ |
| 1854 | CH₃ | 3-NH₂-C₆H₄ | n-C₄H₉ |
| 1855 | CH₃ | 3-NH₂-C₆H₄ | t-C₄H₉ |
| 1856 | CH₃ | 3-NH₂-C₆H₄ | n-C₆H₁₃ |
| 1857 | CH₃ | 3-NH₂-C₆H₄ | Prop-1-en-3-yl |
| 1858 | CH₃ | 3-NH₂-C₆H₄ | (E)-1-Chlorprop-1-en-3-yl |
| 1859 | CH₃ | 3-NH₂-C₆H₄ | Propin-3-yl |
| 1860 | CH₃ | 3-NH₂-C₆H₄ | 3-Methyl-but-2-en-1-yl |
| 1861 | CH₃ | 4-NH₂-C₆H₄ | H |
| 1862 | CH₃ | 4-NH₂-C₆H₄ | CH₃ |
| 1863 | CH₃ | 4-NH₂-C₆H₄ | C₂H₅ |
| 1864 | CH₃ | 4-NH₂-C₆H₄ | n-C₃H₇ |
| 1865 | CH₃ | 4-NH₂-C₆H₄ | i-C₃H₇ |
| 1866 | CH₃ | 4-NH₂-C₆H₄ | n-C₄H₉ |
| 1867 | CH₃ | 4-NH₂-C₆H₄ | t-C₄H₉ |
| 1868 | CH₃ | 4-NH₂-C₆H₄ | n-C₆H₁₃ |
| 1869 | CH₃ | 4-NH₂-C₆H₄ | Prop-1-en-3-yl |
| 1870 | CH₃ | 4-NH₂-C₆H₄ | (E)-1-Chlorprop-1-en-3-yl |
| 1871 | CH₃ | 4-NH₂-C₆H₄ | Propin-3-yl |
| 1872 | CH₃ | 4-NH₂-C₆H₄ | 3-Methyl-but-2-en-1-yl |
| 1873 | CH₃ | 2-NMe₂-C₆H₄ | H |
| 1874 | CH₃ | 2-NMe₂-C₆H₄ | CH₃ |
| 1875 | CH₃ | 2-NMe₂-C₆H₄ | C₂H₅ |
| 1876 | CH₃ | 2-NMe₂-C₆H₄ | n-C₃H₇ |
| 1877 | CH₃ | 2-NMe₂-C₆H₄ | i-C₃H₇ |
| 1878 | CH₃ | 2-NMe₂-C₆H₄ | n-C₄H₉ |
| 1879 | CH₃ | 2-NMe₂-C₆H₄ | t-C₄H₉ |
| 1880 | CH₃ | 2-NMe₂-C₆H₄ | n-C₆H₁₃ |
| 1881 | CH₃ | 2-NMe₂-C₆H₄ | Prop-1-en-3-yl |
| 1882 | CH₃ | 2-NMe₂-C₆H₄ | (E)-1-Chlorprop-1-en-3-yl |
| 1883 | CH₃ | 2-NMe₂-C₆H₄ | Propin-3-yl |
| 1884 | CH₃ | 2-NMe₂-C₆H₄ | 3-Methyl-but-2-en-1-yl |
| 1885 | CH₃ | 3-NMe₂-C₆H₄ | H |
| 1886 | CH₃ | 3-NMe₂-C₆H₄ | CH₃ |
| 1887 | CH₃ | 3-NMe₂-C₆H₄ | C₂H₅ |
| 1888 | CH₃ | 3-NMe₂-C₆H₄ | n-C₃H₇ |
| 1889 | CH₃ | 3-NMe₂-C₆H₄ | i-C₃H₇ |
| 1890 | CH₃ | 3-NMe₂-C₆H₄ | n-C₄H₉ |
| 1891 | CH₃ | 3-NMe₂-C₆H₄ | t-C₄H₉ |
| 1892 | CH₃ | 3-NMe₂-C₆H₄ | n-C₆H₁₃ |
| 1893 | CH₃ | 3-NMe₂-C₆H₄ | Prop-1-en-3-yl |
| 1894 | CH₃ | 3-NMe₂-C₆H₄ | (E)-1-Chlorprop-1-en-3-yl |
| 1895 | CH₃ | 3-NMe₂-C₆H₄ | Propin-3-yl |
| 1896 | CH₃ | 3-NMe₂-C₆H₄ | 3-Methyl-but-2-en-1-yl |
| 1897 | CH₃ | 4-NMe₂-C₆H₄ | H |
| 1898 | CH₃ | 4-NMe₂-C₆H₄ | CH₃ |
| 1899 | CH₃ | 4-NMe₂-C₆H₄ | C₂H₅ |
| 1900 | CH₃ | 4-NMe₂-C₆H₄ | n-C₃H₇ |
| 1901 | CH₃ | 4-NMe₂-C₆H₄ | i-C₃H₇ |
| 1902 | CH₃ | 4-NMe₂-C₆H₄ | n-C₄H₉ |
| 1903 | CH₃ | 4-NMe₂-C₆H₄ | t-C₄H₉ |
| 1904 | CH₃ | 4-NMe₂-C₆H₄ | n-C₆H₁₃ |
| 1905 | CH₃ | 4-NMe₂-C₆H₄ | Prop-1-en-3-yl |
| 1906 | CH₃ | 4-NMe₂-C₆H₄ | (E)-1-Chlorprop-1-en-3-yl |
| 1907 | CH₃ | 4-NMe₂-C₆H₄ | Propin-3-yl |
| 1908 | CH₃ | 4-NMe₂-C₆H₄ | 3-Methyl-but-2-en-1-yl |
| 1909 | CH₃ | 2-Aminothiocarbonyl-C₆H₄ | H |
| 1910 | CH₃ | 2-Aminothiocarbonyl-C₆H₄ | CH₃ |
| 1911 | CH₃ | 2-Aminothiocarbonyl-C₆H₄ | C₂H₅ |
| 1912 | CH₃ | 2-Aminothiocarbonyl-C₆H₄ | n-C₃H₇ |
| 1913 | CH₃ | 2-Aminothiocarbonyl-C₆H₄ | i-C₃H₇ |
| 1914 | CH₃ | 2-Aminothiocarbonyl-C₆H₄ | n-C₄H₉ |
| 1915 | CH₃ | 2-Aminothiocarbonyl-C₆H₄ | t-C₄H₉ |
| 1916 | CH₃ | 2-Aminothiocarbonyl-C₆H₄ | n-C₆H₁₃ |
| 1917 | CH₃ | 2-Aminothiocarbonyl-C₆H₄ | Prop-1-en-3-yl |
| 1918 | CH₃ | 2-Aminothiocarbonyl-C₆H₄ | (E)-1-Chlorprop-1-en-3-yl |
| 1919 | CH₃ | 2-Aminothiocarbonyl-C₆H₄ | Propin-3-yl |
| 1920 | CH₃ | 2-Aminothiocarbonyl-C₆H₄ | 3-Methyl-but-2-en-1-yl |
| 1921 | CH₃ | 3-Aminothiocarbonyl-C₆H₄ | H |
| 1922 | CH₃ | 3-Aminothiocarbonyl-C₆H₄ | CH₃ |
| 1923 | CH₃ | 3-Aminothiocarbonyl-C₆H₄ | C₂H₅ |
| 1924 | CH₃ | 3-Aminothiocarbonyl-C₆H₄ | n-C₃H₇ |
| 1925 | CH₃ | 3-Aminothiocarbonyl-C₆H₄ | i-C₃H₇ |
| 1926 | CH₃ | 3-Aminothiocarbonyl-C₆H₄ | n-C₄H₉ |
| 1927 | CH₃ | 3-Aminothiocarbonyl-C₆H₄ | t-C₄H₉ |
| 1928 | CH₃ | 3-Aminothiocarbonyl-C₆H₄ | n-C₆H₁₃ |
| 1929 | CH₃ | 3-Aminothiocarbonyl-C₆H₄ | Prop-1-en-3-yl |
| 1930 | CH₃ | 3-Aminothiocarbonyl-C₆H₄ | (E)-1-Chlorprop-1-en-3-yl |
| 1931 | CH₃ | 3-Aminothiocarbonyl-C₆H₄ | Propin-3-yl |
| 1932 | CH₃ | 3-Aminothiocarbonyl-C₆H₄ | 3-Methyl-but-2-en-1-yl |
| 1933 | CH₃ | 4-Aminothiocarbonyl-C₆H₄ | H |
| 1934 | CH₃ | 4-Aminothiocarbonyl-C₆H₄ | CH₃ |
| 1935 | CH₃ | 4-Aminöthiocarbonyl-C₆H₄ | C₂H₅ |
| 1936 | CH₃ | 4-Aminothiocarbonyl-C₆H₄ | n-C₃H₇ |
| 1937 | CH₃ | 4-Aminothiocarbonyl-C₆H₄ | i-C₃H₇ |
| 1938 | CH₃ | 4-Aminöthiocarbonyl-C₆H₄ | n-C₄H₉ |
| 1939 | CH₃ | 4-Aminothiocarbonyl-C₆H₄ | t-C₄H₉ |
| 1940 | CH₃ | 4-Aminothiocarbonyl-C₆H₄ | n-C₆H₁₃ |
| 1941 | CH₃ | 4-Aminothiocarbonyl-C₆H₄ | Prop-1-en-3-yl |
| 1942 | CH₃ | 4-Aminothiocarbonyl-C₆H₄ | (E)-1-Chlorprop-1-en-3-yl |
| 1943 | CH₃ | 4-Aminothiocarbonyl-C₆H₄ | Propin-3-yl |
| 1944 | CH₃ | 4-Aminothiocarbonyl-C₆H₄ | 3-Methyl-but-2-en-1-yl |
| 1946 | CH₃ | 2-OCF₃-C₆H₄ | CH₃ |
| 1947 | CH₃ | 2-OCF₃-C₆H₄ | C₂H₅ |
| 1960 | CH₃ | 3-OCF₃-C₆H₄ | CH₃ |
| 1961 | CH₃ | 3-OCF₃-C₆H₄ | C₂H₅ |
| 1972 | CH₃ | 4-OCF₃-C₆H₄ | CH₃ |
| 1973 | CH₃ | 4-OCF₃-C₆H₄ | C₂H₅ |
| 1984 | CH₃ | 2-SCH₃-C₆H₄ | CH₃ |
| 1985 | CH₃ | 2-SCH₃-C₆H₄ | C₂H₅ |
| 1996 | CH₃ | 3-SCH₃-C₆H₄ | CH₃ |
| 1997 | CH₃ | 3-SCH₃-C₆H₄ | C₂H₅ |
| 2008 | CH₃ | 4-SCH₃-C₆H₄ | CH₃ |
| 2009 | CH₃ | 4-SCH₃-C₆H₄ | C₂H₅ |
| 2020 | CH₃ | 2-Methylsulfonyl-C₆H₄ | CH₃ |
| 2021 | CH₃ | 2-Methylsulfonyl-C₆H₄ | C₂H₅ |
| 2032 | CH₃ | 3-Methylsulfonyl-C₆H₄ | CH₃ |
| 2033 | CH₃ | 3-Methylsulfonyl-C₆H₄ | C₂H₅ |
| 2044 | CH₃ | 4-Methylsulfonyl-C₆H₄ | CH₃ |
| 2045 | CH₃ | 4-Methylsulfonyl-C₆H₄ | C₂H₅ |
| 2055 | CH₃ | 2-Methoxycarbonyl-C₆H₄ | H |
| 2056 | CH₃ | 2-Methoxycarbonyl-C₆H₄ | CH₃ |
| 2057 | CH₃ | 2-Methoxycarbonyl-C₆H₄ | C₂H₅ |
| 2058 | CH₃ | 2-Methoxycarbonyl-C₆H₄ | n-C₃H₇ |
| 2059 | CH₃ | 2-Methoxycarbonyl-C₆H₄ | i-C₃H₇ |
| 2060 | CH₃ | 2-Methoxycarbonyl-C₆H₄ | n-C₄H₉ |
| 2061 | CH₃ | 2-Methoxycarbonyl-C₆H₄ | t-C₄H₉ |
| 2062 | CH₃ | 2-Methoxycarbonyl-C₆H₄ | n-C₆H₁₃ |
| 2063 | CH₃ | 2-Methoxycarbonyl-C₆H₄ | Prop-1-en-3-yl |
| 2064 | CH₃ | 2-Methoxycarbonyl-C₆H₄ | (E)-1-Chlorprop-1-en-3-yl |
| 2065 | CH₃ | 2-Methoxycarbonyl-C₆H₄ | Propin-3-yl |
| 2066 | CH₃ | 2-Methoxycarbonyl-C₆H₄ | 3-Methyl-but-2-en-1-yl |
| 2067 | CH₃ | 3-Methoxycarbonyl-C₆H₄ | H |
| 2068 | CH₃ | 3-Methoxycarbonyl-C₆H₄ | CH₃ |
| 2069 | CH₃ | 3-Methoxycarbonyl-C₆H₄ | C₂H₅ |
| 2070 | CH₃ | 3-Methoxycarbonyl-C₆H₄ | n-C₃H₇ |
| 2071 | CH₃ | 3-Methoxycarbonyl-C₆H₄ | i-C₃H₇ |
| 2072 | CH₃ | 3-Methoxycarbonyl-C₆H₄ | n-C₄H₉ |
| 2073 | CH₃ | 3-Methoxycarbonyl-C₆H₄ | t-C₄H₉ |
| 2074 | CH₃ | 3-Methoxycarbonyl-C₆H₄ | n-C₆H₁₃ |
| 2075 | CH₃ | 3-Methoxycarbonyl-C₆H₄ | Prop-1-en-3-yl |
| 2076 | CH₃ | 3-Methoxycarbonyl-C₆H₄ | (E)-1-Chlorprop-1-en-3-yl |
| 2077 | CH₃ | 3-Methoxycarbonyl-C₆H₄ | Propin-3-yl |
| 2078 | CH₃ | 3-Methoxycarbonyl-C₆H₄ | 3-Methyl-but-2-en-1-yl |
| 2079 | CH₃ | 4-Methoxycarbonyl-C₆H₄ | H |
| 2080 | CH₃ | 4-Methöxycarbonyl-C₆H₄ | CH₃ |
| 2081 | CH₃ | 4-Methöxycarbonyl-C₆H₄ | C₂H₅ |
| 2082 | CH₃ | 4-Methoxycarbonyl-C₆H₄ | n-C₃H₇ |
| 2083 | CH₃ | 4-Methoxycarbonyl-C₆H₄ | i-C₃H₇ |
| 2084 | CH₃ | 4-Methoxycarbonyl-C₆H₄ | n-C₄H₉ |
| 2085 | CH₃ | 4-Methoxycarbonyl-C₆H₄ | t-C₄H₉ |
| 2086 | CH₃ | 4-Methöxycarbonyl-C₆H₄ | n-C₆H₁₃ |
| 2087 | CH₃ | 4-Methoxycarbonyl-C₆H₄ | Prop-1-en-3-yl |
| 2088 | CH₃ | 4-Methoxycarbonyl-C₆H₄ | (E)-1-Chlorprop-1-en-3-yl |
| 2089 | CH₃ | 4-Methoxycarbonyl-C₆H₄ | Propin-3-yl |
| 2090 | CH₃ | 4-Methoxycarbonyl-C₆H₄ | 3-Methyl-but-2-en-1-yl |
| 2091 | CH₃ | 2-Ethoxycarbonyl-C₆H₄ | H |
| 2092 | CH₃ | 2-Ethoxycarbonyl-C₆H₄ | CH₃ |
| 2093 | CH₃ | 2-Ethoxycarbonyl-C₆H₄ | C₂H₅ |
| 2094 | CH₃ | 2-Ethoxycarbonyl-C₆H₄ | n-C₃H₇ |
| 2095 | CH₃ | 2-Ethoxycarbonyl-C₆H₄ | i-C₃H₇ |
| 2096 | CH₃ | 2-Ethoxycarbonyl-C₆H₄ | n-C₄H₉ |
| 2097 | CH₃ | 2-Ethoxycarbonyl-C₆H₄ | t-C₄H₉ |
| 2098 | CH₃ | 2-Ethoxycarbonyl-C₆H₄ | n-C₆H₁₃ |
| 2099 | CH₃ | 2-Ethoxycarbonyl-C₆H₄ | Prop-1-en-3-yl |
| 2100 | CH₃ | 2-Ethoxycarbonyl-C₆H₄ | (E)-1-Chlorprop-1-en-3-yl |
| 2101 | CH₃ | 2-Ethoxycarbonyl-C₆H₄ | Propin-3-yl |
| 2102 | CH₃ | 2-Ethoxycarbonyl-C₆H₄ | 3-Methyl-but-2-en-1-yl |
| 2103 | CH₃ | 3-Ethoxycarbonyl-C₆H₄ | H |
| 2104 | CH₃ | 3-Ethoxycarbonyl-C₆H₄ | CH₃ |
| 2105 | CH₃ | 3-Ethoxycarbonyl-C₆H₄ | C₂H₅ |
| 2106 | CH₃ | 3-Ethoxycarbonyl-C₆H₄ | n-C₃H₇ |
| 2107 | CH₃ | 3-Ethoxycarbonyl-C₆H₄ | i-C₃H₇ |
| 2108 | CH₃ | 3-Ethoxycarbonyl-C₆H₄ | n-C₄H₉ |
| 2109 | CH₃ | 3-Ethoxycarbonyl-C₆H₄ | t-C₄H₉ |
| 2110 | CH₃ | 3-Ethoxycarbonyl-C₆H₄ | n-C₆H₁₃ |
| 2111 | CH₃ | 3-Ethoxycarbonyl-C₆H₄ | Prop-1-en-3-yl |
| 2112 | CH₃ | 3-Ethoxycarbonyl-C₆H₄ | (E)-1-Chlorprop-1-en-3-yl |
| 2113 | CH₃ | 3-Ethoxycarbonyl-C₆H₄ | Propin-3-yl |
| 2114 | CH₃ | 3-Ethoxycarbonyl-C₆H₄ | 3-Methyl-but-2-en-1-yl |
| 2115 | CH₃ | 4-Ethoxcarbonyl-C₆H₄ | H |
| 2116 | CH₃ | 4-Ethoxycarbonyl-C₆H₄ | CH₃ |
| 2117 | CH₃ | 4-Ethoxycarbonyl-C₆H₄ | C₂H₅ |
| 2118 | CH₃ | 4-Ethoxycarbonyl-C₆H₄ | n-C₃H₇ |
| 2119 | CH₃ | 4-Ethoxycarbonyl-C₆H₄ | i-C₃H₇ |
| 2120 | CH₃ | 4-Ethoxycarbonyl-C₆H₄ | n-C₄H₉ |
| 2121 | CH₃ | 4-Ethoxycarbonyl-C₆H₄ | t-C₄H₉ |
| 2122 | CH₃ | 4-Ethoxycarbonyl-C₆H₄ | n-C₆H₁₃ |
| 2123 | CH₃ | 4-Ethoxycarbonyl-C₆H₄ | Prop-1-en-3-yl |
| 2124 | CH₃ | 4-Ethoxycarbonyl-C₆H₄ | (E)-1-Chlorprop-1-en-3-yl |
| 2125 | CH₃ | 4-Ethoxycarbonyl-C₆H₄ | Propin-3-yl |
| 2126 | CH₃ | 4-Ethoxycarbonyl-C₆H₄ | 3-Methyl-but-2-en-1-yl |
| 2127 | CH₃ | 2-Aminocarbonyl-C₆H₄ | H |
| 2128 | CH₃ | 2-Aminocarbonyl-C₆H₄ | CH₃ |
| 2129 | CH₃ | 2-Aminocarbonyl-C₆H₄ | C₂H₅ |
| 2130 | CH₃ | 2-Aminocarbonyl-C₆H₄ | n-C₃H₇ |
| 2131 | CH₃ | 2-Aminocarbonyl-C₆H₄ | i-C₃H₇ |
| 2132 | CH₃ | 2-Aminocarbonyl-C₆H₄ | n-C₄H₉ |
| 2133 | CH₃ | 2-Aminocarbonyl-C₆H₄ | t-C₄H₉ |
| 2134 | CH₃ | 2-Aminocarbonyl-C₆H₄ | n-C₆H₁₃ |
| 2135 | CH₃ | 2-Aminocarbonyl-C₆H₄ | Prop-1-en-3-yl |
| 2136 | CH₃ | 2-Aminocarbonyl-C₆H₄ | (E)-1-Chlorprop-1-en-3-yl |
| 2137 | CH₃ | 2-Aminocarbonyl-C₆H₄ | Propin-3-yl |
| 2138 | CH₃ | 2-Aminocarbonyl-C₆H₄ | 3-Methyl-but-2-en-1-yl |
| 2139 | CH₃ | 3-Aminocarbonyl-C₆H₄ | H |
| 2140 | CH₃ | 3-Aminocarbonyl-C₆H₄ | CH₃ |
| 2141 | CH₃ | 3-Aminocarbonyl-C₆H₄ | C₂H₅ |
| 2142 | CH₃ | 3-Aminocarbonyl-C₆H₄ | n-C₃H₇ |
| 2143 | CH₃ | 3-Aminocarbonyl-C₆H₄ | i-C₃H₇ |
| 2144 | CH₃ | 3-Aminocarbonyl-C₆H₄ | n-C₄H₉ |
| 2145 | CH₃ | 3-Aminocarbonyl-C₆H₄ | t-C₄H₉ |
| 2146 | CH₃ | 3-Aminocarbonyl-C₆H₄ | n-C₆H₁₃ |
| 2147 | CH₃ | 3-Aminocarbonyl-C₆H₄ | Prop-1-en-3-yl |
| 2148 | CH₃ | 3-Aminocarbonyl-C₆H₄ | (E)-1-Chlorprop-1-en-3-yl |
| 2149 | CH₃ | 3-Aminocarbonyl-C₆H₄ | Propin-3-yl |
| 2150 | CH₃ | 3-Aminocarbonyl-C₆H₄ | 3-Methyl-but-2-en-1-yl |
| 2151 | CH₃ | 4-Aminocarbonyl-C₆H₄ | H |
| 2152 | CH₃ | 4-Aminocarbonyl-C₆H₄ | CH₃ |
| 2153 | CH₃ | 4-Aminocarbonyl-C₆H₄ | C₂H₅ |
| 2154 | CH₃ | 4-Aminocarbonyl-C₆H₄ | n-C₃H₇ |
| 2155 | CH₃ | 4-Aminocarbonyl-C₆H₄ | i-C₃H₇ |
| 2156 | CH₃ | 4-Aminocarbonyl-C₆H₄ | n-C₄H₉ |
| 2157 | CH₃ | 4-Aminocarbonyl-C₆H₄ | t-C₄H₉ |
| 2158 | CH₃ | 4-Aminocarbonyl-C₆H₄ | n-C₆H₁₃ |
| 2159 | CH₃ | 4-Aminocarbonyl-C₆H₄ | Prop-1-en-3-yl |
| 2160 | CH₃ | 4-Aminocarbonyl-C₆H₄ | (E)-1-Chlorprop-1-en-3-yl |
| 2161 | CH₃ | 4-Aminocarbonyl-C₆H₄ | Propin-3-yl |
| 2162 | CH₃ | 4-Aminocarbonyl-C₆H₄ | 3-Methyl-but-2-en-1-yl |
| 2163 | CH₃ | 2-(N-Methyl-aminocarbonyl)-C₆H₄ | H |
| 2164 | CH₃ | 2-(N-Methyl-aminocarbonyl)-C₆H₄ | CH₃ |
| 2165 | CH₃ | 2-(N-Methyl-aminocarbonyl)-C₆H₄ | C₂H₅ |
| 2166 | CH₃ | 2-(N-Methyl-aminocarbonyl)-C₆H₄ | n-C₃H₇ |
| 2167 | CH₃ | 2-(N-Methyl-aminocarbonyl)-C₆H₄ | i-C₃H₇ |
| 2168 | CH₃ | 2-(N-Methyl-aminocarbonyl)-C₆H₄ | n-C₄H₉ |
| 2169 | CH₃ | 2-(N-Methyl-amino carbonyl)-C₆H₄ | t-C₄H₉ |
| 2170 | CH₃ | 2-(N-Methyl-aminocarbonyl)-C₆H₄ | n-C₆H₁₃ |
| 2171 | CH₃ | 2-(N-Methyl-aminocarbonyl)-C₆H₄ | Prop-1-en-3-yl |
| 2172 | CH₃ | 2-(N-Methyl-aminocarbonyl)-C₆H₄ | (E)-1-Chlorprop-1-en-3-yl |
| 2173 | CH₃ | 2-(N-Methyl-aminocarbonyl)-C₆H₄ | Propin-3-yl |
| 2174 | CH₃ | 2-(N-Methyl-aminocarbonyl)-C₆H₄ | 3-Methyl-but-2-en-1-yl |
| 2175 | CH₃ | 3-(N-Methyl-aminocarbonyl)-C₆H₄ | H |
| 2176 | CH₃ | 3-(N-Methyl-aminocarbonyl)-C₆H₄ | CH₃ |
| 2177 | CH₃ | 3-(N-Methyl-aminocarbonyl)-C₆H₄ | C₂H₅ |
| 2178 | CH₃ | 3-(N-Methyl-aminocarbonyl)-C₆H₄ | n-C₃H₇ |
| 2179 | CH₃ | 3-(N-Methyl-aminocarbonyl)-C₆H₄ | i-C₃H₇ |
| 2180 | CH₃ | 3-(N-Methyl-aminocarbonyl)-C₆H₄ | n-C₄H₉ |
| 2181 | CH₃ | 3-(N-Methyl-aminocarbonyl)-C₆H₄ | t-C₄H₉ |
| 2182 | CH₃ | 3-(N-Methyl-aminocarbonyl)-C₆H₄ | n-C₆H₁₃ |
| 2183 | CH₃ | 3-(N-Methyl-aminocarbonyl)-C₆H₄ | Prop-1-en-3-yl |
| 2184 | CH₃ | 3-(N-Methyl-aminocarbonyl)-C₆H₄ | (E)-1-Chlorprop-1-en-3-yl |
| 2185 | CH₃ | 3-(N-Methyl-aminocarbonyl)-C₆H₄ | Propin-3-yl |
| 2186 | CH₃ | 3-(N-Methyl-aminocarbonyl)-C₆H₄ | 3-Methyl-but-2-en-1-yl |
| 2187 | CH₃ | 4-(N-Methyl-aminocarbonyl)-C₆H₄ | H |
| 2188 | CH₃ | 4-(N-Methyl-aminocarbonyl)-C₆H₄ | CH₃ |
| 2189 | CH₃ | 4-(N-Methyl-aminocarbonyl)-C₆H₄ | C₂H₅ |
| 2190 | CH₃ | 4-(N-Methyl-aminocarbonyl)-C₆H₄ | n-C₃H₇ |
| 2191 | CH₃ | 4-(N-Methyl-aminocarbonyl)-C₆H₄ | i-C₃H₇ |
| 2192 | CH₃ | 4-(N-Methyl-aminocarbonyl)-C₆H₄ | n-C₄H₉ |
| 2193 | CH₃ | 4-(N-Methyl-aminocarbonyl)-C₆H₄ | t-C₄H₉ |
| 2194 | CH₃ | 4-(N-Methyl-aminocarbonyl)-C₆H₄ | n-C₆H₁₃ |
| 2195 | CH₃ | 4-(N-Methyl-aminocarbonyl)-C₆H₄ | Prop-1-en-3-yl |
| 2196 | CH₃ | 4-(N-Methyl-aminocarbonyl)-C₆H₄ | (E)-1-Chlorprop-1-en-3-yl |
| 2197 | CH₃ | 4-(N-Methyl-aminocarbonyl)-C₆H₄ | Propin-3-yl |
| 2198 | CH₃ | 4-(N-Methyl-aminocarbonyl)-C₆H₄ | 3-Methyl-but-2-en-1-yl |
| 2199 | CH₃ | 2-Dimethyl-aminocarbonyl-C₆H₄ | H |
| 2200 | CH₃ | 2-Dimethyl-aminocarbonyl-C₆H₄ | CH₃ |
| 2201 | CH₃ | 2-Dimethyl-aminocarbonyl-C₆H₄ | C₂H₅ |
| 2202 | CH₃ | 2-Dimethyl-aminocarbonyl-C₆H₄ | n-C₃H₇ |
| 2203 | CH₃ | 2-Dimethyl-aminocarbonyl-C₆H₄ | i-C₃H₇ |
| 2204 | CH₃ | 2-Dimethyl-aminocarbonyl-C₆H₄ | n-C₄H₉ |
| 2205 | CH₃ | 2-Dimethyl-aminocarbonyl-C₆H₄ | t-C₄H₉ |
| 2206 | CH₃ | 2-Dimethyl-aminocarbonyl-C₆H₄ | n-C₆H₁₃ |
| 2207 | CH₃ | 2-Dimethyl-aminocarbonyl-C₆H₄ | Prop-1-en-3-yl |
| 2208 | CH₃ | 2-Dimethyl-aminocarbonyl-C₆H₄ | (E)-1-Chlorprop-1-en-3-yl |
| 2209 | CH₃ | 2-Dimethyl-aminocarbonyl-C₆H₄ | Propin-3-yl |
| 2210 | CH₃ | 2-Dimethyl-aminocarbonyl-C₆H₄ | 3-Methyl-but-2-en-1-yl |
| 2211 | CH₃ | 3-Dimethyl-aminocarbonyl-C₆H₄ | H |
| 2212 | CH₃ | 3-Dimethyl-aminocarbonyl-C₆H₄ | CH₃ |
| 2213 | CH₃ | 3-Dimethyl-aminocarbonyl-C₆H₄ | C₂H₅ |
| 2214 | CH₃ | 3-Dimethyl-aminocarbonyl-C₆H₄ | n-C₃H₇ |
| 2215 | CH₃ | 3-Dimethyl-aminocarbonyl-C₆H₄ | i-C₃H₇ |
| 2216 | CH₃ | 3-Dimethyl-aminocarbonyl-C₆H₄ | n-C₄H₉ |
| 2217 | CH₃ | 3-Dimethyl-aminocarbonyl-C₆H₄ | t-C₄H₉ |
| 2218 | CH₃ | 3-Dimethyl-aminocarbonyl-C₆H₄ | n-C₆H₁₃ |
| 2219 | CH₃ | 3-Dimethyl-aminocarbonyl-C₆H₄ | Prop-1-en-3-yl |
| 2220 | CH₃ | 3-Dimethyl-aminocarbonyl-C₆H₄ | (E)-1-Chlorprop-1-en-3-yl |
| 2221 | CH₃ | 3-Dimethyl-aminocarbonyl-C₆H₄ | Propin-3-yl |
| 2222 | CH₃ | 3-Dimethyl-aminocarbonyl-C₆H₄ | 3-Methyl-but-2-en-1-yl |
| 2223 | CH₃ | 4-Dimethyl-aminocarbonyl-C₆H₄ | H |
| 2224 | CH₃ | 4-Dimethyl-aminocarbonyl-C₆H₄ | CH₃ |
| 2225 | CH₃ | 4-Dimethyl-aminocarbonyl-C₆H₄ | C₂H₅ |
| 2226 | CH₃ | 4-Dimethyl-aminocarbonyl-C₆H₄ | n-C₃H₇ |
| 2227 | CH₃ | 4-Dimethyl-aminocarbonyl-C₆H₄ | i-C₃H₇ |
| 2228 | CH₃ | 4-Dimethyl-aminocarbonyl-C₆H₄ | n-C₄H₉ |
| 2229 | CH₃ | 4-Dimethyl-aminocarbonyl-C₆H₄ | t-C₄H₉ |
| 2230 | CH₃ | 4-Dimethyl-aminocarbonyl-C₆H₄ | n-C₆H₁₃ |
| 2231 | CH₃ | 4-Dimethyl-aminocarbonyl-C₆H₄ | Prop-1-en-3-yl |
| 2232 | CH₃ | 4-Dimethyl-aminocarbonyl-C₆H₄ | (E)-1-Chlorprop-1-en-3-yl |
| 2233 | CH₃ | 4-Dimethyl-aminocarbonyl-C₆H₄ | Propin-3-yl |
| 2234 | CH₃ | 4-Dimethyl-aminocarbonyl-C₆H₄ | 3-Methyl-but-2-en-1-yl |

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blattund Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusa7rium- und Verticillium-Arten an verschiedenen Pflanzen, Plasmopara viticola an Reben, Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung des ortho-substituierten Benzylesters einer Cyclopropancarbonsäure gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäuredi-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazonp)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäureanilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecylmorpholin bzw. dessen Salze, N-[3-(p-tert.-Butylphenyl)-2-methylpropyl]-cis-2,6-dimethyl- morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H- 1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'imidazol-yl- harnstoff, 1-(4-Chlorphenoxy)-3,3-dimethyl-1-(1H-1,2,4-triazol-1-yl)-2- butanon, 1-(4-Chlorphenoxy)-3,3-dimethyl-1-(1H-1,2,4-triazol-1-yl)-2- butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl (2)-alaninat, DL-N-(2,6-Dimethyl-phenyl)-N-(2'-methoxyacetyl)-alanin-methyl- ester, N-(2,6-Dimethylphenyl)-N-chloracetyl-D, L-2-aminobutyrolacton, DL-N-(2,6-Dimethylphenyl) -N- (phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi; Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria; Melanoplus bivittatus, Melanoplus femur-rubrum, Melanoplus mexicanus, Melanoplus sanguinipes, Nelanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure,. Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
- I.: 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.% des Wirkstoffs enthält.
- II.: 30 Gew.-Teile einer erfindungsemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
- III.: 10 Gew.-Teile einer erfindungsemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
- IV.: 20 Gew.-Teile einer erfindungsemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
- V.: 80 Gew.-Teile einer erfindungsemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
- VI.: Man vermischt 90 Gew.-Teile einer erfindungsemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.%).
- VII.: 20 Gew.-Teile einer erfindungsemäßen Verbindung Nr. 11 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
- VIII:: 20 Gew.-Teile einer erfindungsemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt Werden.

### Synthesebeispiele:

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1

### Darstellung von (E,E)-2-Methoxyimino-2-[2'-(1''-methyl,1''acetyl)-iminooxymethyl]phenylessigsäuremethylester.

Zu 6,4 g (0,21 mol) Natriumhydrid (80 %) in 150 ml trockenem Dimethylformamid gibt man unter Schutzgas und leichtem Kühlen bei Raumtemperatur 21 g (0,21 mol) Diacetylmonoxim und rührt den Ansatz 30 min bei Raumtemperatur. Anschließend wird eine Lösung von 60 g (0,21 mol) 2-Methoxyimino-2-(2'brommethyl)phenylessigsäure-. methylester in 360 ml Dimethylformamid zugetropft und 16 h bei Raumtemperatur gerührt. Nach Zugabe von 10 %iger Salzsäure wird mit Methyl-tert.-butyl-ether extrahiert. Die vereinigten organischen Phasen werden mit Wasser gewaschen, über Na₂SO₄ getrocknet und eingeengt. Der Rückstand wird in wenig kaltem Methanol suspendiert. Nach Absaugen erhält man 38 g (59 %) der Titelverbindung als hellbraune Kristalle mit einem Schmelzpunkt von 69 - 71°C.

¹H-NMR (CDCl₃):δ= 1,87(s,3H); 2,30(s,3H); 3,85(s,3H); 4,05(s,3H); 5,15(s,2H); 7,17-7,48(m,4H) ppm.

### Beispiel 2

### Darstellung von (E,E,E)-2-Methoxyimino-2-[2'-(1''-methyl,1''-(1'''-ethoxyiminoethyl))iminooxymethyl]phenylessigsäuremethylester

Zur Lösung von 2,5 g (8,2 mmol) (E,E)-2-Methoxyimino-2-[2'-(1''-methyl, 1"-acetyl)iminooxymethyl]phenylessigsäuremethylester in 60 ml warmen Methanol gibt man nach Abkühlen auf Raumtemperatur 0,96 g (9,8 mmol) O-Ethylhydroxylamin-Hydrochlorid sowie 0,6 g trockene Molekularsiebperlen (3 A) und läßt 5 Tage bei Raumtemperatur stehen. Nach Abfiltrieren des Molekularsiebes wird die Lösung eingeengt, der Rückstand zwischen Methyl-tert.-butyl-ether und Wasser verteilt, die organische Phase mit Wasser gewaschen, über Na₂SO₄ getrocknet und eingeengt. Nach Verreiben des Rückstandes mit n-Hexan und Absaugen erhält man . 1,8 g (63 %) der Titelverbindung als hellgelbe Kristalle mit einem Schmelzpunkt von 69 - 72°C.

¹H-NMR (CDCl₃) : δ= 1,27(t,3H); 1,96(s,3H); 1,99(s,3H); 3,84 (s,3H); 4,04(s,3H); 4,17(q,2H); 5,06(s,2H); 7,17-7,49(m,4H) ppm

### Beispiel 3 (nicht erfindungsgemäß)

### Darstellung von (E,E,E)-2-Methoxyimino-2-[2'-(1''-methyl, 1''-(1'''-ethoxyiminoethyl))iminooxymethyl]phenylessigsäuremonomethylamid

0,90 g (2,60 mmol) (E,E,E)-2-Methoxyimino-2-[2'-(1"-methyl, 1''-(1'''-ethoxyiminoethyl))iminooxymethyl]phenylessigäsuremethylester werden in 50 ml Tetrahydrofuran gelöst, mit 2,0 g 40%iger wäßriger Monomethylaminlösung versetzt und 16 h bei Raumtemperatur gerührt. Anschließend wird mit Wasser versetzt, mit Methyltert.-butyl-ether extrahiert, die organische Phase mit Wasser gewaschen, über Na₂SO₄ getrocknet und einrotiert. Als Rückstand verbleiben 0,80 g (89 %) der Titelverbindung als hellgelbes Öl.

¹H-NMR (CDCl₃):δ = 1,28(t,3H); 1,97(s,3H); 1,99(s,3H); 2,90(d,3H); 3,96(s,3H); 4,18(q,2H); 5,07(s,2H); 6,74(br,1H); 7,17-7,48(m,4H) ppm

### Beispiel 4

### Darstellung von 4-Hydroxyimino-2,2-dimethylpentan-3-on

Zu 96 g (0,84 mol) 2,2-Dimethyl-3-pentanon in 960 g Toluol wird. eine Lösung aus 40 g Chlorwasserstoff in 156 g Diethylether bei Raumtemperatur zugetropft. Nach Abkühlen auf -10°C wird eine Lösung von 95 g n-Butylnitrit in 470 g Diethylether zugetropft. Man läßt 4 Stunden bei -10°C bis 0°C rühren und läßt anschließend auf Raumtemperatur kommen. Nach insgesamt 16 h wird der Reaktionsansatz dreimal mit je 1 l Eiswasser gewaschen und anschließend zweimal mit je 1 l 1M-Natronlauge extrahiert. Die alkalische Phase wird abgetrennt und mit 20 %iger Schwefelsäure neutralisiert. Das Rohprodukt wird abgesaugt und nach dem Trocknen aus n-Hexan umkristallisiert. Man erhält 66 g (55 %) der Titelverbindung als hellgelbes Pulver vom Schmelzpunkt 107 - 110°C.

¹H-NMR (CDCl₃):δ= 1,29(s,9H); 1,99(s,3H); 8,30(s,1H) ppm.

### Beispiel 5

### Darstellung von (E)-2-Methoxyimino-2-[2'-(1''-methyl, 1''-(1''',1'''-dimethylethylcarbonyl))iminooxymethyl]phenylessigsäuremethylester

Zu 6,4 g (0,21 mol) Natriumhydrid (80 %) in 150 ml trockenem Dimethylformamid gibt man unter Schutzgas portionsweise 25 g (0,17 mol) 4-Hydroxyimino-2,2-dimethylpentan-3-on, wobei sich das Reaktionsgemisch bis auf 50°C erwärmt. Man läßt 30 min nachrühren, tropft anschließend eine Lösung von 50 g (0,17 mol) 2-Methoxyimino-2-(2'-brommethyl)phenylessigsäuremethylester in 300 ml Dimethylformamid zu und läßt 16 h bei Raumtemperatur rühren. Nach Zugabe von 10 %iger Salzsäure wird mit Methyl-tert.-butyl-ether extrahiert. Die vereinigten organischen Phasen werden mit Wasser gewaschen, über Na₂SO₂ getrocknet und eingeengt. Der schwarze ölige Rückstand wird säulenchromatographisch an Kieselgel (Methyl-tert-butyl-ether/n-Hexan) gereinigt und das so erhaltene Rohrprodukt in eiskaltem Methanol suspendiert. Nach Absaugen erhält man 24 g (41 %) der Titelverbindung als fast farbloses Pulver vom Schmelzpunkt 58 - 62°C.

¹H-NMR (CDCl₃) : δ= 1.19(s,9H); 1,90(s,3H); 3,83(s,3H); 4,04(s,3H); 5,11(s,2H); 7,18-7,45(m,4H) ppm.

### Beispiel 6

### Darstellung von (E)-2-Methoxyimino-2-[2'-(1''-methyl, 1''-(1'''-(6''''-(4'''''-chlorphenyl)hexyloxyimino), 2''', 2'''-dimethylpropyl))iminooxymethyl]phenylessigsäuremethylester

Zur Lösung von 3,0 g (8,6 mmol) (E)-2-Methoxyimino-2-[2'-(1''-methyl, 1''-(1''', 1'''-dimethylethylcarbonyl))-iminooxymethyl]-phenylessigsäuremethylester in 60 ml warmen Methanol gibt man nach Abkühlen auf Raumtemperatur 5,9 g (26 mmol) 0-6-(4'-Chlorphenyl)-hexylhydroxylamin, 3,6 g trockene Molekularsiebperlen (3 A) sowie 1,6 g (8,6 mmol) p-Toluolsulfonsäurehydrat und erhitzt 3 h am Rückfluß. Nach Abfiltrieren des Molekularsiebes wird die Lösung eingeengt, der Rückstand zwischen Methyl-tert.-butyl-ether und Wasser verteilt, die organische Phase mit Wasser gewaschen, über Na₂SO₄ getrocknet und eingeengt. Nach Säulenchromatographie an Kieselgel (Hexan/Methyl-tert.-butylether) erhält man 3,8 g (79 %) der Titelverbindung als hellgelbes Öl.

¹H-NMR (CDCl₃):δ= 1,09(s,9H); 1,26-1,42(m,4H); 1,52-1,67(m,4H); 1,90(s,3H); 2,57(t,2H); 3,84(s,3H); 3,99(t,2H); 4,03(s,3H); 5,02(s,2H); 7,07-7,47(m,8H) ppm.

### Beispiel 7

### Darstellung von (E)-2-Methoxyimino-2-[2'-(1"-methyl, 1''-(1'''-(6''''-(4'''''-chlorphenyl)hexyloxyimino), 2''', 2'''-dimethylpropyl))iminooxymethyl]phenylessigsäuremonomethylamid

2,8 g (5,0 mmol) (E)-2-Methoxyimino-2-[2'-(1''-methyl, 1''-(1'''-(6''''-(4'''''-chlorphenyl)hexyloxyimino), 2''', 2'''-dimethylpropyl)iminooxymethyl]phenylessigsäuremethylester werden in 10 ml Tetrahydrofuran gelöst, mit 3,9 g 40 %iger wäßriger Monomethylaminlösung versetzt und 16 h bei Raumtemperatur gerührt. Anschließend wird mit Wasser versetzt, mit Methyl-tert.butyl-ether extrahiert, die organische Phase mit Wasser gewaschen, über Na₂SO₄ getrocknet und einrotiert. Als Rückstand verbleiben 2,3 g (82 %) der Titelverbindung als farbloses Öl.

¹H-NMR (CDCl₃):δ= 1,08(s,9H); 1,26-1,41(m,4H); 1,53-1,67(m,4H); 1,89(s,3H); 2,56(t,2H); 2,87(d,3H); 3,93(s,3H); 3,99(t,2H); 5,02(s,2H); 6,74(s,breit,1H); 7,05-7,45(m,8H) ppm.

### Beispiel 8

### Darstellung von (E)-2-Methoxyimino-2-[2'-(1"-Methyl, 1"(1'''methoxyimino, 1'''para-methoxyphenyl)-methyl)iminooxymethyl]phenylessigsäuremonomethylthioamid (Verb. II.01, Tabelle II)

1,9 g (4,5 mmol) (E)-2-Methoxyimino-2-[2'-(1"-Methyl, 1"(1'''-methoxyimino, 1'''para-methoxyphenyl)-methyl)iminooxymethyl]-phenylessigsäuremonomethylamid werden in 80 ml Xylol gelöst, mit 1,8 g (4,5 mmol) Lawesson-Reagenz versetzt und 45 min bei 100°C gerührt.

Die Reaktionslösung wird eingeengt und der Rückstand säulenchromatographisch gereinigt.

Man isoliert 1,5 g (75 %) der Titelverbindung als Isomerengemisch in Form eines gelblichen Öls.
IR [cm⁻¹] Film:
834, 977, 1027, 1065, 1175, 1251, 1358, 1512, 1608, 2936, 3330

### Beispiel 9

### Isomerisierung von (E-)-2-Methoxyimono-2-[2'-(1"-Methyl, 1"(1'''-(Z/E)methoxyimino, 1"'-phenyl)methyl)-(E)-iminooxymethyl]-phenylessigsäuremonomethylamid zu (E)-2-Methoxyimino-2-[2'-(!"-Methyl, 1"(1"'-(E)-methoxyimino, 1'''-phenyl)methyl)-(E)-iminooxymethyl]-phenylessigsäuremonomethylamid:

5 g E,E,E/E,Z,E-Isomeremgemisch (30:70) werden in 50 ml Methanol gelöst, mit 15 ml mit HCl, gesättigtem Methanol versetzt und 18 h bei Raumtemperatur stehengelassen. Man gibt die Reaktionslösung auf Eiswasser, extrahiert mit Dichlormethan und trocknet über Na₂SO₄. Nach Einengen am Rotationsverdampfer erhält man 5 g eines Öls (E,E,E : E,Z,E, ca. 65:35). Das gewünschte (E,E,E)-Isomer kristallisiert bei Zugabe von Methanol (2,1 g = 42 %) in Form eines farblosen Feststoffs.
Smp. (E,E,E-Isomer): 134-136°C

Anmerkung: Das Filtrat kann mit Methanol HCl erneut isomerisiert werden.

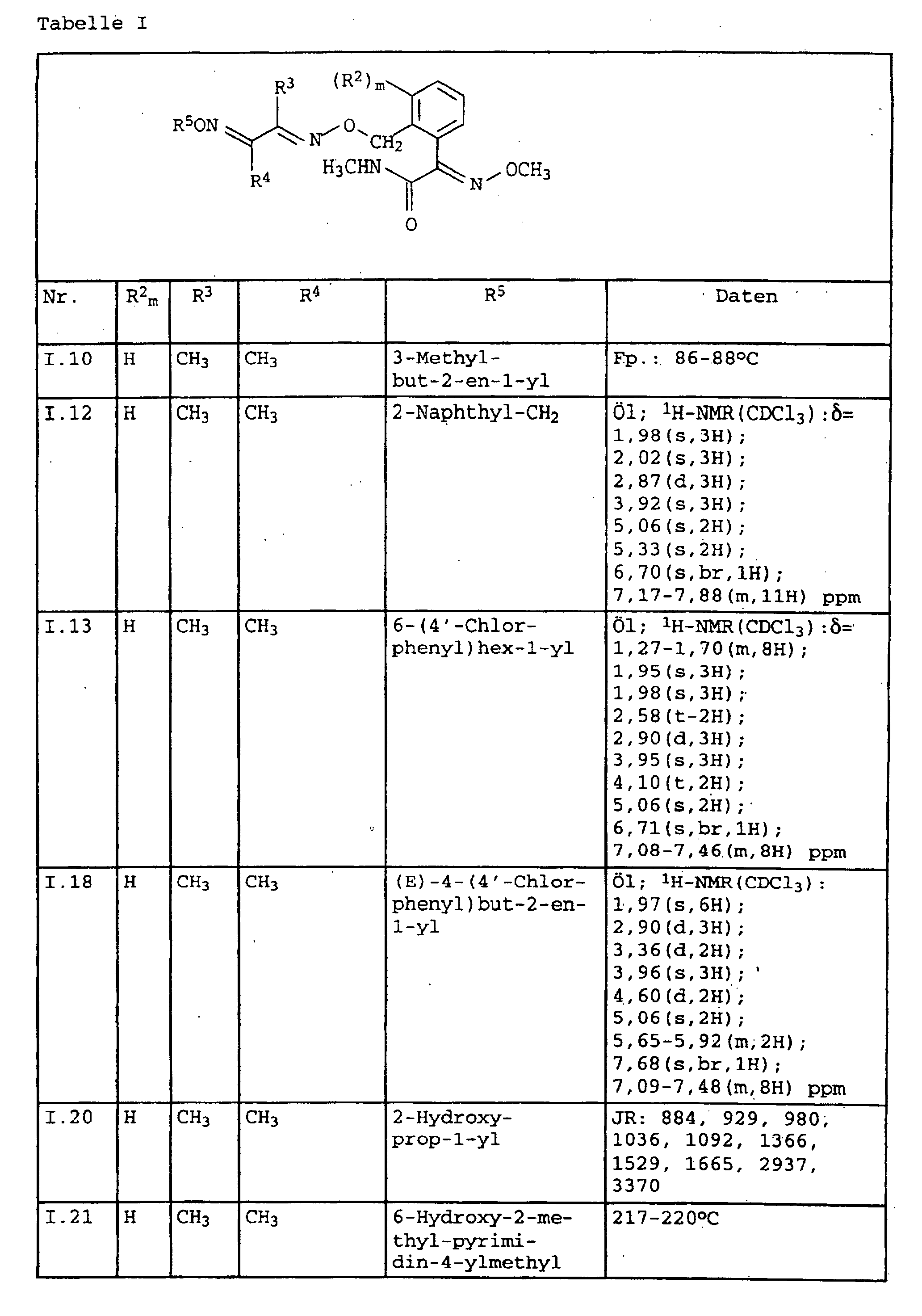

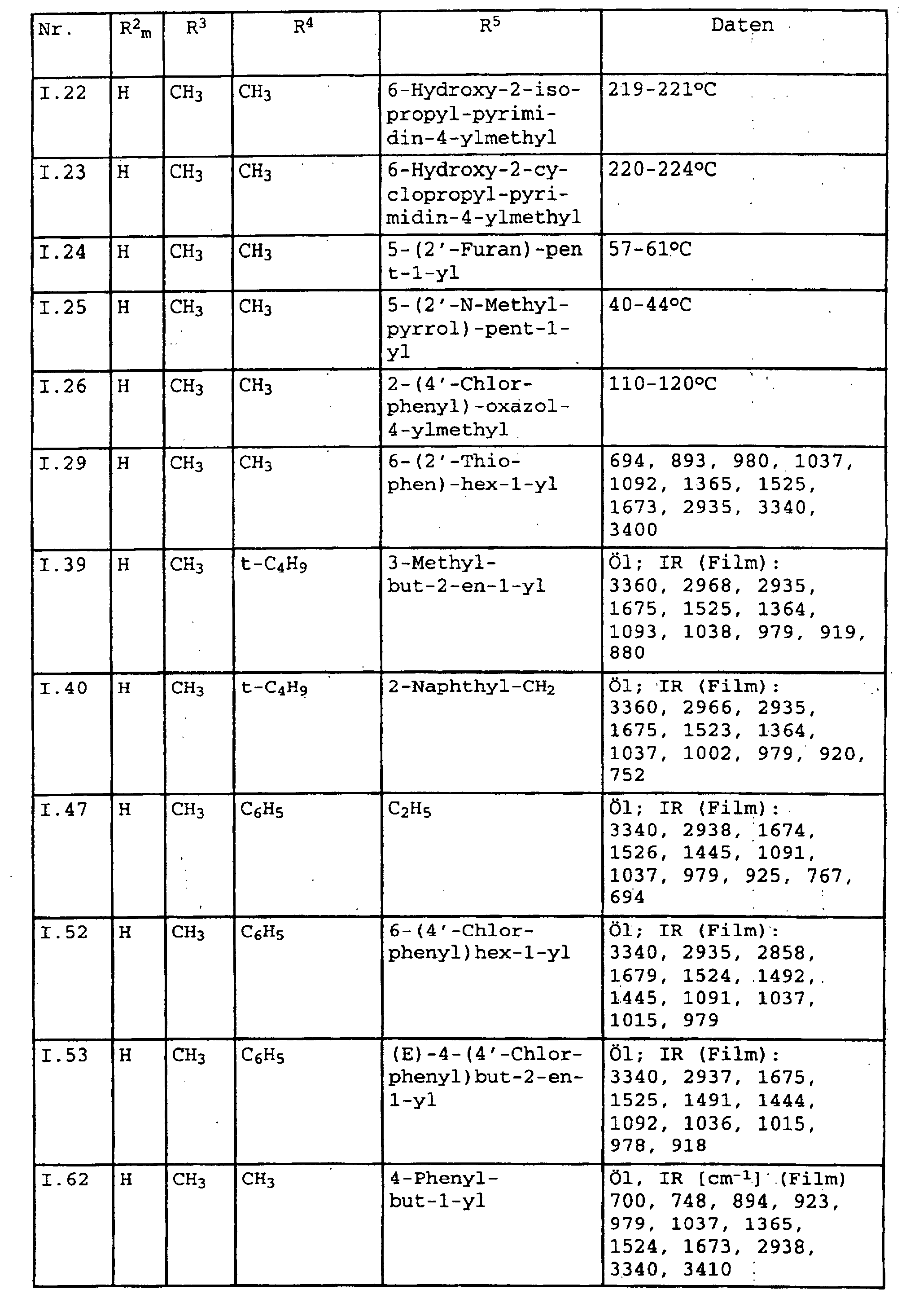

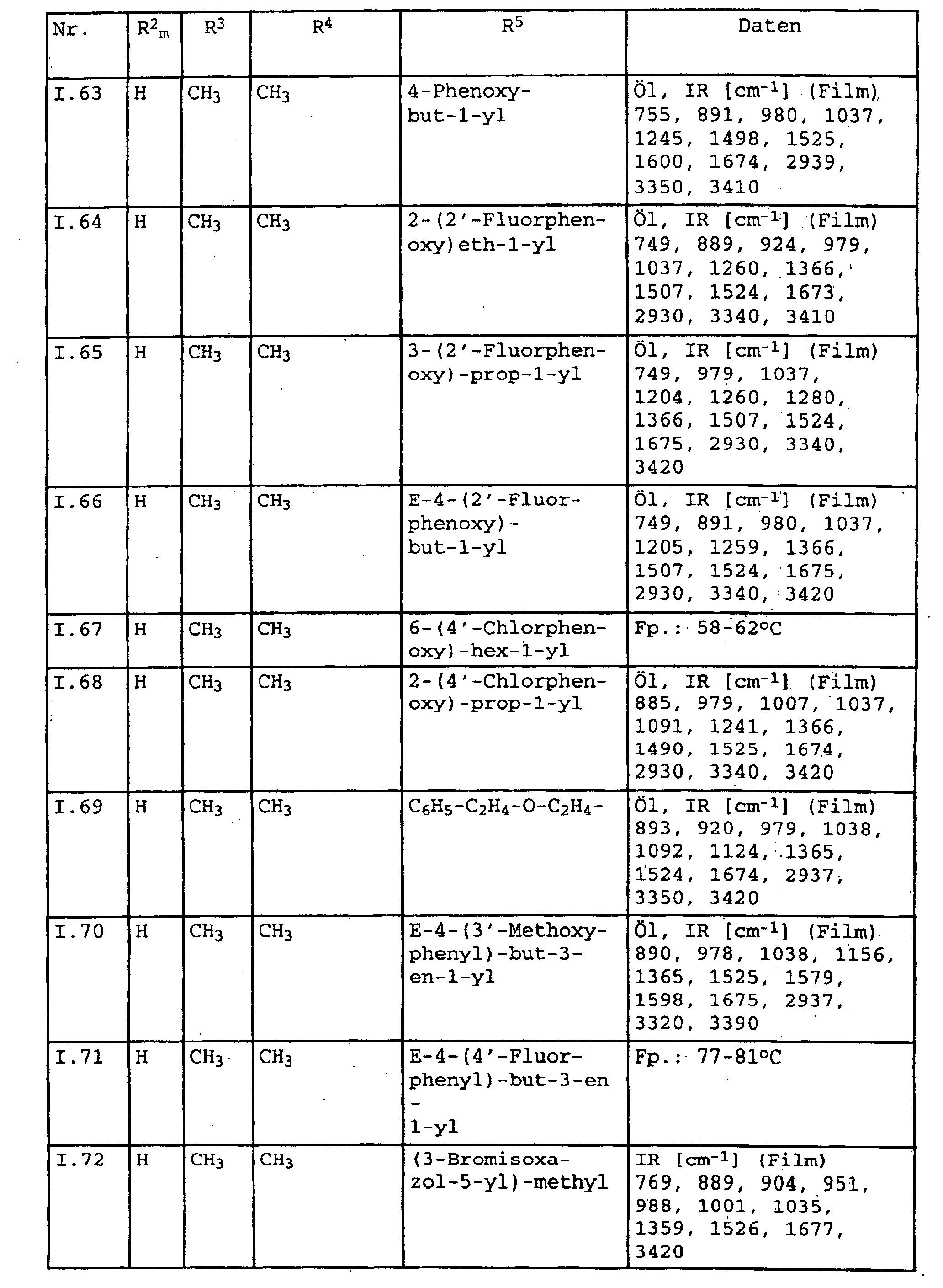

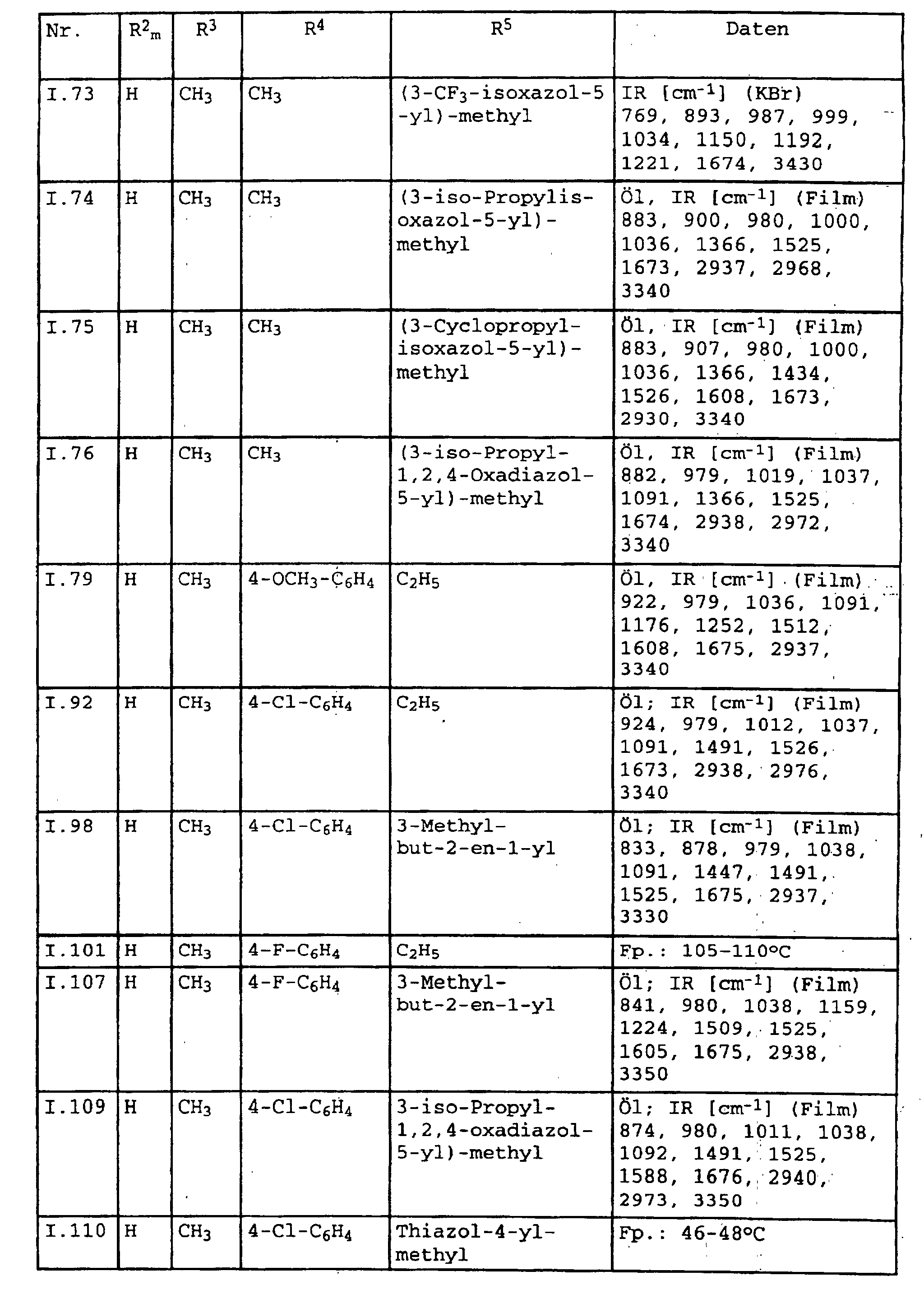

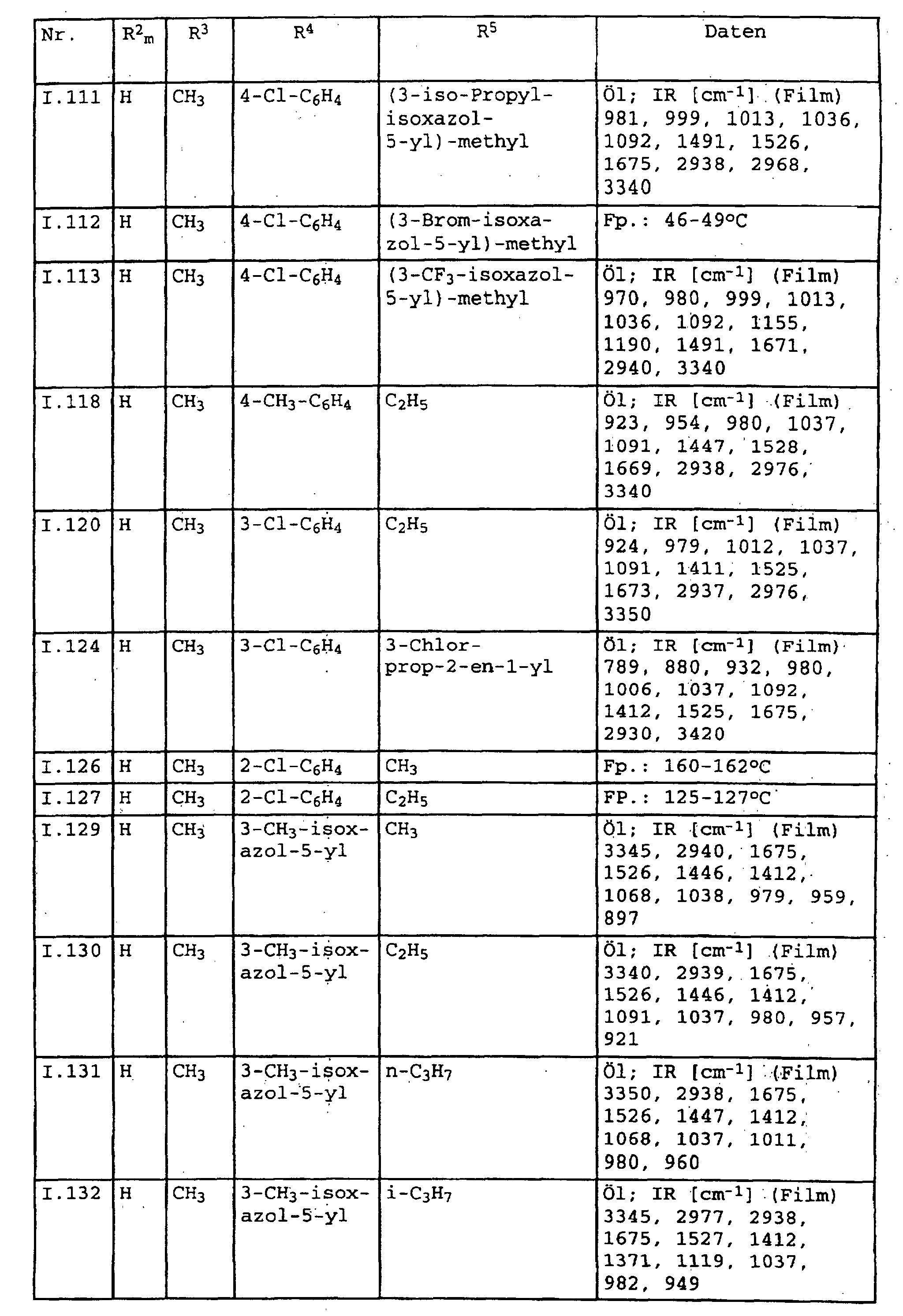

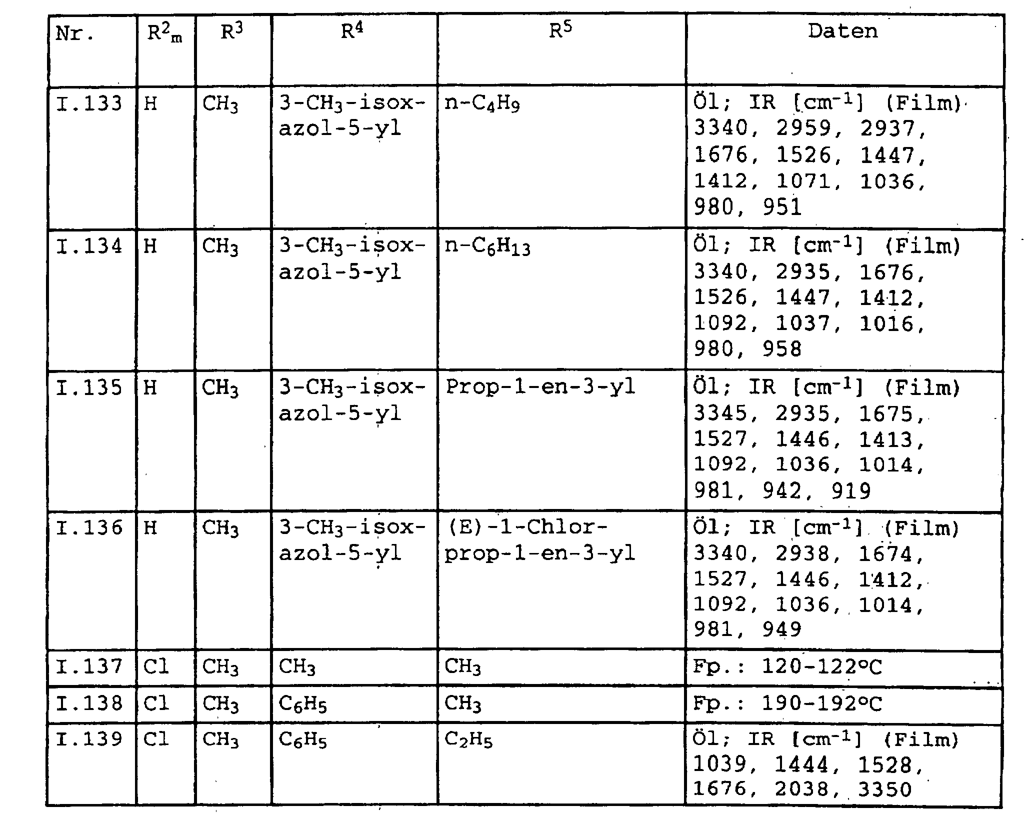

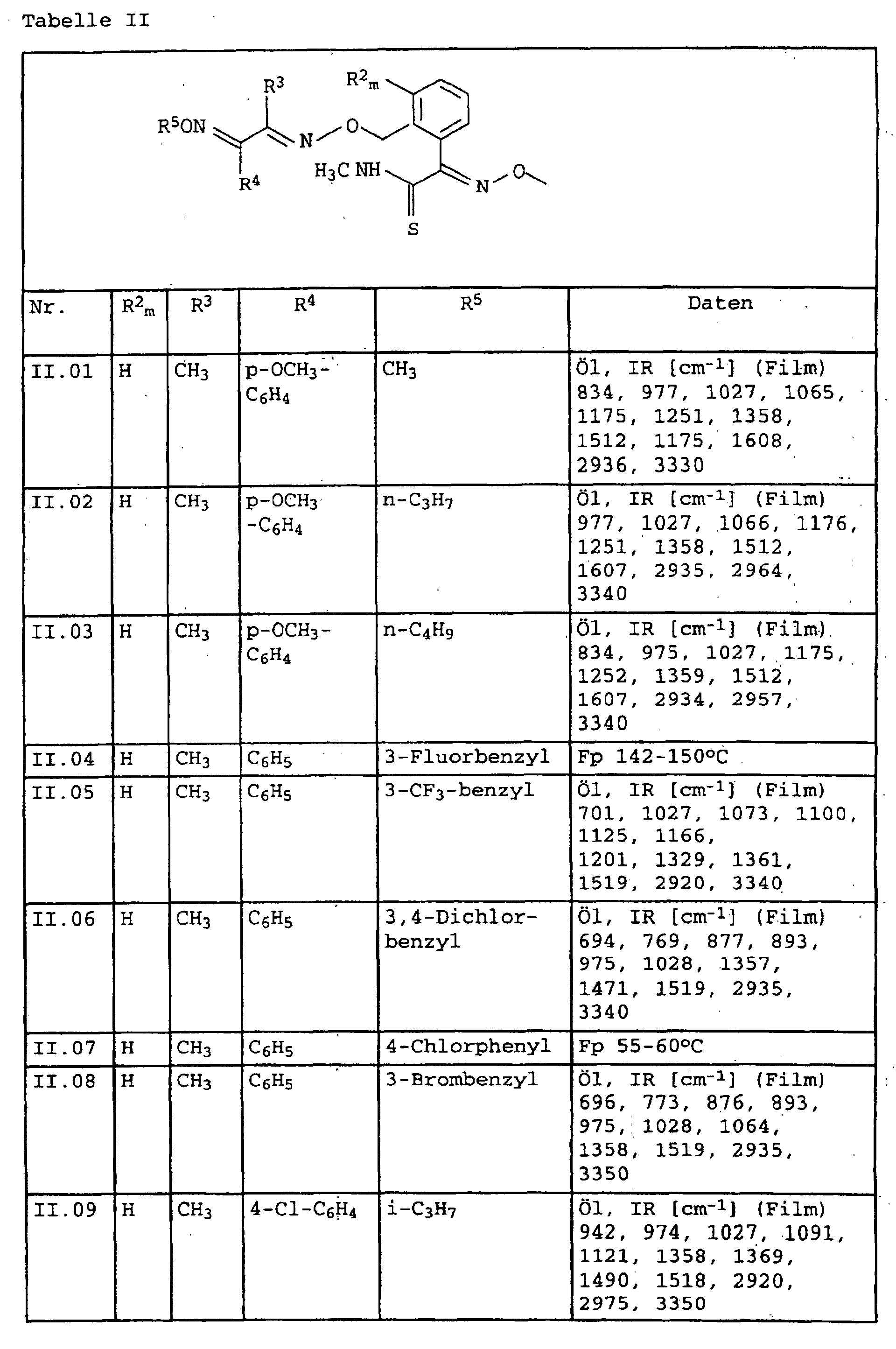

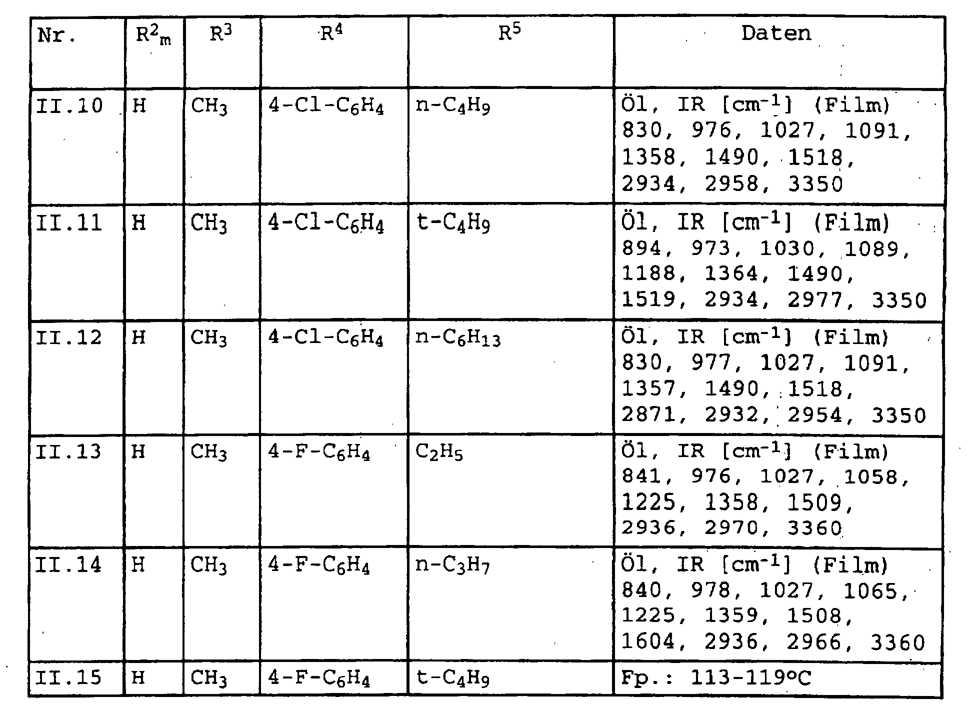

### Beispiele zur Wirkung gegen Schadpilze:

Die fungizide Wirkung der Verbindungen der allgemeinen Formel I ließ sich durch folgende Versuche zeigen:

Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### 1. Erysiphe graminis var. tritici

Blätter von Weizenkeimlingen (Sorte "Kanzler") wurden zunächst mit der wäßrigen Aufbereitung der Wirkstoffe (250 ppm-haltig) behandelt. Nach ca. 24 h wurden die Pflanzen mit Sporen des Weizenmehltaus (Erysiphe graminis var. tritici) bestäubt. Die so behandelten Pflanzen wurden anschließend für 7 Tage bei 20-22°C und einer relativen Luftfeuchtigkeit von 75-80% inkubiert. Anschließend wurde das Ausmaß der Pilzentwicklung ermittlelt.

In diesem Test zeigten die mit den erfindungsgemäßen Verbindungen behandelten Pflanzen einen Befall von 5% und weniger, die mit einem bekannten Wirkstoff (Verbindung Nr. 195, Tabelle 3, EP-A 463 488) behandelten Pflanzen 25% Befall und die unbehandelten Pflanzen 70% Befall.

In einem entsprechenden Versuch (Weizenkeimlinge der Sorte "Kanzler", 250 ppm Auwandmenge) wurden die Pflanzen zunächst infiziert. und inkubiert und anschließend mit den Wirksatoffen behandelt. In diesem Test zeigten die mit den erfindungsgemäßen Verbindungen behandelten Pflanzen einen Befall von 5% und weniger und die unbehandelten Pflanzen 60% Befall.

### Beispiele zur Wirkung gegen tierische Schädlinge:

Die insektizide Wirkung der Verbindungen der allgemeinen Formel I ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

### Aphis fabae (Schwarze Laus), Kontaktwirkung

Stark befallene Buschbohnen (Vicia faba) wurden mit der wässrigen Wirkstoffaufbereitung behandelt. Nach 24 h wurde die Mortalitätsrate bestimmt.

In diesem Test zeigten die erfindungsgemäßen Verbindungen I.68, I.69, I.70, I.71, II.12 und II.13 Wirkschwellen von 400 ppm und weniger.

### Nephotettix cincticeps (Grüne Reiszikade), Kontaktwirkung

Rundfilter wurden mit der wäßrigen Wirkstoffaufbereitung behandelt und anschließend mit 5 adulten Zikaden belegt. Nach 24 h wurde die Mortalität beurteilt.

In diesem Test zeigten die erfindungsgemäßen Verbindungen I.10, I.24, I.29, I.47, I.52, I.74, I.75, I.79 und I.92 Wirkschwellen von 0,4 mg und weniger.

### Prodenia litura (Ägyptischer Baumwollwurm), Kontaktwirkung

Mit der wäßrigen Wirkstoffaufbereitung behandelte Filter wurden mit 5 Raupen belegt. Die erste Beurteilung erfolgt nach 4 h. Sofern noch mindestens eine Raupe lebt, wird eine Futtermischung zugegeben. Nach 24 h wurde die Mortalität bestimmt.

In diesem Test zeigten die erfindungsgemäßen Verbindungen I.79, I.92, und I.101, Wirkschwellen von 0,4 mg und weniger.

### Tetranychus telarius (Rote Spinne), Kontaktwirkung

Getopfte Buschbohnen, die das zweite Folgeblattpaar zeigten, wurden mit wässrigen Wirkstoffaufbereitung behandelt. Nach 24 h wurden die Pflanzen mit Hilfe von stark befallenen Blattstücken infiziert. Nach 12 Tagen im Gewächshaus wurde der Befall bestimmt.

In diesem Test zeigten die Verbindungen I.92, I.101, II.13, II.14 und II.15 Wirkschwellen von 400 ppm und weniger.

In diesem Test zeigten die erfindungsgemäßen Verbindungen I.79, I.92, und I.101, Wirkschwellen von 0,4 mg und weniger.

### Tetranychus telarius (Rote Spinne), Kontaktwirkung

Getopfte Buschbohnen, die das zweite Folgeblattpaar zeigten, wurden mit wässrigen Wirkstoffaufbereitung behandelt. Nach 24 h wurden die Pflanzen mit Hilfe von stark befallenen Blattstücken infiziert. Nach 12 Tagen im Gewächshaus wurde der Befall bestimmt.

In diesem Test zeigten die Verbindungen I.92, I.101, II.13, II.14 und II.15 Wirkschwellen von 400 ppm und weniger.

## Patentansprüche

1. Phenylessigsäurederivate der Formel I 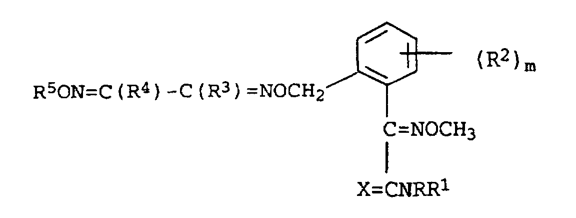 in der die Substituenten und der Index die folgende Bedeutung haben:
X Sauerstoff oder Schwefel;
R Wasserstoff und C₁-C₄-Alkyl;
R¹ Wasserstoff und C₁-C₄-Alkyl;
R² Cyano, Nitro, Trifluormethyl, Halogen, C₁-C₄-Alkyl und C₁-C₄-Alkoxy;
m 0, 1 oder 2, wobei die Reste R² verschieden sein können, wenn m für 2 steht;
R³ Wasserstoff, Cyano, Nitro, Hydroxy, Amino, Halogen,
C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylamino oder Di-C₁-C₄-alkylamino;
R⁴ Wasserstoff, Nitro, Hydroxy, Amino, Halogen, C₁-C₆-Alkyl, C₁-C₆-Alkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkenylthio, C₂-C₆-Alkenylamino, N-C₂-C₆-Alkenyl-N-C₁-C₆-alkylamino, C₂-C₆-Alkinyl, C₂-C₆-Alkinyloxy, C₂-C₆-Alkinylthio, C₂-C₆-Alkinylamino, N-C₂-C₆-Alkinyl-N-C₁-C₆-alkylamino, wobei die Kohlenwasserstoffreste dieser Gruppen partiell oder vollständig halogeniert sein können oder einen bis drei der folgenden Reste tragen können: Cyano, Nitro, Hydroxy, Merkapto, Amino, Carboxyl, Aminocarbonyl, Aminothiocarbonyl, Halogen, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Hetaryl, Hetaryloxy, Hetaryl-C₁-C₄-alkoxy, Hetarylthio, Hetaryl-C₁-C₄-alkylthio, wobei die cyclischen Reste ihrerseitspartiell oder vollständig halogeniert sein können und/oder ein bis drei der folgenden Gruppen tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio und C (=NOR⁶)-Aₙ-R⁷;
C₃-C₆-Cycloalkyl, C₃-C₆-Cycloalkyloxy, C₃-C₆-Cycloalkylthio, C₃-C₆-Cycloalkylamino, N-C₃-C₆-Cycloalkyl-N-C₁-C₆-alkylamino, C₃-C₆-Cycloalkenyl, C₃-C₆-Cycloalkenyloxy, C₃-C₆-Cycloalkenylthio, C₃-C₆-Cycloalkenylamino, N-C₃-C₆-Cycloalkenyl-N-C₁-C₆-alkylamino, Heterocyclyloxy, Heterocyclylthio, Heterocyclylamino, N-Heterocyclyl-N-C₁-C₆-alkylamino, Aryl, Aryloxy, Arylthio, Arylamino, N-Aryl-N-C₁-C₆-alkylamino, Hetaryl, Hetaryloxy, Hetarylthio, Hetarylamino, N-Hetaryl-N-C₁-C₆-alkylamino, wobei die cyclischen Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl und Hetaryloxy;
R⁵ Wasserstoff, C₁-C₁₀-Alkyl, C₃-C₆-Cycloalkyl, C₂-C₁₀-Alkenyl, C₂-C₁₀-Alkinyl, C₁-C₁₀-Alkylcarbonyl, C₂-C₁₀-Alkenylcarbonyl, C₃-C₁₀-Alkinylcarbonyl oder C₁-C₁₀-Alkylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkyloxy, Heterocyclyl, Heterocyclyloxy, Benzyl, Benzyloxy, Aryl, Aryloxy, Arylthio, Hetaryl, Hetaryloxy und Hetarylthio, wobei die cyclischen Gruppen ihrerseits partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C(=NOR⁶)-Aₙ-R⁷;
Aryl, Arylcarbonyl, Arylsulfonyl, Hetaryl, Hetarylcarbonyl oder Hetarylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Hetaryl, Hetaryloxy oder C(=NOR⁶)-Aₙ-R⁷;
wobei
A für Sauerstoff, Schwefel oder Stickstoff steht und wobei der Stickstoff Wasserstoff oder C₁-C₆-Alkyl trägt;
n 0 oder 1 bedeutet;
R⁶ Wasserstoff oder C₁-C₆-Alkyl bedeutet;
R⁷ Wasserstoff oder C₁-C₆-Alkyl bedeutet;
Aryl für Phenyl, Naphthyl oder Phenanthrenyl steht;
Hetaryl
5-gliedriges Heteroaryl, enthaltend ein bis vier Stickstoffatome oder ein bis drei Stickstoffatome und ein Schwefeloder Sauerstoffatom oder ein Sauerstoff oder ein Schwefelatom,
benzokondensiertes 5-gliedriges Heteroaryl, enthaltend ein bis drei Stickstoffatome oder ein Stickstoffatom und/oder ein Sauerstoff- oder Schwefelatom,
über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome,
6-gliedriges Heteroaryl, enthaltend ein bis vier Stickstoffatome oder
benzokondensiertes 6-gliedriges Heteroaryl, enthaltend ein bis vier Stickstoffatome bedeutet;
Heterocyclyl drei- bis sechsgliedrige, gesättigte oder partiell ungesättigte mono- oder polycyclische Heterocyclen, die ein bis drei Heteroatome ausgewählt aus einer Gruppe bestehend aus Sauerstoff, Stickstoff und Schwefel enthalten, bedeutet;
ausgenommen Verbindungen der Formel I, in denen
X Sauerstoff;
NRR¹ NHCH₃;
m 0;
R³ Wasserstoff, Cyano, C₁-C₄-Alkyl, C₁-C₄-Halogenalkyl oder Methylthio;
R⁴ Wasserstoff, unsubstituiertes C₁-C₆-Alkyl, unsubstituiertes C₃-C₆-Cycloalkyl, Thienyl oder
Phenyl, das ggf. substituiert ist durch:
Halogen, Cyano, Nitro, C₁-C₄-Alkyl, C₁-C₄-Alkoxy, C₁-C₂-Halogenalkyl, C₁-C₂-Halogenalkoxy, C₃-C₆-Alkenyloxy, C₃-C₆-Alkinyloxy, C₃-C₄-Alkylendioxy; oder
folgende über Sauerstoff, C₁-C₄-Alkylenoxy, C₁-C₄-Oxyalkylen, S(O)ₙ, C₁-C₄-Alkylen-S(O)ₙ, S(O)ₙ-C₁-C₄-Alkylen, wobei n für 0, 1 oder 2 steht, gebundene Reste:
C₁-C₆-Alkyl, C₁-C₆-Halogenalkyl, C₃-C₆-Cycloalkyl oder C₂-C₆-Alkenyl oder C₂-C₄-Alkinyl-C₁-C₂-alkyl, wobei diese Reste 1 bis 3-fach halogeniert sein können; oder
ggf. durch Halogen, Trimethylsilyl, C₁-C₆-Alkyl, C₁-C₆-Halogenalkyl, C₁-C₆-Alkoxy, C₁-C₆-Halogenalkoxy, ggf. substituiertes Cyclopropyl oder Cyclopropyl-C₁-C₃-alkyl substituierte Aryl- oder Heterocyclylreste;
und
R⁵ Wasserstoff, C₁-C₆-Alkyl, C₁-C₆-Halogenalkyl; C₁-C₄-Alkoxy-C₁-C₂-alkyl, ggf. 1- bis 3-fach halogeniertes C₂-C₄-Alkenyl-C₁-C₂-alkyl, C₂-C₄-Alkinyl-C₁-C₂-alkyl, ggf. 1- bis 4-fach halogeniertes C₃-C₆-Cycloalkyl, ggf. 1- bis 4-fach halogeniertes C₃-C₆-Cycloalkyl-C₁-C₂-alkyl, Cyano-C₁-C₄-alkyl, C₁-C₄-Alkoxycarbonyl-C₁-C₂-alkyl;
Phenyl-C₁-C₃-alkyl, das substituiert sein kann durch: Halogen, Cyano, Nitro, C₁-C₃-Alkyl, C₁-C₄-Alkoxy, C₁-C₄-Halogenalkyl, wobei die Phenylgruppe ggf. 1- bis 3-fach durch gleiche oder verschiedene Reste substituiert sein kann;
Phenyl, das ggf. 1- oder 2-fach substituiert sein kann durch: Halogen, Cyano, Nitro, C₁-C₄-Alkyl, C₁-C₄-Alkoxy, C₁-C₂-Halogenalkyl, wobei dieser Rest 1 bis 3 Halogenatome trägt;
oder
Pyridyl, das 1- bis 2-fach substituiert sein kann durch: Halogen, Cyano, Nitro, C₁-C₄-Alkyl, C₁-C₄-Alkoxy, C₁-C₂-Halogenalkyl, wobei dieser Rest 1 bis 3 Halogenatome trägt, bedeuten,
wobei von den ausgenommenen Verbindungen die folgenden Einzelverbindungen der Formel I.3a 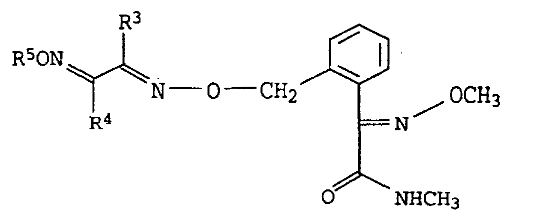
**Tabelle A**
| Nr. | R³ | R⁴ | R⁵ |
|---|---|---|---|
| 631 | CH₃ | C₆H₅ | C₂H₅ |
| 1157 | CH₃ | 2-F-C₆H₄ | C₂H₅ |
| 1168 | CH₃ | 3-F-C₆H₄ | CH₃ |
| 1169 | CH₃ | 3-F-C₆H₄ | C₂H₅ |
| 1181 | CH₃ | 4-F-C₆H₄ | C₂H₅ |
| 1192 | CH₃ | 2-Cl-C₆H₄ | CH₃ |
| 1193 | CH₃ | 2-Cl-C₆H₄ | C₂H₅ |
| 1205 | CH₃ | 3-Cl-C₆H₄ | C₂H₅ |
| 1217 | CH₃ | 4-Cl-C₆H₄ | C₂H₅ |
| 1228 | CH₃ | 2,3-Cl₂-C₆H₃ | CH₃ |
| 1229 | CH₃ | 2,3-Cl₂-C₆H₃ | C₂H₅ |
| 1240 | CH₃ | 2,4-Cl₂-C₆H₃ | CH₃ |
| 1241 | CH₃ | 2,4-Cl₂-C₆H₃ | C₂H₅ |
| 1252 | CH₃ | 2,5-Cl₂-C₆H₃ | CH₃ |
| 1253 | CH₃ | 2,5-Cl₂-C₆H₃ | C₂H₅ |
| 1264 | CH₃ | 2,6-Cl₂-C₆H₃ | CH₃ |
| 1265 | CH₃ | 2,6-Cl₂-C₆H₃ | C₂H₅ |
| 1276 | CH₃ | 3,4-Cl₂-C₆H₃ | CH₃ |
| 1277 | CH₃ | 3,4-Cl₂-C₆H₃ | C₂H₅ |
| 1288 | CH₃ | 3,5-Cl₂-C₆H₃ | CH₃ |
| 1289 | CH₃ | 3,5-Cl₂-C₆H₃ | C₂H₅ |
| 1300 | CH₃ | 2-Br-C₆H₄ | CH₃ |
| 1301 | CH₃ | 2-Br-C₆H₄ | C₂H₅ |
| 1313 | CH₃ | 3-Br-C₆H₄ | C₂H₅ |
| 1325 | CH₃ | 4-Br-C₆H₄ | C₂H₅ |
| 1336 | CH₃ | 2-I-C₆H₄ | CH₃ |
| 1337 | CH₃ | 2-I-C₆H₄ | C₂H₅ |
| 1348 | CH₃ | 3-I-C₆H₄ | CH₃ |
| 1349 | CH₃ | 3-I-C₆H₄ | C₂H₅ |
| 1360 | CH₃ | 4-I-C₆H₄ | CH₃ |
| 1361 | CH₃ | 4-I-C₆H₄ | C₂H₅ |
| 1372 | CH₃ | 2-CN-C₆H₄ | CH₃ |
| 1373 | CH₃ | 2-CN-C₆H₄ | C₂H₅ |
| 1384 | CH₃ | 3-CN-C₆H₄ | CH₃ |
| 1385 | CH₃ | 3-CN-C₆H₄ | C₂H₅ |
| 1396 | CH₃ | 4-CN-C₆H₄ | CH₃ |
| 1397 | CH₃ | 4-CN-C₆H₄ | C₂H₅ |
| 1408 | CH₃ | 2-NO₂-C₆H₄ | CH₃ |
| 1409 | CH₃ | 2-NO₂-C₆H₄ | C₂H₅ |
| 1420 | CH₃ | 3-NO₂-C₆H₄ | CH₃ |
| 1421 | CH₃ | 3-NO₂-C₆H₄ | C₂H₅ |
| 1432 | CH₃ | 4-NO₂-C₆H₄ | CH₃ |
| 1433 | CH₃ | 4-NO₂-C₆H₄ | C₂H₅ |
| 1445 | CH₃ | 2-CH₃-C₆H₄ | C₂H₅ |
| 1456 | CH₃ | 3-CH₃-C₆H₄ | CH₃ |
| 1457 | CH₃ | 3-CH₃-C₆H₄ | C₂H₅ |
| 1469 | CH₃ | 4-CH₃-C₆H₄ | C₂H₅ |
| 1480 | CH₃ | 2,3-(CH₃)₂-C₆H₃ | CH₃ |
| 1481 | CH₃ | 2,3-(CH₃)₂-C₆H₃ | C₂H₅ |
| 1492 | CH₃ | 2,4-(CH₃)₂-C₆H₃ | CH₃ |
| 1493 | CH₃ | 2,4-(CH₃)₂-C₆H₃ | C₂H₅ |
| 1504 | CH₃ | 2,5-(CH₃)₂-C₆H₃ | CH₃ |
| 1505 | CH₃ | 2,5-(CH₃)₂-C₆H₃ | C₂H₅ |
| 1516 | CH₃ | 2,6-(CH₃)₂-C₆H₃ | CH₃ |
| 1517 | CH₃ | 2,6-(CH₃)₂-C₆H₃ | C₂H₅ |
| 1528 | CH₃ | 3,4-(CH₃)₂-C₆H₃ | CH₃ |
| 1529 | CH₃ | 3,4-(CH₃)₂-C₆H₃ | C₂H₅ |
| 1540 | CH₃ | 3,5-(CH₃)₂-C₆H₃ | CH₃ |
| 1541 | CH₃ | 3,5-(CH₃)₂-C₆H₃ | C₂H₅ |
| 1552 | CH₃ | 2-C₂H₅-C₆-H₄ | CH₃ |
| 1553 | CH₃ | 2-C₂H₅-C₆-H₄ | C₂H₅ |
| 1562 | CH₃ | 3-C₂H₅-C₆-H₄ | CH₃ |
| 1563 | CH₃ | 3-C₂H₅-C₆-H₄ | C₂H₅ |
| 1574 | CH₃ | 4-C₂H₅-C₆-H₄ | CH₃ |
| 1575 | CH₃ | 4-C₂H₅-C₆-H₄ | C₂H₅ |
| 1586 | CH₃ | 2-i-C₃H₇-C₆H₄ | CH₃ |
| 1587 | CH₃ | 2-i-C₃H₇-C₆H₄ | C₂H₅ |
| 1598 | CH₃ | 3-i-C₃H₇-C₆H₄ | CH₃ |
| 1599 | CH₃ | 3-i-C₃H₇-C₆H₄ | C₂H₅ |
| 1610 | CH₃ | 4-i-C₃H₇-C₆H₄ | CH₃ |
| 1611 | CH₃ | 4-i-C₃H₇-C₆H₄ | C₂H₅ |
| 1658 | CH₃ | 2-OCH₃-C₆H₄ | CH₃ |
| 1659 | CH₃ | 2-OCH₃-C₆H₄ | C₂H₅ |
| 1670 | CH₃ | 3-OCH₃-C₆H₄ | CH₃ |
| 1671 | CH₃ | 3-OCH₃-C₆H₄ | C₂H₅ |
| 1683 | CH₃ | 4-OCH₃-C₆H₄ | C₂H₅ |
| 1694 | CH₃ | 2-OC₂H₅-C₆H₄ | CH₃ |
| 1695 | CH₃ | 2-OC₂H₅-C₆H₄ | C₂H₅ |
| 1706 | CH₃ | 3-OC₂H₅-C₆H₄ | CH₃ |
| 1707 | CH₃ | 3-OC₂H₅-C₆H₄ | C₂H₅ |
| 1719 | CH₃ | 4-OC₂H₅-C₆H₄ | C₂H₅ |
| 1730 | CH₃ | 2-O-(i-C₃H₇)-C₆H₄ | CH₃ |
| 1731 | CH₃ | 2-O-(i-C₃H₇)-C₆H₄ | C₂H₅ |
| 1742 | CH₃ | 3-O-(i-C₃H₇)-C₆H₄ | CH₃ |
| 1743 | CH₃ | 3-O-(i-C₃H₇)-C₆H₄ | C₂H₅ |
| 1754 | CH₃ | 4-O-(i-C₃H₇)-C₆H₄ | CH₃ |
| 1755 | CH₃ | 4-O-(i-C₃H₇)-C₆H₄ | C₂H₅ |
| 1766 | CH₃ | 2-O-(t-C₄H₉)-C₆H₄ | CH₃ |
| 1767 | CH₃ | 2-O-(t-C₄H₉)-C₆H₄ | C₂H₅ |
| 1778 | CH₃ | 3-O-(t-C₄H₉)-C₆H₄ | CH₃ |
| 1779 | CH₃ | 3-O-(t-C₄H₉)-C₆H₄ | C₂H₅ |
| 1790 | CH₃ | 4-O-(t-C₄H₉)-C₆H₄ | CH₃ |
| 1791 | CH₃ | 4-O-(t-C₄H₉)-C₆H₄ | C₂H₅ |
| 1803 | CH₃ | 2-CF₃-C₆H₄ | C₂H₅ |
| 1815 | CH₃ | 3-CF₃-C₆H₄ | C₂H₅ |
| 1826 | CH₃ | 4-CF₃-C₆H₄ | CH₃ |
| 1827 | CH₃ | 4-CF₃-C₆H₄ | C₂H₅ |
| 1946 | CH₃ | 2-OCF₃-C₆H₄ | CH₃ |
| 1947 | CH₃ | 2-OCF₃-C₆H₄ | C₂H₅ |
| 1960 | CH₃ | 3-OCF₃-C₆H₄ | CH₃ |
| 1961 | CH₃ | 3-OCF₃-C₆H₄ | C₂H₅ |
| 1972 | CH₃ | 4-OCF₃-C₆H₄ | CH₃ |
| 1973 | CH₃ | 4-OCF₃-C₆H₄ | C₂H₅ |
| 1984 | CH₃ | 2-SCH₃-C₆H₄ | CH₃ |
| 1985 | CH₃ | 2-SCH₃-C₆H₄ | C₂H₅ |
| 1996 | CH₃ | 3-SCH₃-C₆H₄ | CH₃ |
| 1997 | CH₃ | 3-SCH₃-C₆H₄ | C₂H₅ |
| 2008 | CH₃ | 4-SCH₃-C₆H₄ | CH₃ |
| 2009 | CH₃ | 4-SCH₃-C₆H₄ | C₂H₅ |
| 2020 | CH₃ | 2-Methyl-sulfonyl-C₆H₄ | CH₃ |
| 2021 | CH₃ | 2-Methyl-sulfonyl-C₆H₄ | C₂H₅ |
| 2032 | CH₃ | 3-Methyl-sulfonyl-C₆H₄ | CH₃ |
| 2033 | CH₃ | 3-Methyl-sulfonyl-C₆H₄ | C₂H₅ |
| 2044 | CH₃ | 4-Methyl-sulfonyl-C₆H₄ | CH₃ |
| 2045 | CH₃ | 4-Methyl-sulfonyl-C₆H₄ | C₂H₅ |
ausgeschlossen sind; sowie deren Salze.

2. Verbindungen der Formel I gemäß Anspruch 1, in denen R³, R⁴ und R⁵ die folgende Bedeutung haben:
R³ Wasserstoff, Hydroxy, Halogen, C₁-C₄-Alkyl, C₁-C₄-Alkoxy oder C₁-C₄-Alkylthio;
R⁴ Wasserstoff, Hydroxy, Halogen, Cyclopropyl, Cyclohexyl, C₁-C₄-Alkyl, C₁-C₄-Alkoxy, C₁-C₄-Alkylthio,
Aryl oder Hetaryl, wobei diese Gruppen partiell oder vollständig halogeniert sein können oder ein bis 3 der folgenden Gruppen tragen können: Cyano, Nitro, Hydroxy, Merkapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁- C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl,
C₂-C₆-Alkenyloxy, Benzyl, Benzyloxy, Aryl, Aryloxy, Hetaryl und Hetaryloxy;
R⁵ Wasserstoff,
C₁-C₁₀-Alkyl, C₃-C₆-Cycloalkyl, C₂-C₁₀-Alkenyl, C₂-C₁₀-Alkinyl, C₁-C₁₀-Alkylcarbonyl, C₂-C₁₀-Alkenylcarbonyl, C₃-C₁₀-Alkinylcarbonyl oder C₁-C₁₀-Alkylsulfonyl, wobei diese Gruppen partiell oder vollständig halogeniert sein können oder ein bis 3 der folgenden Gruppen tragen können: Cyano, Nitro, Hydroxy, Merkapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy und Hetarylthio, wobei die aromatischen und heteroaromatischen Reste ihrerseits partiell oder vollständig halogeniert sein können und/oder ein bis drei der folgenden Gruppen tragen können: Cyano, Nitro, Hydroxy, Mercapto, Amino, Carboxyl, Aminocarbonyl, Halogen, Aminothiocarbonyl, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C(=NOR⁶)-Aₙ-R⁷;
Aryl, Hetaryl, Arylcarbonyl, Hetarylcarbonyl, Arylsulfonyl oder Hetarylsulfonyl, wobei diese Gruppen partiell oder vollständig halogeniert sein können oder ein bis 3 der folgenden Gruppen tragen können: Cyano, Nitro,. Hydroxy, Merkapto, Amino, Carboxyl, Aminocarbonyl, Aminothiocarbonyl, Halogen, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl, Hetaryloxy oder C(=NOR⁶)-Aₙ-R⁷;
wobei
A für Sauerstoff, Schwefel oder Stickstoff steht und wobei der Stickstoff Wasserstoff oder C₁-C₆-Alkyl trägt;
n 0 oder 1;
R⁶ Wasserstoff oder C₁-C₆-Alkyl bedeutet und
R⁷ Wasserstoff oder C₁-C₆-Alkyl bedeutet,
sowie deren Salze.

3. Verbindungen der Formel I gemäß Anspruch 1, in denen m für 0 steht.

4. Verbindungen der Formel I gemäß Anspruch 1, in denen R¹ für Methyl steht.

5. Verfahren zur Herstellung der Verbindungen der Formel I gemäß Anspruch 1, in denen R³ nicht Halogen bedeutet, **dadurch gekennzeichnet, daß** man ein Benzylderivat der Formel II 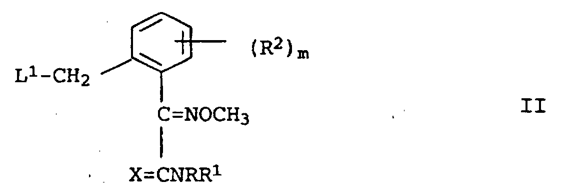 in der L¹ für eine nucleophil austauschbare Abgangsgruppe steht, in an sich bekannter Weise mit einem Hydroxyimin der Formel III
R⁵ON=C(R⁴)-C(R³)=NOH III
umsetzt.

6. Verfahren zur Herstellung der Verbindungen der Formel I gemäß Anspruch 1, in denen R³ und R⁴ nicht Halogen bedeutet, **dadurch gekennzeichnet, daß** man ein Benzylderivat der Formel II gemäß Anspruch 5 in an sich bekannter Weise mit einem Dihydroxyimin der Formel IV
HON=C(R⁴)-C(R³)=NOH IV
zu einer Verbindung der Formel V 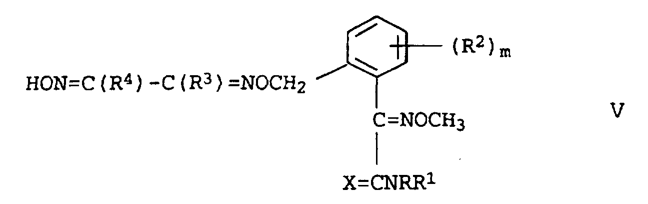 umsetzt und V anschließend mit einer Verbindung der Formel VI
R⁵-L² VI
in der L² für eine nucleophil austauschbare Abgangsgruppe steht, zu I umsetzt.

7. Verfahren zur Herstellung der Verbindungen der Formel I gemäß Anspruch 1, in denen R³ nicht Halogen bedeutet, **dadurch gekennzeichnet, daß** man ein Benzylderivat der Formel II gemäß Anspruch 5 in an sich bekannter Weise mit einem Carbonylhydroxyimin der Formel VII
O=C(R⁴)-C(R³)=NOH VII
zu einer Verbindung der Formel VIII 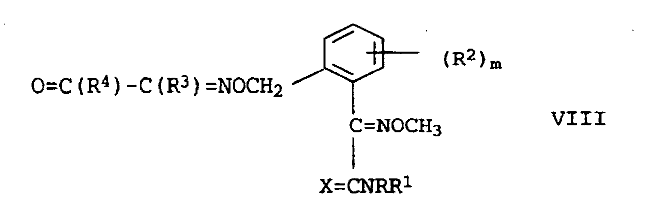 umsetzt, VIII anschließend entweder
a) zunächst mit Hydroxylamin oder dessen Salz und danach mit einer Verbindung der Formel VI (R⁵-L²) gemäß Anspruch 6 oder
b) mit einem Hydroxylamin oder einem Hydroxylammoniumsalz der Formel IXa bzw. IXb
R⁵ONH₂ IXa R⁵ONH₃⁺ Q⁻ IXb
in der Q⁻ für das Anion einer Säure steht,
zu I umsetzt.

8. Mittel gegen tierische Schädlinge oder Schadpilze, enthaltend übliche Zusatzstoffe und eine wirksame Menge einer Verbindung der Formel I gemäß Anspruch 1.

9. Verfahren zur Bekämpfung von tierischen Schädlingen oder Schadpilzen, **dadurch gekennzeichnet, daß** man die Schädlinge oder Schadpilze, deren Lebensraum oder die von ihnen freizuhaltenden Pflanzen, Flächen, Materialien oder Räume mit einer wirksamen Menge einer Verbindung der Formel I gemäß Anspruch 1 behandelt.

10. Verwendung der Verbindungen I gemäß Anspruch 1 zur Herstellung von Mitteln gegen tierische Schädlinge oder Schadpilze.

11. Verwendung der Verbindungen I gemäß Anspruch 1 zur Bekämpfung von tierischen Schädlingen oder Schadpilzen.

## Claims

1. A phenylacetic acid derivative of the formula I 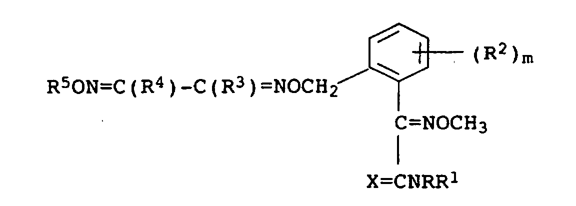 where the substituents and the index have the following meanings:
X is oxygen or sulfur;
R is hydrogen or C₁-C₄-alkyl;
R¹ is hydrogen or C₁-C₄-alkyl;
R² is cyano, nitro, trifluoromethyl, halogen, C₁-C₄-alkyl or C₁-C₄-alkoxy;
m is 0, 1 or 2, it being possible for the R² radicals to be different if m is 2;
R³ is hydrogen, cyano, nitro, hydroxyl, amino, halogen, C₁-C₄-alkyl, C₁-C₄-haloalkyl, C₁-C₄-alkoxy, C₁-C₄-haloalkoxy, C₁-C₄-alkylthio, C₁-C₄-alkylamino or di-C₁-C₄-alkylamino;
R⁴ is hydrogen, cyano, nitro, hydroxyl, amino, halogen, C₁-C₆-alkyl, C₁-C₆-alkoxy, C₁-C₆-alkylthio, C₁-C₆-alkylamino, di-C₁-C₆-alkylamino, C₂-C₆-alkenyl, C₂-C₆-alkenyloxy, C₂-C₆-alkenylthio, C₂-C₆-alkenylamino, N-C₂-C₆-alkenyl-N-C₁-C₆-alkylamino, C₂-C₆-alkynyl, C₂-C₆-alkynyloxy, C₂-C₆-alkynylthio, C₂-C₆-alkynylamino, N-C₂-C₆-alkynyl-N-C₁-C₆-alkylamino, it being possible for the hydrocarbon radicals of these groups to be partly or completely halogenated or to carry one to three of the following radicals: cyano, nitro, hydroxyl, mercapto, amino, carboxyl, aminocarbonyl, aminothiocarbonyl, halogen, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₁-C₆-alkylsulfonyl, C₁-C₆-alkylsulfoxyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₂-C₆-alkenyloxy, C₃-C₆-cycloalkyl, C₃-C₆-cycloalkoxy, heterocyclyl, heterocyclyloxy, aryl, aryloxy, aryl-C₁-C₄-alkoxy, arylthio, aryl-C₁-C₄-alkylthio, hetaryl, hetaryloxy, hetaryl-C₁-C₄-alkoxy, hetarylthio, hetaryl-C₁-C₄-alkylthio, it being possible for the cyclic radicals in turn to be partly or completely halogenated and/or to carry one to three of the following groups: cyano, nitro, hydroxyl, mercapto, amino, carboxyl, aminocarbonyl, aminothiocarbonyl, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio and C(=NOR⁶)-Aₙ-R⁷;
C₃-C₆-cycloalkyl, C₃-C₆-cycloalkoxy, C₃-C₆-cycloalkylthio, C₃-C₆-cycloalkylamino, N-C₃-C₆-cycloalkyl-N-C₁-C₆-alkylamino, C₃-C₆-cycloalkenyl, C₃-C₆-cycloalkenyloxy, C₃-C₆-cycloalkenylthio, C₃-C₆-cycloalkenylamino, N-C₃-C₆-cycloalkenyl-N-C₁-C₆-alkylamino, heterocyclyloxy, heterocyclylthio, heterocyclylamino, N-heterocyclyl-N-C₁-C₆-alkylamino, aryl, aryloxy, arylthio, arylamino, N-aryl-N-C₁-C₆-alkylamino, hetaryl, hetaryloxy, hetarylthio, hetarylamino, N-hetaryl- N-C₁-C₆-alkylamino, it being possible for the cyclic radicals to be partly or completely halogenated or to carry one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, hetaryl and hetaryloxy;
R⁵ is hydrogen,
C₁-C₁₀-alkyl, C₃-C₆-cycloalkyl, C₂-C₁₀-alkenyl, C₂-C₁₀-alkynyl, C₁-C₁₀-alkylcarbonyl, C₂-C₁₀-alkenylcarbonyl, C₃-C₁₀-alkynylcarbonyl or C₁-C₁₀-alkylsulfonyl, it being possible for these radicals to be partly or completely halogenated or to carry one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, C₃-C₆-cycloalkyl, C₃-C₆-cycloalkoxy, heterocyclyl, heterocyclyloxy, benzyl, benzyloxy, aryl, aryloxy, arylthio, hetaryl, hetaryloxy and hetarylthio, it being possible for the cyclic groups in turn to be partly or completely halogenated or to carry one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio or C(=NOR⁶)-Aₙ-R⁷;
aryl, arylcarbonyl, arylsulfonyl, hetaryl, hetarylcarbonyl or hetarylsulfonyl, it being possible for these radicals to be partly or completely halogenated or to carry one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylcarbonyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, hetaryl, hetaryloxy or C(=NOR⁶)-Aₙ-R⁷;
where
A is oxygen, sulfur or nitrogen and where the nitrogen carries hydrogen or C₁-C₆-alkyl;
n is 0 or 1;
R⁶ is hydrogen or C₁-C₆-alkyl;
R⁷ is hydrogen or C₁-C₆-alkyl;
aryl is phenyl, naphthyl or phenanthrenyl;
hetaryl
5-membered heteroaryl, comprising one to four nitrogen atoms or one to three nitrogen atoms and a sulfur or oxygen atom or an oxygen or a sulfur atom,
benzo-fused 5-membered heteroaryl, comprising one to three nitrogen atoms or a nitrogen atom and/or an oxygen or sulfur atom,
5-membered heteroaryl bonded via nitrogen, comprising one to four nitrogen atoms, or benzo-fused 5-membered heteroaryl bonded via nitrogen, comprising one to three nitrogen atoms,
6-membered heteroaryl, comprising one to four nitrogen atoms or
benzo-fused 6-membered heteroaryl, comprising one to four nitrogen atoms;
heterocyclyl, three- to six-membered, saturated or partially unsaturated mono- or polycyclic heterocycles which contain one to three heteroatoms selected from a group consisting of oxygen, nitrogen and sulfur;
excepting compounds of the formula I in which
X is oxygen;
NRR¹NHCH₃;
m is 0;
R³ is hydrogen, cyano, C₁-C₄-alkyl, C₁-C₄-haloalkyl or methylthio;
R⁴ is hydrogen, unsubstituted C₁-C₆-alkyl, unsubstituted C₃-C₆-cycloalkyl, thienyl or
phenyl which is optionally substituted by:
halogen, cyano, nitro, C₁-C₄-alkyl, C₁-C₄-alkoxy, C₁-C₂-haloalkyl, C₁-C₂-haloalkoxy, C₃-C₆-alkenyloxy, C₃-C₆-alkynyloxy, C₃-C₄-alkylenedioxy; or
the following radicals which are bonded via oxygen, C₁-C₄-alkylenoxy, C₁-C₄-oxyalkylene, S(O)ₙ, C₁-C₄-alkylene-S(O)ₙ, S(O)ₙ-C₁-C₄-alkylene, where n is 0, 1 or 2:
C₁-C₆-alkyl, C₁-C₆-haloalkyl, C₃-C₆-cycloalkyl or C₂-C₆-alkenyl or C₂-C₄-alkynyl-C₁-C₂-alkyl, it being possible for these radicals to be halogenated 1 to 3 times; or
aryl or heterocyclyl radicals which are optionally substituted by halogen, trimethylsilyl, C₁-C₆-alkyl, C₁-C₆-haloalkyl, C₁-C₆-alkoxy, C₁-C₆-haloalkoxy, optionally substituted cyclopropyl or cyclopropyl-C₁-C₃-alkyl;
and
R⁵ is hydrogen, C₁-C₆-alkyl, C₁-C₆-haloalkyl, C₁-C₄-alkoxy-C₁-C₂-alkyl, C₂-C₄-alkenyl-C₁-C₂-alkyl which is optionally halogenated 1 to 3 times,
C₂-C₄-alkynyl-C₁-C₂-alkyl, C₃-C₆-cycloalkyl which is optionally halogenated 1 to 4 times, C₃-C₆-cycloalkyl-C₁-C₂-alkyl which is optionally halogenated 1 to 4 times, cyano-C₁-C₄-alkyl, C₁-C₄-alkoxycarbonyl-C₁-C₂-alkyl;
phenyl-C₁-C₃-alkyl, which can be substituted by: halogen, cyano, nitro, C₁-C₃-alkyl, C₁-C₄-alkoxy, C₁-C₄-haloalkyl, it being possible for the phenyl group optionally to be substituted 1 to 3 times by identical or different radicals;
phenyl which can optionally be substituted 1 or 2 times by: halogen, cyano, nitro, C₁-C₄-alkyl, C₁-C₄-alkoxy, C₁-C₂-haloalkyl, where this radical carries 1 to 3 halogen atoms;
or
pyridyl which can be substituted 1 or 2 times by: halogen, cyano, nitro, C₁-C₄-alkyl, C₁-C₄-alkoxy, C₁-C₂-haloalkyl, where this radical carries 1 to 3 halogen atoms;
of the excepted compounds the following individual compounds of the formula I.3a 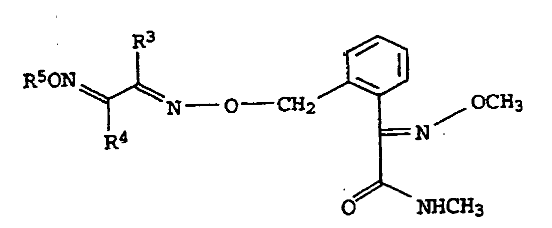
**Table A**
| No, | R³ | R⁴ | R⁵ |
|---|---|---|---|
| 631 | CH₃ | C₆H₅ | C₂H₅ |
| 1157 | CH₃ | 2-F-C₆H₄ | C₂H₅ |
| 1168 | CH₃ | 3-F-C₆H₄ | CH₃ |
| 1169 | CH₃ | 3-F-C₆H₄ | C₂H₅ |
| 1181 | CH₃ | 4-F-C₆H₄ | C₂H₅ |
| 1192 | CH₃ | 2-Cl-C₆H₄ | CH₃ |
| 1193 | CH₃ | 2-Cl-C₆H₄ | C₂H₅ |
| 1205 | CH₃ | 3-Cl-C₆H₄ | C₂H₅ |
| 1217 | CH₃ | 4-Cl-C₆H₄ | C₂H₅ |
| 1228 | CH₃ | 2,3-Cl₂-C₆H₃ | CH₃ |
| 1229 | CH₃ | 2,3-Cl₂-C₆H₃ | C₂H₅ |
| 1240 | CH₃ | 2,4-Cl₂-C₆H₃ | CH₃ |
| 1241 | CH₃ | 2,4-Cl₂-C₆H₃ | C₂H₅ |
| 1252 | CH₃ | 2,5-Cl₂-C₆H₃ | CH₃ |
| 1253 | CH₃ | 2,5-Cl₂-C₆H₃ | C₂H₅ |
| 1264 | CH₃ | 2,6-Cl₂-C₆H₃ | CH₃ |
| 1265 | CH₃ | 2,6-Cl₂-C₆H₃ | C₂H₅ |
| 1276 | CH₃ | 3,4-Cl₂-C₆H₃ | CH₃ |
| 1277 | CH₃ | 3,4-Cl₂-C₆H₃ | C₂H₅ |
| 1288 | CH₃ | 3,5-Cl₂-C₆H₃ | CH₃ |
| 1289 | CH₃ | 3,5-Cl₂-C₆H₃ | C₂H₅ |
| 1300 | CH₃ | 2-Br-C₆H₄ | CH₃ |
| 1301 | CH₃ | 2-Br-C₆H₄ | C₂H₅ |
| 1313 | CH₃ | 3-Br-C₆H₄ | C₂H₅ |
| 1325 | CH₃ | 4-Br-C₆H₄ | C₂H₅ |
| 1336 | CH₃ | 2-I-C₆H₄ | CH₃ |
| 1337 | CH₃ | 2-I-C₆H₄ | C₂H₅ |
| 1348 | CH₃ | 3-I-C₆H₄ | CH₃ |
| 1349 | CH₃ | 3-I-C₆H₄ | C₂H₅ |
| 1360 | CH₃ | 4-I-C₆H₄ | CH₃ |
| 1361 | CH₃ | 4-I-C₆H₄ | C₂H₅ |
| 1372 | CH₃ | 2-CN-C₆H₄ | CH₃ |
| 1373 | CH₃ | 2-CN-C₆H₄ | C₂H₅ |
| 1384 | CH₃ | 3-CN-C₆H₄ | CH₃ |
| 1385 | CH₃ | 3-CN-C₆H₄ | C₂H₅ |
| 1396 | CH₃ | 4-CN-C₆H₄ | CH₃ |
| 1397 | CH₃ | 4-CN-C₆H₄ | C₂H₅ |
| 1408 | CH₃ | 2-NO₂-C₆H₄ | CH₃ |
| 1409 | CH₃ | 2-NO₂-C₆H₄ | C₂H₅ |
| 1420 | CH₃ | 3-NO₂-C₆H₄ | CH₃ |
| 1421 | CH₃ | 3-NO₂-C₆H₄ | C₂H₅ |
| 1432 | CH₃ | 4-NO₂-C₆H₄ | CH₃ |
| 1433 | CH₃ | 4-NO₂-C₆H₄ | C₂H₅ |
| 1445 | CH₃ | 2-CH₃-C₆H₄ | C₂H₅ |
| 1456 | CH₃ | 3-CH₃-C₆H₄ | CH₃ |
| 1457 | CH₃ | 3-CH₃-C₆H₄ | C₂H₅ |
| 1469 | CH₃ | 4-CH₃-C₆H₄ | C₂H₅ |
| 1480 | CH₃ | 2,3-(CH₃)₂-C₆H₃ | CH₃ |
| 1481 | CH₃ | 2,3-(CH₃)₂-C₆H₃ | C₂H₅ |
| 1492 | CH₃ | 2,4-(CH₃)₂-C₆H₃ | CH₃ |
| 1493 | CH₃ | 2,4-(CH₃)₂-C₆H₃ | C₂H₅ |
| 1504 | CH₃ | 2,5-(CH₃)₂-C₆H₃ | CH₃ |
| 1505 | CH₃ | 2,5-(CH₃)₂-C₆H₃ | C₂H₅ |
| 1516 | CH₃ | 2,6-(CH₃)₂-C₆H₃ | CH₃ |
| 1517 | CH₃ | 2,6-(CH₃)₂-C₆H₃ | C₂H₅ |
| 1528 | CH₃ | 3,4-(CH₃)₂-C₆H₃ | CH₃ |
| 1529 | CH₃ | 3,4-(CH₃)₂-C₆H₃ | C₂H₅ |
| 1540 | CH₃ | 3,5-(CH₃)₂-C₆H₃ | CH₃ |
| 1541 | CH₃ | 3,5-(CH₃)₂-C₆H₃ | C₂H₅ |
| 1552 | CH₃ | 2-C₂H₅-C₆H₄ | CH₃ |
| 1553 | CH₃ | 2-C₂H₅-C₆H₄ | C₂H₅ |
| 1562 | CH₃ | 3-C₂H₅-C₆H₄ | CH₃ |
| 1563 | CH₃ | 3-C₂H₅-C₆H₄ | C₂H₅ |
| 1574 | CH₃ | 4-C₂H₅-C₆H₄ | CH₃ |
| 1575 | CH₃ | 4-C₂H₅-C₆H₄ | C₂H₅ |
| 1586 | CH₃ | 2-i-C₃H₇-C₆H₄ | CH₃ |
| 1587 | CH₃ | 2-i-C₃H₇-C₆H₄ | C₂H₅ |
| 1598 | CH₃ | 3-i-C₃H₇-C₆H₄ | CH₃ |
| 1599 | CH₃ | 3-i-C₃H₇-C₆H₄ | C₂H₅ |
| 1610 | CH₃ | 4-i-C₃H₇-C₆H₄ | CH₃ |
| 1611 | CH₃ | 4-i-C₃H₇-C₆H₄ | C₂H₅ |
| 1658 | CH₃ | 2-OCH₃-C₆H₄ | CH₃ |
| 1659 | CH₃ | 2-OCH₃-C₆H₄ | C₂H₅ |
| 1670 | CH₃ | 3-OCH₃-C₆H₄ | CH₃ |
| 1671 | CH₃ | 3-OCH₃-C₆H₄ | C₂H₅ |
| 1683 | CH₃ | 4-OCH₃-C₆H₄ | C₂H₅ |
| 1694 | CH₃ | 2-OC₂H₅-C₆H₄ | CH₃ |
| 1695 | CH₃ | 2-OC₂H₅-C₆H₄ | C₂H₅ |
| 1706 | CH₃ | 3-OC₂H₅-C₆H₄ | CH₃ |
| 1707 | CH₃ | 3-OC₂H₅-C₆H₄ | C₂H₅ |
| 1719 | CH₃ | 4-OC₂H₅-C₆H₄ | C₂H₅ |
| 1730 | CH₃ | 2-O-(i-C₃H₇)-C₆H₄ | CH₃ |
| 1731 | CH₃ | 2-O-(i-C₃H₇)-C₆H₄ | C₂H₅ |
| 1742 | CH₃ | 3-O-(i-C₃H₇)-C₆H₄ | CH₃ |
| 1743 | CH₃ | 3-O-(i-C₃H₇)-C₆H₄ | C₂H₅ |
| 1754 | CH₃ | 4-O-(i-C₃H₇)-C₆H₄ | CH₃ |
| 1755 | CH₃ | 4-O-(i-C₃H₇)-C₆H₄ | C₂H₅ |
| 1766 | CH₃ | 2-O-(t-C₄H₉)-C₆H₄ | CH₃ |
| 1767 | CH₃ | 2-O-(t-C₄H₉)-C₆H₄ | C₂H₅ |
| 1778 | CH₃ | 3-O-(t-C₄H₉)-C₆H₄ | CH₃ |
| 1779 | CH₃ | 3-O-(t-C₄H₉)-C₆H₄ | C₂H₅ |
| 1790 | CH₃ | 4-O-(t-C₄H₉)-C₆H₄ | CH₃ |
| 1791 | CH₃ | 4-O-(t-C₄H₉)-C₆H₄ | C₂H₅ |
| 1803 | CH₃ | 2-CF₃-C₆H₄ | C₂H₅ |
| 1815 | CH₃ | 3-CF₃-C₆H₄ | C₂H₅ |
| 1826 | CH₃ | 4-CF₃-C₆H₄ | CH₃ |
| 1827 | CH₃ | 4-CF₃-C₆H₄ | C₂H₅ |
| 1946 | CH₃ | 2-OCF₃-C₆H₄ | CH₃ |
| 1947 | CH₃ | 2-OCF₃-C₆H₄ | C₂H₅ |
| 1960 | CH₃ | 3-OCF₃-C₆H₄ | CH₃ |
| 1961 | CH₃ | 3-OCF₃-C₆H₄ | C₂H₅ |
| 1972 | CH₃ | 4-OCF₃-C₆H₄ | CH₃ |
| 1973 | CH₃ | 4-OCF₃-C₆H₄ | C₂H₅ |
| 1984 | CH₃ | 2-SCF₃-C₆H₄ | CH₃ |
| 1985 | CH₃ | 2-SCF₃-C₆H₄ | C₂H₅ |
| 1996 | CH₃ | 3-SCF₃-C₆H₄ | CH₃ |
| 1997 | CH₃ | 3-SCF₃-C₆H₄ | C₂H₅ |
| 2008 | CH₃ | 4-SCF₃-C₆H₄ | CH₃ |
| 2009 | CH₃ | 4-SCF₃-C₆H₄ | C₂H₅ |
| 2020 | CH₃ | 2-methylsulfonyl-C₆H₄ | CH₃ |
| 2021 | CH₃ | 2-methylsulfonyl-C₆H₄ | C₂H₅ |
| 2032 | CH₃ | 3-methylsulfonyl-C₆H₄ | CH₃ |
| 2033 | CH₃ | 3-methylsulfonyl-C₆H₄ | C₂H₅ |
| 2044 | CH₃ | 4-methylsulfonyl-C₆H₄ | CH₃ |
| 2045 | CH₃ | 4-methylsulfonyl-C₆H₄ | C₂H₅ |
being excluded; or its salts.

2. A compound of the formula I as claimed in claim 1, in which R³, R⁴ and R⁵ have the following meanings:
R³ is hydrogen, hydroxyl, halogen, C₁-C₄-alkyl, C₁-C₄-alkoxy or C₁-C₄-alkylthio,
R⁴ is hydrogen, hydroxyl, halogen, cyclopropyl, cyclohexyl, C₁-C₄-alkyl, C₁-C₄-alkoxy, C₁-C₄-alkylthio,
aryl or hetaryl, it being possible for these groups to be partly or completely halogenated or to carry one to 3 of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl,
C₂-C₆-alkenyloxy, benzyl, benzyloxy, aryl, aryloxy, hetaryl and hetaryloxy;
R⁵ is hydrogen,
C₁-C₁₀-alkyl, C₃-C₆-cycloalkyl, C₂-C₁₀-alkenyl, C₂-C₁₀-alkynyl, C₁-C₁₀-alkylcarbonyl, C₂-C₁₀-alkenylcarbonyl, C₃-C₁₀-alkynylcarbonyl or C₁-C₁₀-alkylsulfonyl, it being possible for these groups to be partly or completely halogenated or to carry one to 3 of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy and hetarylthio, it being possible for the aromatic and heteroaromatic radicals, in turn, to be partly or completely halogenated and/or to carry one to three of the following groups: cyano, nitro, hydroxyl, mercapto, amino, carboxyl, aminocarbonyl, halogen, aminothiocarbonyl, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio or C(=NOR⁶)-Aₙ-R⁷;
aryl, hetaryl, arylcarbonyl, hetarylcarbonyl, arylsulfonyl or hetarylsulfonyl, it being possible for these groups to be partly or completely halogenated or to carry one to 3 of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylcarbonyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, hetaryl, hetaryloxy or C(=NOR⁶)-Aₙ-R⁷;
where
A is oxygen, sulfur or nitrogen and where the nitrogen carries hydrogen or C₁-C₆-alkyl;
n is 0 or 1;
R⁶ is hydrogen or C₁-C₆-alkyl and
R⁷ is hydrogen or C₁-C₆-alkyl,
and its salts.

3. A compound of the formula I as claimed in claim 1, in which m is 0.

4. A compound of the formula I as claimed in claim 1, in which R¹ is methyl.

5. A process for preparing the compounds of the formula I as claimed in claim 1, in which R³ is not halogen, which comprises reacting a benzyl derivative of the formula II 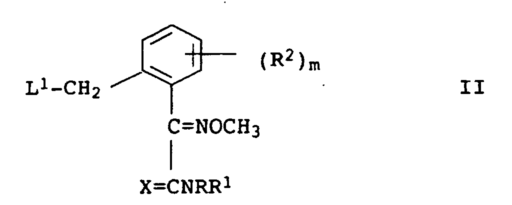 in which L¹ is a nucleophilically replaceable leaving group, in a manner known per se with a hydroxyimine of the formula III
R⁵ON=C(R⁴)-C(R³)=NOH III

6. A process for preparing the compounds of the formula I as claimed in claim 1, in which R³ and R⁴ are not halogen, which comprises reacting a benzyl derivative of the formula II as in claim 5 in a manner known per se with a dihydroxyimine of the formula IV
HON=C(R⁴)-C(R³)=NOH IV
to give a compound of the formula V 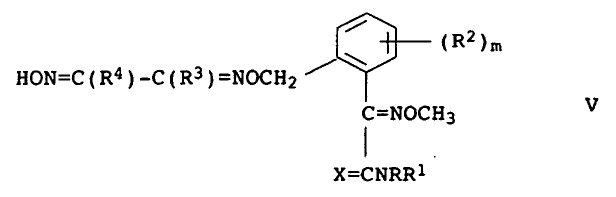 and then reacting V with a compound of the formula VI
R⁵-L² VI
where L² is a nucleophilically replaceable leaving group, to give I.

7. A process for preparing the compounds of the formula I as claimed in claim 1, in which R³ is not halogen, which comprises reacting a benzyl derivative of the formula II as in claim 5 in a manner known per se with a carbonylhydroxyimine of the formula VII
O=C(R⁴)-C(R³)=NOH VII
to give a compound of the formula VIII 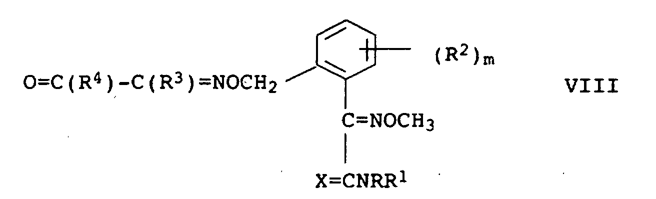 then either reacting VIII
a) first with hydroxylamine or its salt and then with a compound of the formula VI (R⁵-L²) as in claim 6 or
b) with a hydroxylamine or a hydroxylammonium salt of the formula IXa or IXb
R⁵ONH₂ IXa R⁵ONH₃^{⊕} Q^{⊖} IXb
where Q^{⊖} is the anion of an acid,
to give I.

8. A composition against animal pests or harmful fungi, containing customary additives and an effective amount of a compound of the formula I as claimed in claim 1.

9. A method for controlling animal pests or harmful fungi, which comprises treating the pests or harmful fungi, their environment or the plants, surfaces, materials or spaces to be kept free from them with an effective amount of a compound of the formula I as claimed in claim 1.

10. The use of the compounds I as claimed in claim 1 for the production of compositions against animal pests or harmful fungi.

11. The use of the compounds I as claimed in claim 1 for the control of animal pests or harmful fungi.

## Revendications

1. Dérivés de l'acide phénylacétique répondant à la formule I 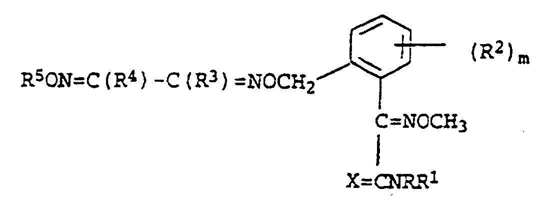 dans laquelle les symboles et l'indice ont les significations suivantes :
X : l'oxygène ou le soufre ;
R : l'hydrogène ou un groupe alkyle en C1-C4 ;
R¹: l'hydrogène ou un groupe alkyle en C1-C4 ;
R² : un groupe cyano, nitro, trifluorométhyle, un halogène, un groupe alkyle en C1-C4 ou alcoxy en C1-C4 ;
m : 0, 1 ou 2, les substituants R² pouvant être différents lorsque m est égal à 2 ;
R³ : l'hydrogène, un groupe cyano, nitro, hydroxy, amino, un halogène, un groupe alkyle en C1-C4, halogénoalkyle en C1-C4, alcoxy en C1-C4, halogénoalcoxy en C1-C4, alkylthio en C1-C4, alkylamino en C1-C4 ou di-(alkyle en C1-C4)amino ;
R⁴ : l'hydrogène, un groupe nitro, hydroxy, amino, un halogène, un groupe alkyle en C1-C6, alcoxy en C1-C6, alkylthio en C1-C6, alkylamino en C1-C6, di-(alkyle en C1-C6)amino, alcényle en C2-C6, alcényloxy en C2-C6, alcénylthio en C2-C6, alcénylamino en C2-C6, N-(alcényle en C2-C6)-N-alkylamino en C1-C6, alcynyle en C2-C6, alcynyloxy en C2-C6, alcynylthio en C2-C6, alcynylamino en C2-C6, N-(alcynyle en C2-C6)-N-alkylamino en C1-C6, les radicaux hvdrocarbonés de ces groupes pouvant être halogénés en totalité ou en partie ou pouvant porter un à trois des substituants suivants : cyano, nitro, hydroxy, mercapto, amino, carboxyle, aminocarbonyle, aminothiocarbonyle, halogéno, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alkylsulfonyle en C1-C6, alkylsulfoxyle en C1-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, alcényloxy en C2-C6, cycloalkyle en C3-C6, cycloalcoxy en C3-C6, hétérocyclyle, hétérocyclyloxy, aryle, aryloxy, aryl-alcoxy en C1-C4, arylthio, aryl-alkylthio en C1-C4, hétéroaryle, hétéroaryloxy, hétéroaryl-alcoxy en C1-C4, hétéroarylthio, hétéroaryl-alkylthio en C1-C4, les groupes cycliques pouvant eux-mêmes être halogénés en totalité ou en partie et/ou pouvant porter un à trois des substituants suivants: cyano, nitro, hydroxy, mercapto, amino, carboxyle, aminocarbonyle, aminothiocarbonyle, alkyle en C1-C6, aminoalkyle en C1-C6, alkylsulfonyle en C1-C6, alkylsulfoxyle en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6) amino, (alkyle en C1-C6) aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, arylthio, hétéroaryle, hétéroaryloxy, hétéroarylthio et C(=NOR⁶)-Aₙ-R⁷ ;
un groupe cycloalkyle en C3-C6, cycloalcoxy en C3-C6, cycloalkylthio en C3-C6, cycloalkylamino en C3-C6, N-(cycloalkyle en C3-C6)-N-alkylamino en C1-C6, cycloalcényle en C3-C6, cycloalcényloxy en C3-C6, cycloalcénylthio en C3-C6, cycloalcénylamino en C3-C6, N-(cycloalcényle en C3-C6)-N-alkylamino en C1-C6, hétérocyclyloxy, hétérocyclylthio, hétérocyclylamino, N-hétérocyclyl-N-alkylamino en C1-C6, aryle, aryloxy, arylthio, arylamino; N-aryl-N-alkylamino en C1-C6, hétéroaryle, hétéroaryloxy, hétéroarylthio, hétéroarylamino, N-hétéroaryl-N-alkylamino en C1-C6, les radicaux cycliques pouvant être halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, alkylsulfonyle en C1-C6, alkylsulfoxyle en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényle en C2-C6, alcényloxy en C2-C6, benzyle, benzyloxy, aryle, aryloxy, hétéroaryle et hétéroaryloxy ;
R⁵ : l'hydrogène,
un groupe alkyle en C1-C10, cycloalkyle en C3-C6, alcényle en C2-C10, alcynyle en C2-C10, (alkyle en C1-C10)carbonyle, (alcényle en C2-C10)carbonyle, (alcynyle en C3-C10)carbonyle ou alkylsulfonyle en C1-C10, ces groupes pouvant être halogénés en totalité ou en partie ou pouvant porter un à trois des substituants suivants cyano, nitro, hydroxy, mercapto, amino, carboxyle, aminocarbonyle, aminothiocarbonyle, halogéno, alkyle en C1-C6, halogénoalkyle en C1-C6, alkylsulfonyle en C1-C6, alkylsulfoxyle en C1-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, cycloalkyle en C3-C6, cycloalcoxy en C3-C6, hétérocyclyle, hétérocyclyloxy, benzyle, benzyloxy, aryle, aryloxy, arylthio, hétéroaryle, hétéroaryloxy et hétéroarylthio, les groupes cycliques pouvant eux-mêmes être halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, alkylsulfonyle en C1-C6, alkylsulfoxyle en C1-C6, cycloalkyle en C3-C6, alcoxy en C1-C6, halogénoalcoxy en C1-C6, (alkyle en C1-C6)oxycarbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6) aminothiocarbonyle, alcényle en C2-C6, alcényloxy en C2-C6, benzyle, benzyloxy, aryle, aryloxy, arylthio, hétéroaryle, hétéroaryloxy, hétéroarylthio ou C(=NOR⁶)-Aₙ-R⁷ ;
un groupe aryle, arylcarbonyle, arylsulfonyle, hétéroaryle, hétéroarylcarbonyle ou hétéroarylsulfonyle, ces groupes pouvant être halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, (alkyle en C1-C6)carbonyle, alkylsulfonyle en C1-C6, alkylsulfoxyle en C1-C6, cycloalkyle en C3-C6, alcoxy en C1-C6, halogénoalcoxy en C1-C6, (alkyle en C1-C6)oxycarbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, hétéroaryle, hétéroaryloxy ou C(=NOR⁶)-Aₙ-R⁷ ;
dans lequel
A représente l'oxygène, le soufre ou l'azote et l'azote porte de l'hydrogène ou un groupe alkyle en C1-C6 ;
n est égal à 0 ou 1 ;
R⁶ représente l'hydrogène ou un groupe alkyle en C1-C6 ;
R⁷ représente l'hydrogène ou un groupe alkyle en C1-C6 ;
un groupe "aryle" est un groupe phényle, naphtyle ou phénanthrényle ;
un groupe "hétéroaryle" est :
un groupe hétéroaryle à cinq chaînons contenant un à quatre atomes d'azote ou un à trois atomes d'azote et un atome de soufre ou d'oxygène ou un atome d'oxygène ou un atome de soufre,
un groupe hétéroaryle à cinq chaînons, benzocondensé, contenant un à trois atomes d' azote ou un atome d'azote et/ou un atome d'oxygène ou de soufre,
un groupe hétéroaryle à cinq chaînons, relié par l'azote, contenant un à quatre atomes d'azote, ou un groupe hétéroaryle à cinq chaînons, benzocondensé, relié par l'azote, qui contient un à trois atomes d'azote,
un groupe hétéroaryle à six chaînons qui contient un à quatre atomes d'azote ou bien un groupe hétéroaryle à six chaînons, benzocondensé, contenant un à quatre atomes d'azote ;
un groupe "hétérocyclyle" est un groupe hétérogène mono- ou poly-cyclique saturé ou partiellement insaturé de trois à six chaînons, qui contient un à trois hétéroatomes choisis parmi l'oxygène, l'azote et le soufre ;
à l'exception des composés répondant à la formule I dans laquelle
X représente l'oxygène ;
NRR¹ représente NHCH₃
m est égal à 0 ;
R³ représente l'hydrogèn un groupe cyano, alkyle en C1-C4, halogénoalkyle en C1-C4 ou méthylthio ;
R⁴ représente l'hydrogène, un groupe alkyle en C1-C6 non substitué, un groupe cycloalkyle en C3-C6 non substitué, un groupe thiényle ou
un groupe phényle, éventuellement substitué par :
des halogènes, des groupes cyano, nitro, alkyle en C1-C4, alcoxy en C1-C4, halogénoalkyle en C1-C2, halogénoalcoxy en C1-C2, alcényloxy en C3-C6, alcényloxy en C3-C6, (alkylène en C3-C4)dioxy ; ou bien
les groupes suivants reliés par l'intermédiaire de l'oxygène, d'un groupe (alkylène en C1-C4)oxy, oxyalkylène en C1-C4, S(0)ₙ, (alkylène en C1-C4)-S(0)ₙ, S(O)ₙ-alkylène en C1-C4, n étant égal à 0, 1 ou 2 alkyle en C1-C6, halogénoalkyle en C1-C6, cycloalkyle en C3-C6 ou alcényle en C2-C6 ou (alcynyle en C2-C4)alkyle en C1-C2, ces groupes pouvant être halogénés une à trois fois ; ou bien
aryle ou hétérocyclyle éventuellement substitués par des halogènes des groupes triméthylsilyle, alkyle en C1-C6, halogénoalkyle en C1-C6, alcoxy en C1-C6, halogénoalcoxy en C1-C6, cyclopropyle lui-même éventuellement substitué ou cyclopropyle-alkyle en C1-C3 ;
et
R⁵ représente l'hydrogène, un groupe alkyle en C1-C6, halogénoalkyle en C1-C6 ; (alcoxy en C1-C4)alkyle en C1-C2, (alcényle en C2-C4)alkyle en C1-C2 éventuellement halogéné une à trois fois, (alcynyle en C2-C4)alkyle en C1-C2, cycloalkyle en C3-C6 éventuellement halogéné une à quatre fois, {cycloalkyle en C3-C6)alkyle en C1-C2 éventuellement halogéné une à quatre fois, cyano-alkyle en C1-C4, (alcoxy en C1-C4)carbonyl-alkyle en C1-C4 ; phényl-alkyle en C1-C3 qui peut être substitué par des halogènes, des groupes cyano, nitro, alkyle en C1-C3, alcoxy en C1-C4, halogénoalkyle en C1-C4, le groupe phényle pouvant lui-même porter éventuellement un à trois substituants identiques ou différents ;
le groupe phényle portant éventuellement un ou deux substituants choisis parmi les halogènes, les groupes cyano, nitro, alkyle en C1-C4, alcoxy en C1-C4, halogénoalkyle en C1-C2, ce dernier portant un à trois atomes d'halogènes ;
ou bien
un groupe pyridyle qui peut porter un à deux substituants choisis parmi les halogènes, les groupes cyano, nitro, alkyle en C1-C4, alcoxy en C1-C4, halogénoalkyle en C1-C2, ce dernier portant un à trois atomes d'halogènes,
étant précisé que parmi les composés exceptés, les composés individuels suivants de formule I.3a 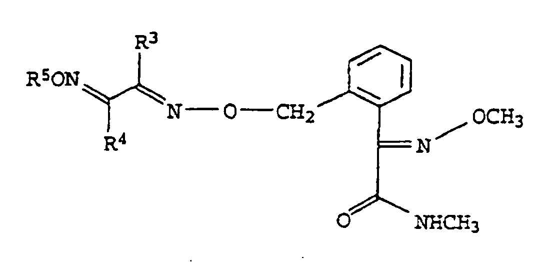
**Tableau A**
| No. | R³ | R⁴ | R⁵ |
|---|---|---|---|
| 631 | CH₃ | C₆H₅ | C₂H₅ |
| 1157 | CH₃ | 2-F-C₆H₄ | C₂H₅ |
| 1168 | CH₃ | 3-F-C₆H₄ | CH₃ |
| 1169 | CH₃ | 3-F-C₆H₄ | C₂H₅ |
| 1181 | CH₃ | 4-F-C₆H₄ | C₂H₅ |
| 1192 | CH₃ | 2-Cl-C₆H₄ | CH₃ |
| 1193 | CH₃ | 2-Cl-C₆H₄ | C₂H₅ |
| 1205 | CH₃ | 3-Cl-C₆H₄ | C₂H₅ |
| 1217 | CH₃ | 4-Cl-C₆H₄ | C₂H₅ |
| 1228 | CH₃ | 2,3-Cl₂-C₆H₃ | CH₃ |
| 1229 | CH₃ | 2,3-Cl₂-C₆H₃ | C₂H₅ |
| 1240 | CH₃ | 2,4-Cl₂-C₆H₃ | CH₃ |
| 1241 | CH₃ | 2,4-Cl₂-C₆H₃ | C₂H₅ |
| 1252 | CH₃ | 2,5-Cl₂-C₆H₃ | CH₃ |
| 1253 | CH₃ | 2,5-Cl₂-C₆H₃ | C₂H₅ |
| 1264 | CH₃ | 2,6-Cl₂-C₆H₃ | CH₃ |
| 1265 | CH₃ | 2,6-Cl₂-C₆H₃ | C₂H₅ |
| 1276 | CH₃ | 3,4-Cl₂-C₆H₃ | CH₃ |
| 1277 | CH₃ | 3,4-Cl₂-C₆H₃ | C₂H₅ |
| 1288 | CH₃ | 3,5-Cl₂-C₆H₃ | CH₃ |
| 1289 | CH₃ | 3,5-Cl₂-C₆H₃ | C₂H₅ |
| 1300 | CH₃ | 2-Br-C₆H₄ | CH₃ |
| 1301 | CH₃ | 2-Br-C₆H₄ | C₂H₅ |
| 1313 | CH₃ | 3-Br-C₆H₄ | C₂H₅ |
| 1325 | CH₃ | 4-Br-C6H₄ | C₂H₅ |
| 1336 | CH₃ | 2-I-C₆H₄ | CH₃ |
| 1337 | CH₃ | 2-I-C₆H₄ | C₂H₅ |
| 1348 | CH₃ | 3-I-C₆H₄ | CH₃ |
| 1349 | CH₃ | 3-I-C₆H₄ | C₂H₅ |
| 1360 | CH₃ | 4-I-C₆H₄ | CH₃ |
| 1361 | CH₃ | 4-I-C₆H₄ | C₂H₅ |
| 1372 | CH₃ | 2-CN-C₆H₄ | CH₃ |
| 1373 | CH₃ | 2-CN-C₆H₄ | C₂H₅ |
| 1384 | CH₃ | 3-CN-C₆H₄ | CH₃ |
| 1385 | CH₃ | 3-CN-C₆H₄ | C₂H₅ |
| 1396 | CH₃ | 4-CN-C₆H₄ | CH₃ |
| 1397 | CH₃ | 4-CN-C₆H₄ | C₂H₅ |
| 1408 | CH₃ | 2-NO₂-C₆H₄ | CH₃ |
| 1409 | CH₃ | 2-NO₂-C₆H₄ | C₂H₅ |
| 1420 | CH₃ | 3-NO₂-C₆H₄ | CH₃ |
| 1421 | CH₃ | 3-NO₂-C₆H₄ | C₂H₅ |
| 1432 | CH₃ | 4-NO₂-C₆H₄ | CH₃ |
| 1433 | CH₃ | 4-NO₂-C₆H₄ | C₂H₅ |
| 1445 | CH₃ | 2-CH₃-C₆H₄ | C₂H₅ |
| 1456 | CH₃ | 3-CH₃-C₆H₄ | CH₃ |
| 1457 | CH₃ | 3-CH₃-C₆H₄ | C₂H₅ |
| 1469 | CH₃ | 4-CH₃-C₆H₄ | C₂H₅ |
| 1480 | CH₃ | 2,3-(CH₃)₂-C₆H₃ | CH₃ |
| 1481 | CH₃ | 2,3-(CH₃)₂-C₆H₃ | C₂H₅ |
| 1492 | CH₃ | 2,4-(CH₃)₂-C₆H₃ | CH₃ |
| 1493 | CH₃ | 2,4-(CH₃)₂-C₆H₃ | C₂H₅ |
| 1504 | CH₃ | 2,5-(CH₃)₂-C₆H₃ | CH₃ |
| 1505 | CH₃ | 2,5-(CH₃)₂-C₆H₃ | C₂H₅ |
| 1516 | CH₃ | 2,6-(CH₃)₂-C₆H₃ | CH₃ |
| 1517 | CH₃ | 2,6-(CH₃)₂-C₆H₃ | C₂H₅ |
| 1528 | CH₃ | 3,4-(CH₃)₂-C₆H₃ | CH₃ |
| 1529 | CH₃ | 3,4-(CH₃)₂-C₆H₃ | C₂H₅ |
| 1540 | CH₃ | 3,5-(CH₃)₂-C₆H₃ | CH₃ |
| 1541 | CH₃ | 3,5-(CH₃)₂-C₆H₃ | C₂H₅ |
| 1552 | CH₃ | 2-C₂H₅-C₆-H₄ | CH₃ |
| 1553 | CH₃ | 2-C₂H₅-C₆-H₄ | C₂H₅ |
| 1562 | CH₃ | 3-C₂H₅-C₆-H₄ | CH₃ |
| 1563 | CH₃ | 3-C₂H₅-C₆-H₄ | C₂H₅ |
| 1574 | CH₃ | 4-C₂H₅-C₆-H₄ | CH₃ |
| 1575 | CH₃ | 4-C₂H₅-C₆-H₄ | C₂H₅ |
| 1586 | CH₃ | 2-i-C₃H₇-C₆H₄ | CH₃ |
| 1587 | CH₃ | 2-i-C₃H₇-C₆H₄ | C₂H₅ |
| 1598 | CH₃ | 3-i-C₃H₇-C₆H₄ | CH₃ |
| 1599 | CH₃ | 3-i-C₃H₇-C₆H₄ | C₂H₅ |
| 1610 | CH₃ | 4-i-C₃H₇-C₆H₄ | CH₃ |
| 1611 | CH₃ | 4-i-C₃H₇-C₆H₄ | C₂H₅ |
| 1658 | CH₃ | 2-OCH₃-C₆H₄ | CH₃ |
| 1659 | CH₃ | 2-OCH₃-C₆H₄ | C₂H₅ |
| 1670 | CH₃ | 3-OCH₃-C₆H₄ | CH₃ |
| 1671 | CH₃ | 3-OCH₃-C₆H₄ | C₂H₅ |
| 1683 | CH₃ | 4-OCH₃-C₆H₄ | C₂H₅ |
| 1694 | CH₃ | 2-OC₂H₅-C₆H₄ | CH₃ |
| 1695 | CH₃ | 2-OC₂H₅-C₆H₄ | C₂H₅ |
| 1706 | CH₃ | 3-OC₂H₅-C₆H₄ | CH₃ |
| 1707 | CH₃ | 3-OC₂H₅-C₆H₄ | C₂H₅ |
| 1719 | CH₃ | 4-OC₂H₅-C₆H₄ | C₂H₅ |
| 1730 | CH₃ | 2-O-(i-C₃H₇)-C₆H₄ | CH₃ |
| 1731 | CH₃ | 2-O-(i-C₃H₇)-C₆H₄ | C₂H₅ |
| 1742 | CH₃ | 3-O-(i-C₃H₇)-C₆H₄ | CH₃ |
| 1743 | CH₃ | 3-O-(i-C₃H₇)-C₆H₄ | C₂H₅ |
| 1754 | CH₃ | 4-O-(i-C₃H₇)-C₆H₄ | CH₃ |
| 1755 | CH₃ | 4-O-(i-C₃H₇)-C₆H₄ | C₂H₅ |
| 1766 | CH₃ | 2-O-(t-C₄H₉)-C₆H₄ | CH₃ |
| 1767 | CH₃ | 2-O-(t-C₄H₉)-C₆H₄ | C₂H₅ |
| 1778 | CH₃ | 3-O-(t-C₄H₉)-C₆H₄ | CH₃ |
| 1779 | CH₃ | 3-O-(t-C₄H₉)-C₆H₄ | C₂H₅ |
| 1790 | CH₃ | 4-O-(t-C₄H₉)-C₆H₄ | CH₃ |
| 1791 | CH₃ | 4-O-(t-C₄H₉)-C₆H₄ | C₂H₅ |
| 1803 | CH₃ | 2-CF₃-C₆H₄ | C₂H₅ |
| 1815 | CH₃ | 3-CF₃-C₆H₄ | C₂H₅ |
| 1826 | CH₃ | 4-CF₃-C₆H₄ | CH₃ |
| 1827 | CH₃ | 4-CF₃-C₆H₄ | C₂H₅ |
| 1946 | CH₃ | 2-OCF₃-C₆H₄ | CH₃ |
| 1947 | CH₃ | 2-OCF₃-C₆H₄ | C₂H₅ |
| 1960 | CH₃ | 3-OCF₃-C₆H₄ | CH₃ |
| 1961 | CH₃ | 3-OCF₃-C₆H₄ | C₂H₅ |
| 1972 | CH₃ | 4-OCF₃-C₆H₄ | CH₃ |
| 1973 | CH₃ | 4-OCF₃-C₆H₄ | C₂H₅ |
| 1984 | CH₃ | 2-SCH₃-C₆H₄ | CH₃ |
| 1985 | CH₃ | 2-SCH₃-C₆H₄ | C₂H₅ |
| 1996 | CH₃ | 3-SCH₃-C₆H₄ | CH₃ |
| 1997 | CH₃ | 3-SCH₃-C₆H₄ | C₂H₅ |
| 2008 | CH₃ | 4-SCH₃-C₆H₄ | CH₃ |
| 2009 | CH₃ | 4-SCH₃-C₆H₄ | C₂H₅ |
| 2020 | CH₃ | 2-Méthyl-sulfonyl-C₆H₄ | CH₃ |
| 2021 | CH₃ | 2-Méthyl-sulfonyl-C₆H₄ | C₂H₅ |
| 2032 | CH₃ | 3-Méthyl-sulfonyl-C₆H₄ | CH₃ |
| 2033 | CH₃ | 3-Méthyl-sulfonyl-C₆H₄ | C₂H₅ |
| 2044 | CH₃ | 4-Méthyl-sulfonyl-C₆H₄ | CH₃ |
| 2045 | CH₃ | 4-Méthyl-sulfonyl-C₆H₄ | C₂H₅ |
sont exclus ; et leurs sels.

2. Composés de formule I selon la revendication 1, dans laquelle R³, R⁴ et R⁵ ont les significations suivantes :
R³ : l'hydrogèn, un groupe hydroxy, un halogène, un groupe alkyle en C1-C4, alcoxy en C1-C4 ou alkylthio en C1-C4 ;
R⁴ : l'hydrogène, un groupe hydroxy, un halogène, un groupe cyclopropyle, cyclohexyle, alkyle en C1-C4, alcoxy en C1-C4, alkylthio en C1-C4,
un groupe aryle ou hétéroaryle, chacun d'eux pouvant être halogéné en totalité ou en partie ou pouvant porter un à trois des substituants suivants : cyano, nitro, hydroxy, mercapto, amino, carboxyle, aminocarbonyle, aminothiocarbonyle, halogénoalkyle en C1-C6, halogénoalkyle en C1-C6, alkylsulfonyle en C1-C6, alkylsulfoxyle en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6,
un groupe alcényloxy en C2-C6, benzyle, benzyloxy, aryle, aryloxy, hétéroaryle ou hétéroaryloxy ;
R⁵ : l'hydrogène,
un groupe alkyle en C1-C10, cycloalkyle en C3-C6, alcényle en C2-C10, alcynyle en C2-C10, (alkyle en C1-C10)carbonyle, (alcényle en C2-C10)carbonyle, (alcynyle en C3-C10)carbonyle ou alkylsulfonyle en C1-C10, ces groupes pouvant être halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, alkylsulfonyle en C1-C6, alkylsulfoxyle en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, arylthio, hétéroaryle, hétéroaryloxy et hétéroarylthio, les radicaux aromatiques et hétéroatomatiques pouvant eux-mêmes être halogénés en totalité ou en partie et/ou pouvant porter un à trois des substituants suivants : cyano, nitro, hydroxy, mercapto, amino, carboxyle, aminocarbonyle, halogéno, aminothiocarbonyle, alkyle en C1-C6, halogénoalkyle en C1-C6, alkylsulfonyle en C1-C6, alkylsulfoxyle en C1-C6, cycloalkyle en C3-C6, alcoxy en C1-C6, halogénoalcoxy en C1-C6, (alkyle en C1-C6)oxycarbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, arylthio, hétéroaryle, hétéroaryloxy, hétéroarylthio ou C(=NOR⁶)-Aₙ-R⁷ ; un groupe aryle, hétéroaryle, arylcarbonyle, hétéroarylcarbonyle, arylsulfonyle; ou hétéroaryl-sulfonyle, ces groupes pouvant être halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, (alkyle en C1-C6)carbonyle, alkylsulfonyle en C1-C6, alkylsulfoxyle en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, hétéroaryle, hétéroaryloxy ou C(=NOR⁶)-Aₙ-R⁷ ;
dans lequel
A représente l'oxygène, le soufre ou l'azote et l'azote porte de l'hydrogène ou un groupe alkyle en C1-C6 ;
n est égal à 0 ou 1 ;
R⁶ représente l'hydrogène ou un groupe alkyle en C1-C6 et
R⁷ représente l'hydrogène ou un groupe alkyle en C1-C6, et leurs sels.

3. Composés de formule I selon la revendication 1 pour lesquels m est égal à 0.

4. Composés de formule I selon la revendication 1 pour lesquels R¹ représente le groupe méthyle.

5. Procédé pour la préparation des composés de formule I selon la revendication 1, pour lesquels R³ a une signification autre qu'un halogène, **caractérisé par le fait que** l'on fait réagir un dérivé benzylique de formule II 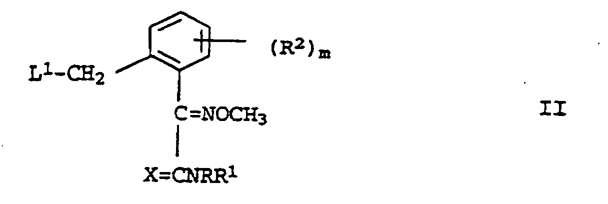 dans laquelle L¹ représente un groupe éliminable par échange nucléophile, de manière connue en soi, avec une hydroxyimine de formule III
R⁵ON=C(R⁴)-C(R³)=NOH III

6. Procédé pour la préparation des composés de formule I selon la revendication 1, pour lesquels R³ et R⁴ ont des significations autres que des halogènes, **caractérisé par le fait que** l'on convertit un dérivé benzylique de formule II selon revendication 5, de manière connue en soi, à l'aide d'une dihydroxyimine de formule IV
HON=C(R⁴)-C(R³)=NOH IV
en un composé de formule V 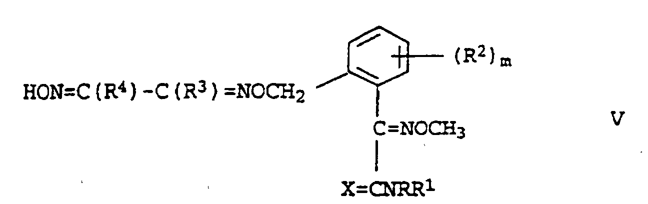 qu'on fait ensuite réagir avec un composé de formule VI
R⁵-L² VI
dans laquelle L² représente un groupe éliminable par échange nucléophile, ce qui donne I.

7. Procédé pour la préparation des composés de formule I selon la revendication 1 pour lesquels R³ a une signification autre qu'un halogène, **caractérisé par le fait que** l'on convertit un dérivé benzylique de formule II selon revendication 5, de manière connue en soi, à l'aide d'une carbonylhydroxyimine de formule VII
O=C(R⁴)-C(R³)=NOH VII
en un composé de formule VIII 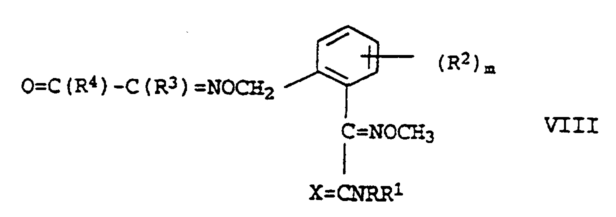 qu'on convertit ensuite en I
a) soit par réaction avec l'hydroxylamine ou l'un de ses sels puis avec un composé de formule VI (R⁵-L²) selon la revendication 6,
b) soit par réaction avec une hydroxylamine ou un sel d'hydroxylammonium de formules respectives IXa et IXb
R⁵ONH₂ Ixa R⁵ONH₃⁺ Q⁻ Ixb
dans lesquelles Q⁻ représente l'anion d'un acide.

8. Produit destiné à combattre les parasites animaux ou les mycètes nuisibles, contenant des additifs usuels et une quantité efficace d'un composé de formule I selon la revendication 1.

9. Procédé pour combattre les parasites animaux ou les mycètes nuisibles, **caractérisé par le fait que** l'on traite les parasites ou les mycètes nuisibles, leur habitat ou les végétaux, aires, matériaux ou locaux qu'on veut protéger contre les parasites ou les mycètes nuisibles par une quantité efficace d'un composé de formule I selon la revendication 1.

10. Utilisation des composés I selon la revendication 1 pour la préparation de produits destinés à combattre les parasites animaux ou les mycètes nuisibles.

11. Utilisation des composés I selon la revendication 1 pour la lutte contre les parasites animaux ou les mycètes nuisibles.
